# DEMANDE DE BREVET EUROPEEN

(11) **EP 3 239 131 A1**
(43) Date de publication de la demande: **01.11.2017**
(21) Numéro de dépôt: 17172035.2
(22) Date de dépôt: 17.06.2009
(51) Int. Cl.: C07C 215/10, C07C 217/58, C07C 229/14, C07C 233/06, C07C 237/40, C07C 271/56, C07C 311/20, C07C 35/02, C07D 209/04, C07D 213/38, C07D 221/22, C07D 239/20, A61P 39/02, A61K 31/137, A61K 31/17

(54) **NOUVEAUX COMPOSÉS AYANT UNE ACTIVITE PROTECTRICE VIS-A-VIS DE L'ACTION DE TOXINES ET DE VIRUS AU MODE D'ACTION INTRACELLULAIRE**

(30) Priorité: 17.06.2008 EP 08290570
(62) Demande divisionnaire de: 09766019.5
(71) Demandeur: Commissariat à l'Energie Atomique et aux Energies Alternatives, 75015 Paris (FR)
(72) Inventeur: LOPEZ, Roman, 92130 ISSY-LES-MOULINEAUX (FR); HEBBE, Séverine, 75012 PARIS (FR); GILLET, Daniel, 75003 PARIS (FR); BARBIER, Julien, 91190 GIF SUR YVETTE (FR)
(74) Mandataire: Gevers & Orès

(57) **Abrégé**

La présente invention a pour objet de nouvelles familles de composés dérivés de benzodiazépine, d'aminoadamantane, d'imine et d'amine aromatique, de médicament les comprenant et leur utilisation en tant qu'inhibiteurs des effets toxiques des toxines à activité intracellulaire, comme par exemple la ricine, et des virus utilisant la voie d'internalisation pour infecter les cellules.

## Description

La présente invention a pour objet de nouvelles familles de composés dérivés de benzodiazépine, d'aminoadamantane, d'imine et d'amine aromatique, de médicament les comprenant et leur utilisation en tant qu'inhibiteurs des effets toxiques des toxines à activité intracellulaire, comme par exemple la ricine, et des virus utilisant la voie d'internalisation pour infecter les cellules.

Les toxines à mode d'action intracellulaire, ou toxines AB, sont des enzymes organisées en plusieurs domaines : un domaine catalytique A qui porte l'activité toxique et un ou plusieurs domaines B assurant la reconnaissance cellulaire et permettant la translocation transmembranaire du fragment A dans le cytoplasme (Falnes, P.O. ; Sandvig, K. Curr. Opin. Cell. Biol. 2000, 12, 407). Il existe de nombreuses toxines bactériennes à activité intracellulaire : par exemple et de manière non exhaustive, la toxine diphtérique et la toxine du choléra ont un domaine A qui porte une activité enzymatique de type ADP-ribosyltransférase ; les grandes toxines clostridiales possèdent une activité glucosyltransférase ; la toxine botulique, la toxine tétanique et la toxine létale du charbon sont des métalloprotéases ; les toxines Shiga et la ricine possédent une activité N-glucosidase.

Plus particulièrement, la ricine est une toxalbumine produite par un arbrisseau de la famille des euphorbiacées, le ricin (*Ricinus communis*). Elle est présente à une concentration variant de 1 à 10 % dans la graine de Ricin.

Il s'agit plus précisément d'une glycoprotéine de 66 kDa composée de deux chaînes reliées par un pont disulfure. La chaîne A (RTA, 267 acides aminés) assure la fonction catalytique de la ricine (N-glucosidase), tandis que la chaîne B (RTB, lectine de 262 résidus) joue le rôle de transporteur permettant l'entrée de la ricine dans la cellule.

L'activité enzymatique de dépurination de l'ARN ribosomal, située sur la chaîne A, provoque dans les cellules intoxiquées l'arrêt de la synthèse des protéines et conduit à la mort cellulaire.

La chaîne B possède deux sites de liaison au galactose et permet la liaison de la toxine à des glycorécepteurs présents à la surface des cellules. La ricine peut alors pénétrer dans ces cellules par de multiples voies d'endocytose, pour atteindre le réseau *trans*-golgien où elle est acheminée vers le réticulum endoplasmique (RE) par transport rétrograde.

La toxine est alors partiellement dépliée et la chaîne A est transloquée dans le cytosol par le translocon Sec61p qui sert normalement à transloquer les protéines néoformées dans le RE ou à transporter les protéines mal repliées hors du RE et vers le cytoplasme pour y être dégradées. La ricine est capable d'échapper à cette protéolyse ce qui lui permet de se fixer sur le ribosome avec une grande efficacité et de cliver l'adénine en position 4324 (ci-après A4324) de l'ARN 28S de la sous-unité 60S du ribosome. Elle peut ainsi inactiver jusqu'à 2000 ribosomes par minute.

La ricine est une cytotoxine qui peut être facilement extraite en grande quantité. Sa toxicité diffère selon les voies d'introduction : par voie digestive, parce qu'elle est peu absorbée ou inactivée par les enzymes digestives, elle est environ 1000 fois moins toxique que par voie pulmonaire (inhalation) ou parentérale. Les symptômes d'intoxication sont nombreux et dépendent de la voie d'introduction, ils apparaissent en quelques heures et peuvent conduire à la mort en 2 à 3 jours. Il n'existe pas d'antidote en cas d'intoxication, le traitement étant essentiellement symptomatique. Comme la ricine est également très soluble dans l'eau et peut se disperser sous forme d'aérosol, cette toxine est considérée comme un agent bioterroriste majeur (agent de la catégorie B sur la liste du centre des contrôles des épidémies américain (CDC, Atlanta)).

Au vu du potentiel d'utilisation de la ricine, d'autres toxines intracellulaires ou de virus comme arme biologique, il est donc impératif de disposer de contre-mesures médicales inhibant l'action de toxine ou de virus.

Pour contrer la menace posée par la ricine, plusieurs types d'antitoxines ont été développés : anticorps neutralisants, inhibiteurs de l'activité enzymatique (petites molécules et analogues de substrat, mimes solubles de récepteurs).

Les inhibiteurs de l'activité enzymatique ont été décrits à la suite de l'élucidation du mécanisme d'action de la ricine étudié en premier lieu par Robertus *et al.* (Lord, J.M.; Roberts, L.M.; Robertus, J.D. *FASEB J.* **1994**, *8*, 201). Cet auteur a décrit le mécanisme de dépurination de la ricine, la N-glycosylase s'attaquant à l'ARN28S du ribosome. Après coupure d'une base adénine (A4324), l'ARNᵣ obtenu ne va plus pouvoir fixer les facteurs d'élongation nécessaires au mouvement du ribosome le long de l'ARNₘ, ce qui arrête la synthèse protéique et cause la mort de la cellule.

L'adénosine ciblée est stabilisée au niveau du site actif par formation de liaisons H entre le cycle purique de l'adénosine et certains résidus de RTA: valine en position 81 (V81), acide glutamique en position 177 (E177) et l'arginine en position 180 (R180).

R180 va permettre ensuite une protonation partielle de la base adénine, affaiblissant la liaison entre la base et le ribose. L'adénine est alors libérée et l'ion oxonium formé, stabilisé par E177, est piégé par l'eau activée par R180, formant ainsi le ribose.

Ainsi parmi les inhibiteurs de l'activité enzymatique, on compte :
- *les petites molécules :* ce sont des molécules qui vont par exemple se fixer sur la ricine au niveau de son site catalytique et empêcher la dépurination des ribosomes. Des études de modélisation moléculaire ont permis au groupe de Robertus de découvrir le premier inhibiteur de l'activité enzymatique de la ricine : l'acide ptéroïque ainsi qu'un dérivé représentés ci-dessous.

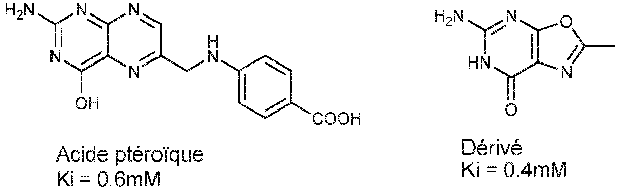

Cependant, l'acide ptéroïque, ainsi que son dérivé, sont des inhibiteurs médiocres de l'activité N-glycosylase avec une constante d'inhibition de l'ordre de 0,6 mM. (Miller, D.; Ravikumar, K.; Shen, H.; Suh, J.; Kerwin, S.; Robertus, J.D. J. Med. Chem. 2002, 45, 90. Yan, X. et al. J. Mol. Biol. 1997, 266, 1043).

Comme la ricine reconnaît une séquence bien précise au niveau de l'ARN28S, la boucle SRL (*Sarcin-Ricin Loop*), des inhibiteurs ont été conçus en se basant sur cette séquence nucléotidique. Il s'agit d'analogues de l'état de transition du substrat naturel de la ricine qui possèdent des groupements non clivables au niveau de l'adénine cible. Les études de Schramm (Schramm, V.L. et al. Biochemistry 2001, 40 (23), 6845 ; Roday, S. et al. Biochemistry 2004, 43, 4923) ont ainsi permis d'identifier différents inhibiteurs dont un des meilleurs composés (P14) est représenté ci-dessous. Cet ARN modifié est capable d'inhiber *in vitro* l'activité enzymatique avec un Kᵢ de 0,18 µM.

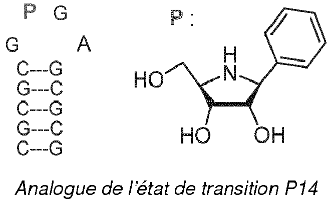

De plus, le développement d'**oligonucléotides circulaires** dérivés de la séquence de la boucle SRL s'est concrétisé par l'obtention de molécules inhibitrices de RTA à des concentrations micro- et nanomolaires (Sturm, M.B.; Roday S.; Schramm, V.L. J. Am. Chem. Soc., 2007, 129, 5544-5550).

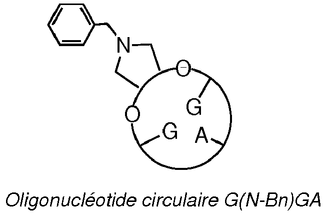

Des méthodes de sélection *in vitro* ont également été utilisées pour générer des ligands ARN, ou **aptamères**, spécifiques de la chaîne catalytique RTA. Ces aptamères ne présentent pas de ressemblance avec le substrat natif de RTA (boucle SRL) et ne sont pas dépurinés par la ricine. Cette étude a permis d'identifier un aptamère de 31 nucléotides (31 RA) capable d'interagir avec RTA (complexe de haute affinité, Kd = 7,3 nM) et d'inhiber par compétition la dépurination des ribosomes (IC₅₀ = 100 nM) (Hesselberth, J. R.; Miller, D.; Robertus, J.D.; Ellington, A. D. J. Biol. Chem. 2000, 275, 4937).

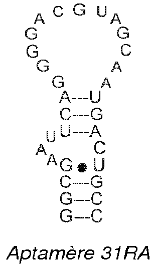

Le potentiel thérapeutique de l'ensemble de ces inhibiteurs enzymatiques semble néanmoins faible. En effet, il s'agit des composés actifs seulement sur des tests enzymatiques ; aucune protection cellulaire ou sur animal n'a été reportée pour ces molécules. D'autre part, ces molécules présentent des défauts tels qu'une faible efficacité pour ce qui est de l'acide ptéroïque et de ses dérivés, ou une instabilité dans des milieux biologiques et peu d'efficacité pour pénétrer les cellules, pour les dérivés d'ARN.

Des anticorps monoclonaux neutralisants ont également été développés. Ainsi des anticorps dirigés contre la chaîne RTB sont capables de protéger des souris intoxiquées avec 10 DL50. (Lemley, P.V.; Amanatides, P.; Wright, D.C. Hybridoma 1994, 13, 417. Guo, J.W. ; Shen, B.F. ; Feng, J.N. ; Sun, Y.X. ; Yu, M. ; Hu, M.R. Hybridoma 2005, 24, 263. Furukawa-Stoffer, T.L.; Mah, D.C.; Cherwonogrodzky, J.W.; Weselake, R.J. Hybridoma 1999, 18, 505).

Plusieurs études sont en cours afin de développer des anticorps « humanisés » neutralisants utilisables en cas d'intoxication à la ricine. Cependant, l'immunothérapie présente plusieurs désavantages : (i) son efficacité est liée à sa rapidité d'administration car les anticorps ne peuvent pas secourir les cellules intoxiquées, ils n'agissent que sur la ricine extracellulaire et (ii) elle semble peu efficace en cas d'intoxication par voie aérienne ou digestive, les anticorps ne pouvant pas atteindre l'épithélium pulmonaire ou digestif touché.

Des études basées sur l'utilisation de mimes solubles de récepteurs pour piéger la ricine ont également été menées. Il s'agit de dérivés de sucres (dont des dendrimères) sans effet inhibiteur supérieur à celui du lactose seul (Rivera-Sagredo, A.; Solis, D.; Diaz-Mauriro, T.; Jimenez-Barbero, J.; Martin-Lomes, M. Eur. J. Biochem. 1991, 197, 217. et Dawson, R.M.; Alderton, M.R.; Wells, D.; Hartley, P.G. J. Appl. Toxicol. 2006, 26, 247).

| | ***Inhibiteurs*** | ***IC₅₀ (mM)*** |
|---|---|---|
| ***1*** | 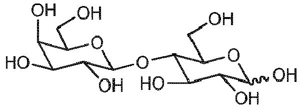 | 0.74 |
| | Lactose | |
| ***2*** | 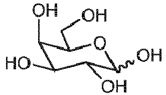 | 1.39 |
| | Galactose | |
| ***3*** | Dendrimère de 3^{ème} génération. Sucres terminaux : galactoses | 1.16 |
| ***4*** | Polymère linéaire dispersé. Sucres terminaux : galactoses | 0.42-0.85 |
| ***5*** | 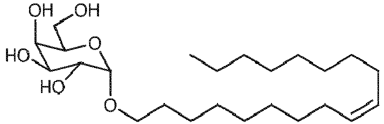 | -- |
| | Galactose avec chaîne lipidique anomérique | |

### Des dérivés de sucre comme inhibiteurs faibles de la ricine

D'autres sucres portant en position anomérique une chaîne lipidique non hydrosoluble ont été aussi synthétisés. Ils forment un gel lyotropique auto-assemblé qui est capable de séquestrer la ricine à l'aide des surfactants à base de galactose.

Un certain nombre de vaccins ont été décrits notamment dans des brevets de l'US ARMY. Ils revendiquent une protection vis-à-vis de la ricine *via* l'administration d'une quantité immunogénique de dérivés de RTA ou RTB (US 6,869,787).

Cependant, une approche vaccinale pour contrer les effets de la ricine ne semble pas à l'heure actuelle une réponse efficace contre cette menace.

En effet, seules quelques catégories de personnes, identifiées comme potentiellement exposées à la ricine, pourraient être vaccinées de manière préventive. Il n'est pas envisagé en l'état de vacciner la population contre ce bio-agent.

Plus récemment, Haslam *et al.* (Saenz, J.B.; Doggett, T.A.; Haslam, D,B. Identification and Characterization of Small Molecules That Inhibit Intracellular Toxin Transport", Infect. Immun. 2007, 75, 4552-4561) ont décrit un criblage à haut débit sur test cellulaire (cellules Vero de rein de singe) de recherche d'inhibiteurs de la ricine et présenté les composés suivants :

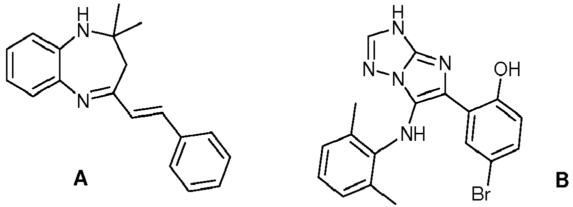

Le mode d'action de ces composés n'est pas précisé, mais ces molécules semblent bloquer le transport de la toxine à l'intérieur des cellules (endosomes et appareil de Golgi). Cependant, ces composés, en particulier le composé **A**, dérivé de benzodiazépine, ont été testés et il a été constaté qu'ils ne protègent pas les cellules épithéliales pulmonaires humaines A549, à l'inverse des composés objets de la présente invention.

Ainsi, à ce jour, ces stratégies n'ont pas permis d'identifier des composés capables de protéger efficacement des cellules ou des animaux exposés à la ricine. Les anticorps qui sont efficaces en cas d'injection de la ricine, ne le sont pas en cas d'inhalation ou d'ingestion.

En conclusion, aucun traitement spécifique n'est encore disponible chez l'homme pour lutter contre les intoxications à la ricine.

Dans cette optique, les Inventeurs ont identifié par criblage à haut débit des molécules capables de protéger des cellules en culture mises en contact avec la ricine. Le mode d'action de ces composés reste inconnu même s'ils semblent agir au niveau cellulaire -et non pas directement sur la toxine- probablement en modifiant une ou plusieurs étapes du routage intracellulaire de la ricine. L'optimisation chimique de ces composés a permis de progresser vers des composés ayant une capacité de protection cellulaire supérieure.

Il convient de souligner que ces composés sont les premiers inhibiteurs actifs sur cellules épithéliales humaines pulmonaires et digestives vis-à-vis de l'activité toxique de la ricine identifiés à ce jour.

Etant donné que les composés identifiés agissent au niveau cellulaire en modifiant le routage intracellulaire de la ricine, ces composés peuvent également être capables de bloquer l'internalisation d'autres toxines AB et de virus. En effet, les toxines AB et les virus (Sieczkarski, S.B., Whittaker, G.R. Dissecting virus entry via endocytosis, J. Gen. Virol.. 2002, 83, 1535) exploitent des voies de trafic cellulaire en partie communes avec celles utilisées par la ricine. Ainsi, une protection cellulaire a été obtenue vis-à-vis de la toxine diphtérique et la vérotoxine-2 (toxine « Shiga-like ») en présence de certains des composés identifiés par criblage.

La présente invention a ainsi pour objet des composés ayant la propriété de protéger les cellules eucaryotes des effets de toxines à activité intracellulaire, telles que la ricine, les toxines botuliques, les toxines diphtériques, les toxines du charbon, la toxine cholérique, la toxine pertussique, la toxine de Shiga (vérotoxine-2), les toxines thermolabiles d'*Escherichia coli,* les grandes toxines clostridiales, les facteurs dermonécrotiques et de virus utilisant la voie d'internalisation pour infecter les cellules, par exemple, les virus à ARN, les *Flaviviridae* (comme la dengue, la fièvre jaune), les *Orthomyxoviridae* (comme la grippe) ou encore les *Rhabdoviridae* (comme la rage). Cette propriété est particulièrement avantageuse puisque lors d'une intoxication à la ricine par voie respiratoire (inhalation) ou par absorption (ingestion), ces cellules sont les premières en contact avec la toxine.

En premier lieu, l'invention se rapporte à l'utilisation de composés de formule générale (I) : 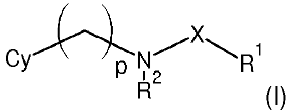 dans laquelle :

Cy représente un groupement choisi parmi : 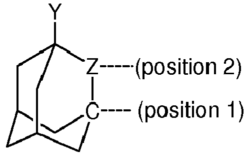 ou 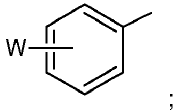 **W** est choisi parmi un atome d'hydrogène ou un atome d'halogène, **Y** est choisi parmi un atome d'hydrogène ou une fonction hydroxyle et **Z** est un atome de carbone ou une liaison (Cy est alors un noyau noradamantyle),
étant entendu que quand **Cy** est un noyau adamantyle, la chaîne 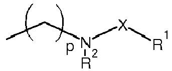 lui est attachée en position 1 ou 2,
**p** représente 0 ou 1 ;
**X** représente soit :
   - une liaison ;
   - une chaîne alkyle en C₁-C₆, éventuellement insaturée, éventuellement ramifiée, éventuellement substituée par un radical phényle, une fonction acide et/ou un radical alkylester en C₁-C₃ ; ladite chaîne étant éventuellement interrompue par un atome d'oxygène ;
   - -CO-, -O-CO-, -CO-NH- ou 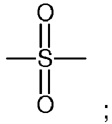
**R¹** représente un radical de 1 à 21 atomes de carbone éventuellement ramifié et/ou cyclique, saturé ou non, dont un ou plusieurs des atomes de carbone peut être remplacé par un atome d'azote, d'oxygène et/ou de soufre ; ledit radical étant éventuellement mono ou bi-substitué par un atome d'halogène, une fonction -COOH, -OH, - NO₂, un radical alkyl en C₁-C₃, un radical alcoxy en C₁-C₃ ou un radical acyloxy en C₁-C₃ ;
**R²** représente soit une liaison, soit est choisi parmi un atome d'hydrogène, un radical alkyl en C₁-C₃, éventuellement insaturé, éventuellement ramifié, un radical acyl en C₂-C₄ ou le radical 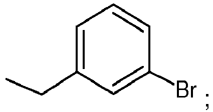 étant entendu que lorsque **R²** est une liaison alors l'atome d'azote portant R² et **X** ou l'atome de carbone adjacent (lorsque p = 1) sont liés par une double liaison,
étant entendu que **X**, **R¹** et **R²** peuvent former avec l'atome d'azote adjacent un cycle imidazole, oxazole, triazole, benzimidazol, éventuellement partiellement saturé tel notamment, que le dihydroimidazole, éventuellement substitué par un radical phényle ou pyridine ;
et leurs sels pharmaceutiquement acceptables
pour la préparation d'une composition pharmaceutique destinée à la prévention et/ou au traitement des intoxications à au moins une toxine à mode d'action intracellulaire ou à au moins un virus utilisant la voie d'internalisation pour infecter les cellules eucaryotes de mammifères.

L'invention se rapporte également aux sels pharmaceutiquement acceptables de ces composés tels que chlorhydrates, bromhydrates, sulfates ou bisulfates, phosphates ou hydrogénophosphates, acétates, oxalates, benzoates, succinates, fumarates, maléates, lactates, citrates, tartrates, gluconates, méthanesulphonates, benzène-sulphonates et paratoluène-sulphonates.

Par atome d'halogène, on entend les éléments chimiques du groupe VII du tableau périodique des éléments, notamment, le fluor, le chlore, le brome et l'iode. Les atomes d'halogène préférés pour la mise en oeuvre de la présente invention sont le brome (Br) et le fluor (F).

Le terme chaîne ou radical alkyl en C₁-C₃ désigne, respectivement, une chaîne ou un radical hydrogénocarboné, linéaire ou ramifié ; on peut citer par exemple le méthyle, l'éthyle, le propyle ou l'isopropyle.

Par radical alcoxy en C₁-C₃, on entend un radical -OCₙH₂ₙ₊₁, n étant un nombre entier compris entre 1 et 3 ; on peut citer par exemple le radical méthoxy, éthoxy, propyloxy, isopropyloxy. De préférence n vaut 1.

Par radical acyloxy en C₁-C₃, on entend un radical -O(CO)CₙH₂ₙ₊₁ ou - (CO)OCₙH₂ₙ₊₁, n étant un nombre entier compris entre 1 et 3 ; on peut citer par exemple le radical acétyle. De préférence n vaut 1.

Par radical acyl en C₁-C₃, on entend un radical -(CO)CₙH₂ₙ₊₁, n étant un nombre entier compris entre 1 et 3.

Selon un mode de réalisation particulier de l'invention, le radical R¹ de 1 à 21 atomes de carbone éventuellement ramifié et/ou cyclique, saturé ou non, dont un ou plusieurs des atomes de carbone peut être remplacé par un atome d'azote, d'oxygène et/ou de soufre, est choisi parmi : un radical alkyle de 1 à 8 atomes de carbone linéaire ou ramifié, saturé ou non, un radical cyclique à 5 ou 6 atomes de carbone, saturé ou non, un radical bicyclique à 9 ou 10 atomes de carbone, saturé ou non, un radical tricyclique à 14 atomes de carbone, saturé ou non, un radical hétérocyclique à 5 atomes, saturé ou non, un hétérocycle à 6 atomes, saturé ou non, un radical bihétérocyclique à 9 ou 10 atomes, saturé ou non.

Plus spécifiquement, le radical alkyle de 1 à 8 atomes de carbone linéaire ou ramifié, saturé ou non, est choisi parmi : un radical tert-butyle, 2,4,4-trimethylpentanyle, 3-hydroxy-2-méthylpropanoate, l'hydroxyméthylpropane-1,3-diol.

Les radicaux cycliques listés ci-dessus sont préférentiellement choisis parmi les radicaux : cyclopentylméthanol, cyclométhanoate, phényl, cyclohexyl, pyridine, furane, thiophène, imidazole, quinoline, indole, benzofurane, adamantyle, naphthalène, anthracène, les radicaux cycliques suivants : 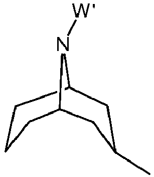 avec **W**' est -H ou -COOCₙH₂ₙ₊₁, avec n compris entre 1 et 3,

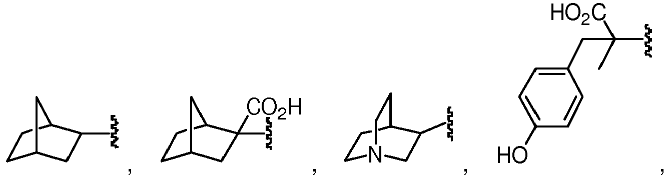

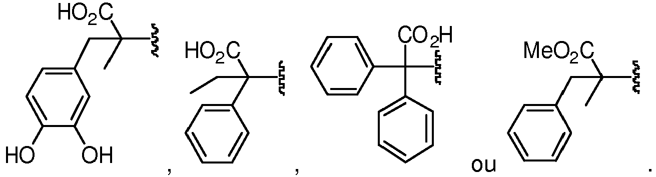

De façon préférée, les composés de formule générale (I) sont tels que R¹ est un radical phényl éventuellement substitué et/ou X est -CH₂- et/ou R² est un atome d'hydrogène et/ou W représente un atome de Br.

Selon une variante particulière de l'invention, les composés de formule générale (I) sont tels que **R¹** est le radical : 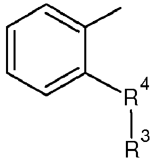 dans lequel :
- **R³** est choisi parmi un radical cyclique à 5 ou 6 atomes de carbone, saturé ou non ; un radical bicyclique à 9 ou 10 atomes de carbone, saturé ou non ; un radical tricyclique à 14 atomes de carbone, saturé ou non ; un radical hétérocyclique à 5 atomes, saturé ou non ; un radical hétérocycle à 6 atomes, saturé ou non ; un radical bihétérocyclique à 9 ou 10 atomes, saturé ou non ; lesdits radicaux étant optionnellement substitués par au moins un atome d'halogène, -NO₂, -OH ou un radical alkyl en C₁-C₃ ; les radicaux sont préférentiellement choisis parmi les radicaux : phényl, furane, indole et thiophène ; et
- **R⁴** est choisi parmi -CO-O-, -N=CH- ou -NH-CH₂-.

Selon une autre variante de l'invention, les composés de formule générale (I) sont définis par la formule générale (I') : 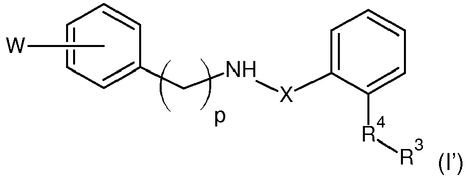 où **W**, **p**, **R³** et **R⁴** sont tels que définis précédemment et **X** est soit une liaison, soit -CO-.

Plus particulièrement, les composés de formule générale (I) sont choisis parmi :

| | | |
|---|---|---|
| 1 | *N*-benzyladamantylamine | 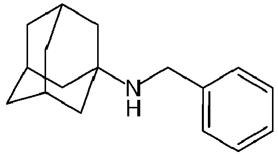 |
| 2 | *N*-(2-bromobenzyl)adamantylamine | 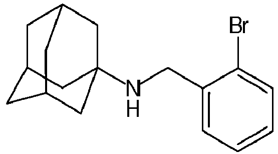 |
| 3 | *N*-(3-bromobenzyl)adamantylamine | 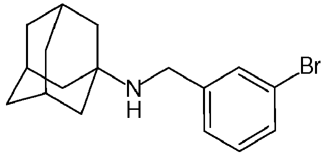 |
| 4 | *N*-(4-bromobenzyl)adamantylamine | 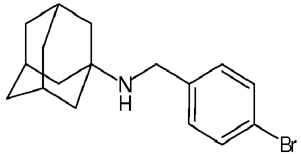 |
| 5 | *N*-(3-fluorobenzyl)adamantylamine | 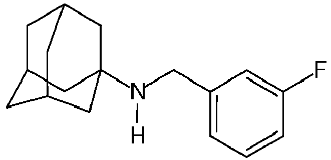 |
| 6 | *N*-(3-hydroxybenzyl)adamantylamine | 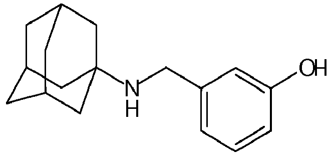 |
| 7 | *N*-(2-methoxybenzyl)adamantylamine | 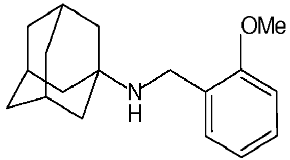 |
| 8 | *N*-(3-methoxybenzyl)adamantylamine | 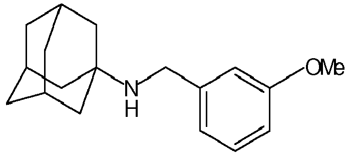 |
| 9 | *N*-(4-methoxybenzyl)adamantylamine | 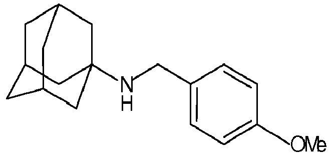 |
| 10 | *N*-(2-nitrobenzyl)adamantylamine | 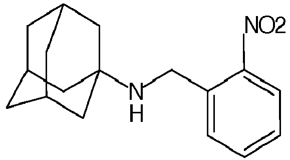 |
| 11 | *N*-(4-nitrobenzyl)adamantylamine | 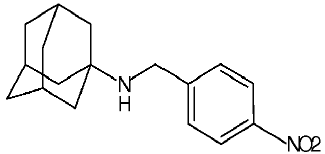 |
| 12 | *N*-(4-carbethoxybenzyl)adamantylamine | 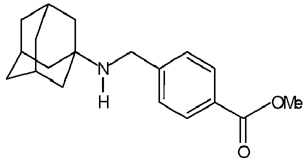 |
| 13 | 4-bromo-2-((1-adamantylino)methyl)phenol | 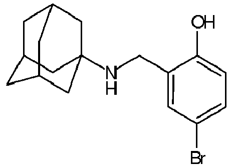 |
| 14 | *N*-(2-bromo-5-methoxybenzyl)adamantylamine | 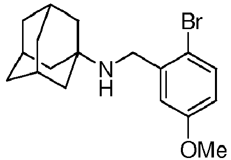 |
| 15 | *N*-[(2-methoxy-5-bromo)benzyl]adamantylamine | 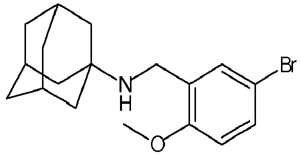 |
| 16 | *N*-((pyridin-2-yl)methyl)adamantylamine | 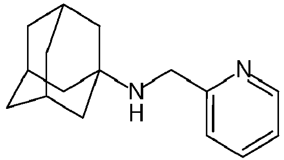 |
| 17 | *N*-((pyridin-3-yl)methyl)adamantylamine | 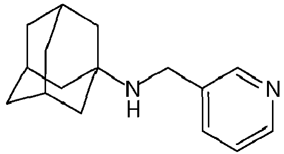 |
| 18 | *N*-((pyridin-4-yl)methyl)adamantylamine | 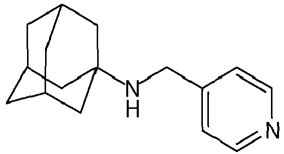 |
| 19 | *N*-((5-methylfuran-2-yl)methyl)adamantylamine | 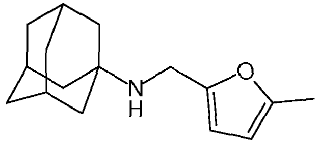 |
| 20 | *N*-((5-methylthiophen-2-yl)methyl)cyclohexanamine | 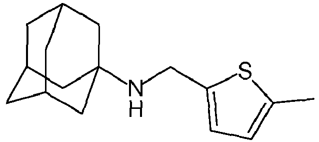 |
| 21 | *N*-[(3-furyl)methyl]adamantylamine | 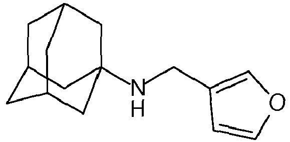 |
| 22 | *N*-((1-methyl-1*H*-imidazol-5-yl)methyl)adamantylamine | 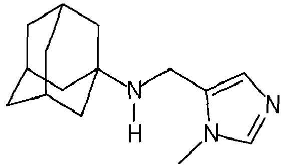 |
| 23 | *N-*[(5*-N-*methylimidazolyl)methyl]adamantylamine | 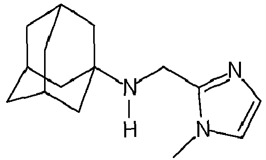 |
| 24 | Benzo[d][1,3]dioxol-4-yl)methyl)adamantylamine | 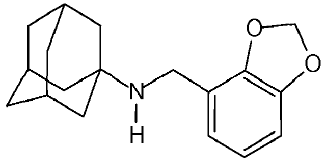 |
| 25 | *N*-((5-nitrobenzo[d][1,3]dioxol-6-yl)methyl)adamantylamine | 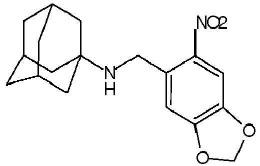 |
| 26 | *N*-((quinolin-3-yl)methyl)adamantylamine | 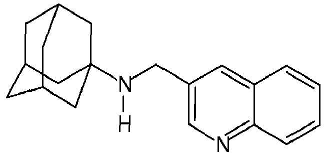 |
| 27 | *N*-((quinolin-4-yl)methyl)adamantylamine | 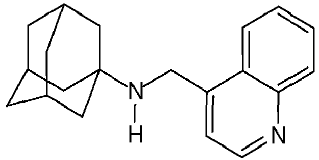 |
| 28 | *N*-((1-methyl-1*H*-indol-2-yl)methyl)adamantylamine | 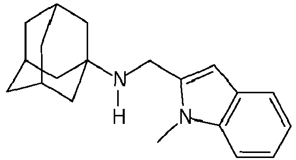 |
| 29 | *N*-phenethyladamantylamine | 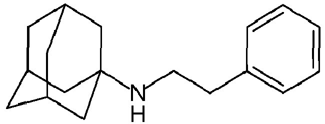 |
| 30 | *N*-(3-phénylpropyl)adamantylamine | 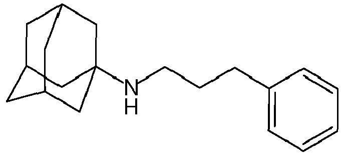 |
| 31 | *N*-(2-(benzyloxy)ethyl)adamantylamine | 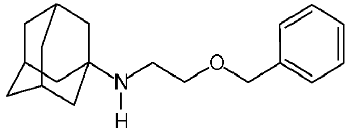 |
| 32 | *N*-cinnamyladamantylamine | 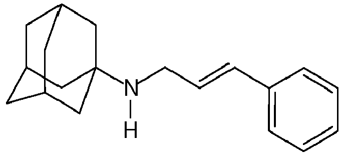 |
| *33* | *N*-methyl(3-bromobenzyl)adamantylamine | 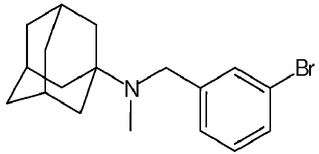 |
| *34* | *N*-benzyl-2-adamantylamine | 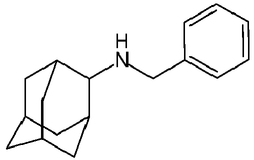 |
| *35* | *N*-(2-bromobenzyl)-2-adamantylamine | 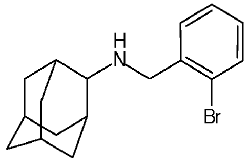 |
| *36* | *N*-(3-bromobenzyl)-2-adamantylamine | 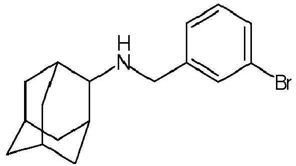 |
| *37* | *N*-(4-bromobenzyl)adamantylamine | 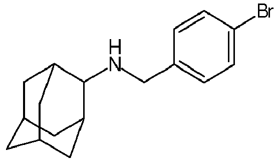 |
| *38* | *N*-(2-fluorobenzyl) -2-adamantylamine | 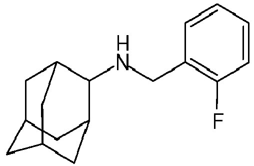 |
| *39* | *N*-(3-fluorobenzyl)-2-adamantylamine | 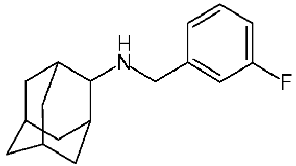 |
| *40* | *N*-(4-fluorobenzyl)-2-adamantylamine | 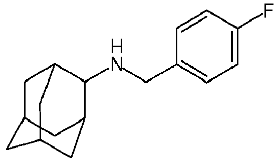 |
| *41* | *N*-(3-hydroxybenzyl)-2-adamantylamine | 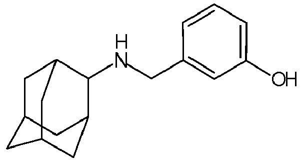 |
| *42* | *N*-(2-methoxybenzyl)-2-adamantylamine | 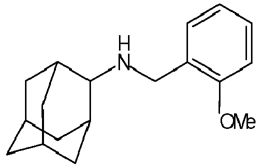 |
| *43* | *N*-(3-methoxybenzyl)-2-adamantylamine | 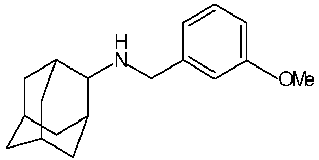 |
| *44* | *N*-(4-methoxybenzyl)-2-adamantylamine | 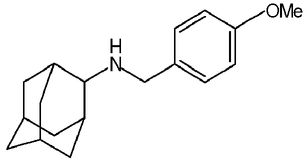 |
| *45* | *N*-(2-nitrobenzyl)-2-adamantylamine | 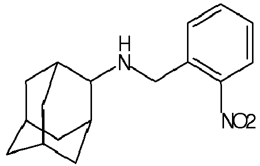 |
| *46* | *N*-(4-nitrobenzyl)-2-adamantylamine | 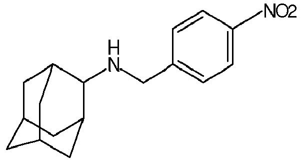 |
| *47* | *N*-(4-carbethoxybenzyl)-2-adamantylamine | 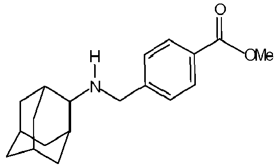 |
| *48* | 4-bromo-2-((2-adamantylamino)methyl)phenol | 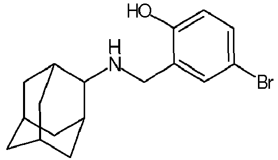 |
| *49* | *N*-(2-bromo-5-nitrobenzyl)-2-adamantylamine | 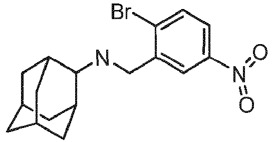 |
| *50* | *N*-(5-bromo-2-methoxybenzyl)-2-adamantylamine | 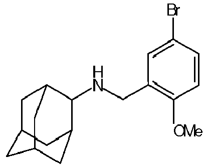 |
| *51* | *N*-(5-fluoro-2-nitrobenzyl)-2-adamantylamine | 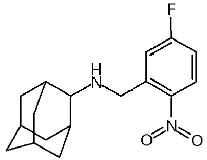 |
| *52* | N-(2,5-difluorobenzyl)-2-adamantylamine | 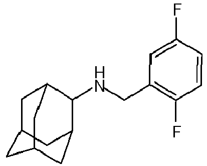 |
| *53* | *N*-((pyridin-2-yl)methyl)-2-adamantylamine | 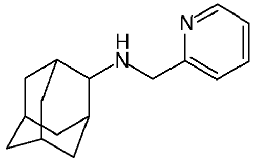 |
| *54* | *N*-((pyridin-3-yl)methyl)-2-adamantylamine | 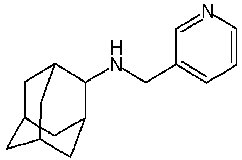 |
| *55* | *N*-((pyridin-4-yl)methyl)-2-adamantylamine | 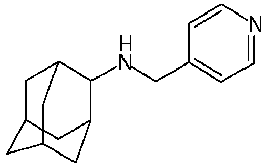 |
| *56* | *N*-((5-methylfuran-2-yl)methyl)-2-adamantylamine | 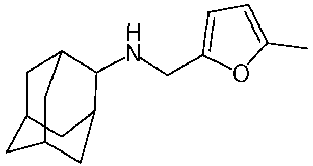 |
| *57* | *N*-((5-methylthiophen-2-yl)methyl)-2-adamantylamine | 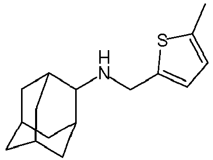 |
| *58* | *N*-((furan-3-yl)methyl)-2-adamantylamine | 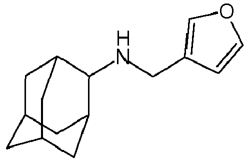 |
| *59* | *N*-((1-methyl-1*H*-imidazol-5-yl)methyl)-2-adamantylamine | 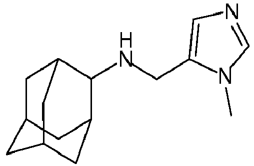 |
| *60* | *N*-[(5-*N*-methylimidazolyl)methyl]-2-adamantylamine | 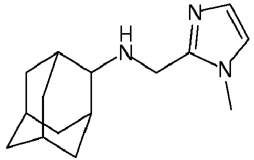 |
| *61* | Benzo[d][1,3]dioxol-4-yl)methyl)-2-adamantylamine | 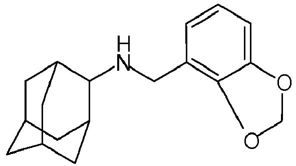 |
| *62* | *N*-((5-nitrobenzo[d][1,3]dioxol-6-yl)methyl)-2-adamantylamine | 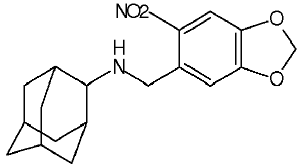 |
| *63* | *N*-((quinolin-3-yl)methyl)-2-adamantylamine | 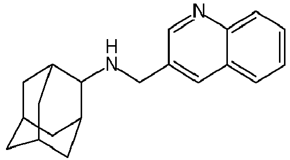 |
| *64* | *N*-((quinolin-4-yl)methyl)-2-adamantylamine | 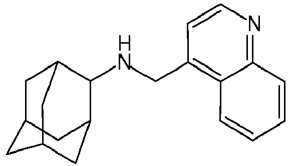 |
| *65* | *N*-((1-methyl-1*H*-indol-2-yl)methyl)-2-adamantylamine | 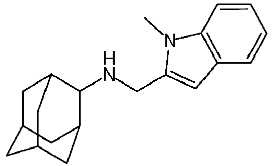 |
| *66* | *N*-(1-(3-bromophényl)ethyl)-2-adamantylamine | 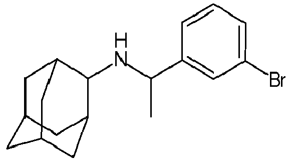 |
| *67* | *N*-benzhydryl-2-adamantylamine | 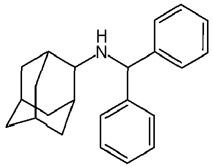 |
| *68* | *N*-(2-(benzyloxy)ethyl)-2-adamantylamine | 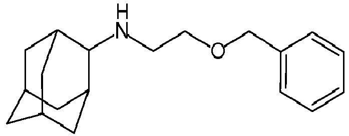 |
| *69* | *N*-(phenylpropyl)-2-adamantylamine | 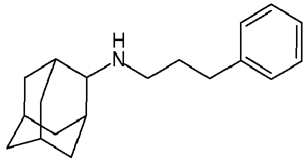 |
| *70* | *N*-(1-phenylethyl)-2-adamantylamine | 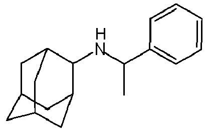 |
| *71* | *N*-(1-(pyridin-2-yl)ethyl)-2-adamantylamine | 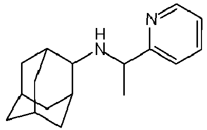 |
| *72* | *N*-(-2-adamantylmethyl)-1-(adamantyl)ethanamine | 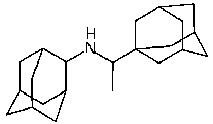 |
| *73* | *N*-methyl(-3bromobenzyl)-2-adamantylamine | 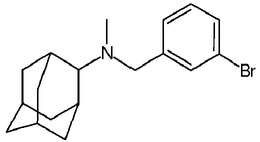 |
| *74* | *N*-(3-bromobenzyl)-2-methylpropan-2-amine | 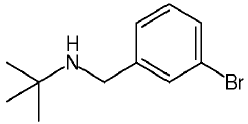 |
| *75* | *N*-(3-bromobenzyl)-2,4,4-trimethylpentan-2-amine | 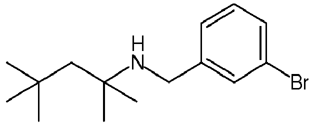 |
| *76* | 2-(3-bromobenzylamino)-3-hydroxy-2-methylpropanoic acid | 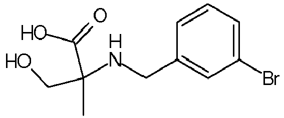 |
| *77* | 2-(3-bromobenzylamino)-2-(hydroxymethyl)propane-1,3-diol | 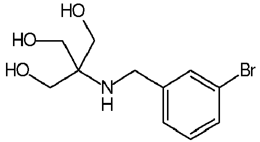 |
| *78* | *N*-(3-bromobenzyl)cyclohexanamine | 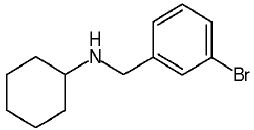 |
| *79* | 4-(3-bromobenzylamino)cyclohexanol | 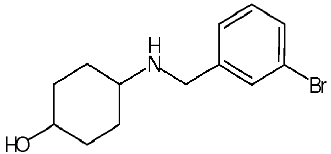 |
| *80* | *N*-(3-fluorobenzyl)cyclohexylamine | 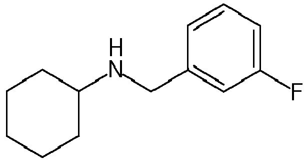 |
| *81* | *4-(3-fluorobenzylamino)cyclohexanol* | 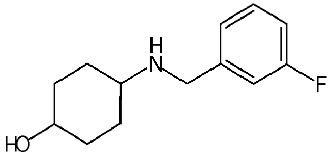 |
| *82* | (1-(3-bromobenzylamino)cyclopentyl)methanol | 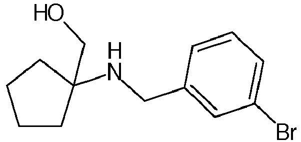 |
| *83* | 1-(3-bromobenzylamino)cyclopentanecarboxyli c acid | 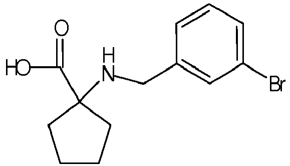 |
| *84* | *N*-(3-bromobenzyl)bicyclo[2.2.1]heptan-2-amine | 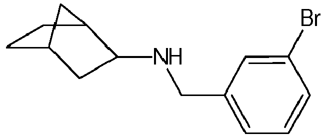 |
| *85* | 2-(3-bromobenzylamino)bicyclo[2.2.1]heptane-2-carboxylic acid | 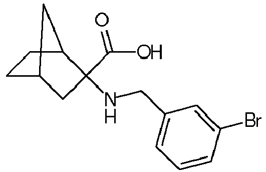 |
| *86* | *N*-(3-bromobenzyl)-noradamantylamine | 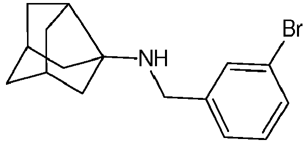 |
| *87* | *N*-(3-bromobenzyl)-1-hydroxy-2-adamantylamine | 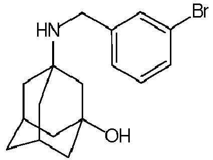 |
| *88* | *N*-(3-bromobenzyl)-*N-*((benzo[*d*][1,3]dioxol-6-yl)methyl)(3-bromophenyl)methanamine | 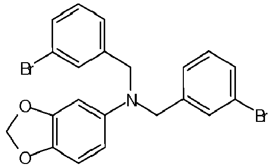 |
| *89* | 2-(3-bromobenzylamino)-2-(4-hydroxybenzyl)propanoic acid | 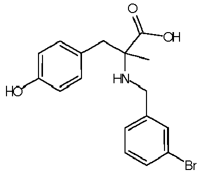 |
| *90* | 2-(3,4-dihydroxybenzyl)-2-(3-bromobenzylamino)propanoic acid | 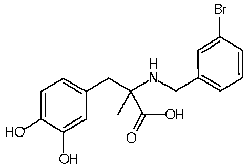 |
| *91* | 2-(3-bromobenzylamino)-2-phenylbutanoic acid | 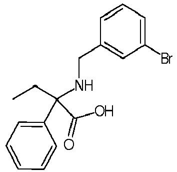 |
| *92* | 2-(3-bromobenzylamino)-2,2-diphenylacetic acid | 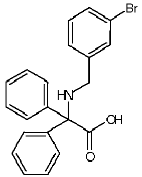 |
| *93* | methyl 2-(3-bromobenzylamino)-2-methyl-3-phenylpropanoate | 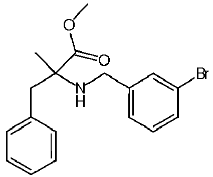 |
| *94* | methyl 3-(3-bromobenzylamino)-8-azabicyclo[3.2.1]octane-8-carboxylate | 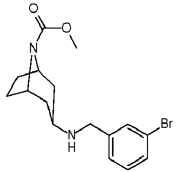 |
| *95* | *N*-(3-bromobenzyl)-9-methyl-9-azabicyclo[3.3.1]nonan-3-amine | 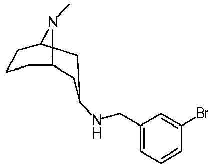 |
| *96* | *N*-(3-bromobenzyl)benzo[*d*][1,3]dioxol-5-amine | 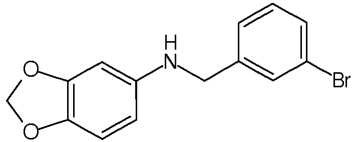 |
| *97* | 2-(3-bromobenzylideneamino)-2-(3,4-dihydroxyphenyl)propanoic acid | 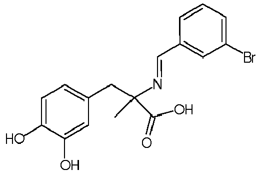 |
| *98* | *N*-benzyl(3-bromophenyl)methanamine | 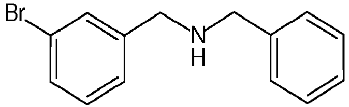 |
| *99* | bis(3-bromobenzyl)amine | 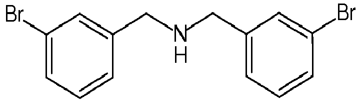 |
| *100* | *N*-(3-fluorobenzyl)(3-bromophenyl)methanamine | 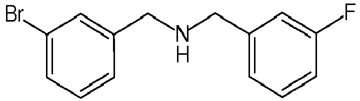 |
| *101* | *N*-(3-bromobenzyl)(1-methyl-1*H*-indol-2-yl)methanamine | 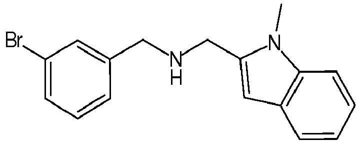 |
| *102* | *N*-(3-bromobenzyl)-1-phenylethanamine | 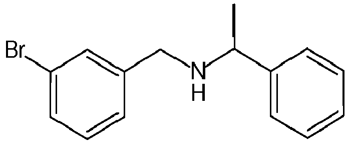 |
| *103* | *N*-(3-bromobenzyl)-1-(3-bromophenyl)ethanamine | 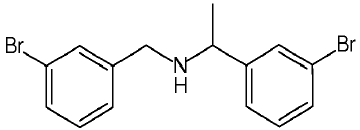 |
| *104* | *N*-(3-bromobenzyl)-1-(pyridin-2-yl)ethanamine | 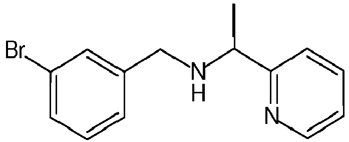 |
| *105* | *N*-(3-bromobenzyl)quinuclidin-3-amine | 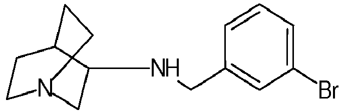 |
| *106* | (1R*,5S*)-*N*-(3-bromobenzyl)bicyclo[3.3.1]nonan-9-amine | 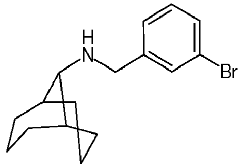 |
| *107* | *N*-(3-bromobenzyl)-8-methyl-8-aza-bicyclo[3.2.1]octan-3-amine | 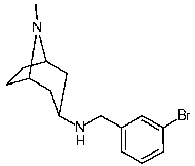 |
| *108* | *N*-(1-(3-bromophenyl)ethyl)adamantylamine | 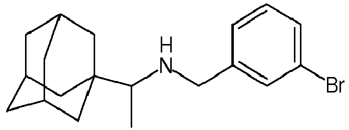 |
| *109* | *N*-(3-fluorobenzyl)noradamantylamine | 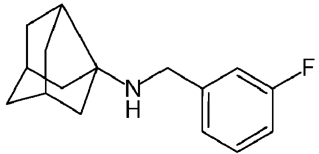 |
| *110* | *N*-(3-bromobenzyl)adamantylamine hydrochloride salt | 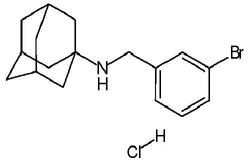 |
| *111* | *N*-(5-bromo-2-methoxybenzyl)adamantylamine hydrochloride salt | 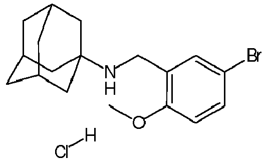 |
| *112* | *N*-(2-bromobenzyl)-2-adamantylamine hydrochloride salt | 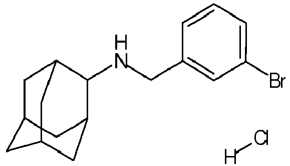 |
| *113* | (*E*)-*N*-(3-bromobenzylidene)adamantylamine | 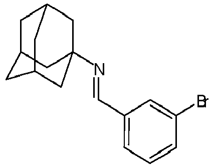 |
| *114* | (*E*)-*N*-(3-fluorobenzylidene)adamantylamine | 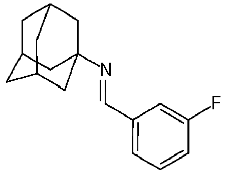 |
| *115* | (*E*)-*N*-((1-methyl-1*H*-indol-2-yl)methylene)adamantylamine | 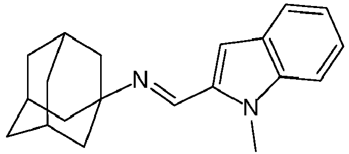 |
| *116* | (*E*)-*N*-benzylideneadamantylamine | 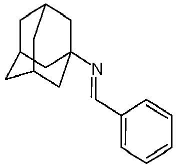 |
| *117* | (*E*)-*N*-(3-bromobenzylidene)adamantylamine | 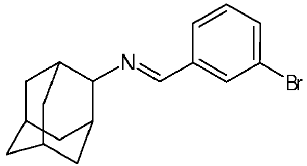 |
| *118* | (*E*)-*N*-(3-fluorobenzylidene)adamantylamine | 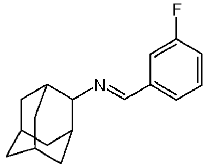 |
| *119* | (*E*)-*N*-(3-bromobenzylidene)noradamantylamine | 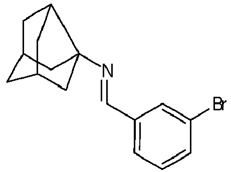 |
| *120* | (*E*)-*N*-((1-methyl-1*H*-indol-2-yl)methylene)noradamantylamine | 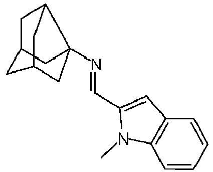 |
| *121* | *N*-1-adamantylbenzamide | 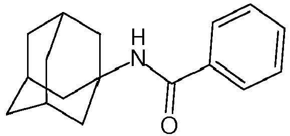 |
| *122* | *N*-2-adamantylbenzamide | 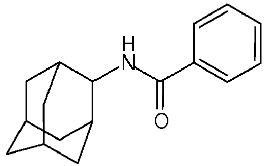 |
| *123* | phenyl *N*-adamantylcarbamate | 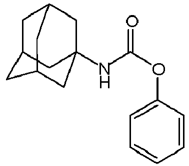 |
| 124 | *N*-adamantyl benzenesulfonamide | 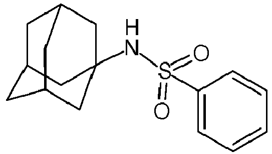 |
| *125* | *N*-adamantyl-N-(3-bromobenzyl)acetamide | 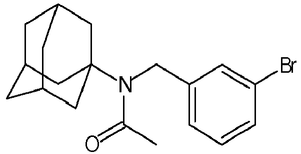 |
| *126* | phenyl *N*-2-adamantylcarbamate | 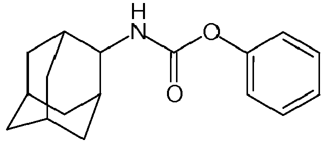 |
| *127* | *N*-adamantyl-*N*-(3-bromobenzyl)benzamide | 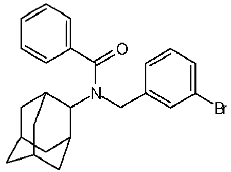 |
| *128* | *N*-2-adamantyl benzenesulfonamide | 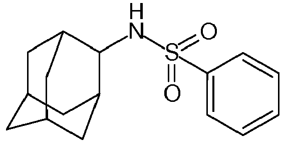 |
| *129* | 1-(adamantyl)-3-phenylurea | 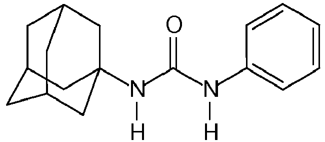 |
| *131* | 1-(adamantyl)-1-(3-bromobenzyl)-3-phenylurea | 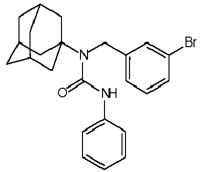 |
| *132* | 1-(2-adamantyl)-3-phenylurea | 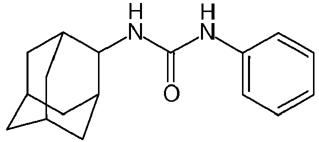 |
| *134* | 1-(adamantyl)-2,5-dihydro-1*H*-imidazole | 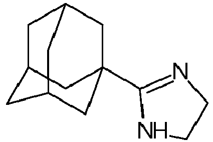 |
| *135* | 1-(adamantyl)-2,5-dihydrooxazole | 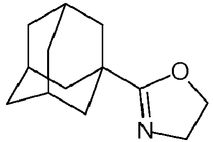 |
| *136* | 1-(adamantyl)-1-phenyl-4,5-dihydro-1*H-*imidazole | 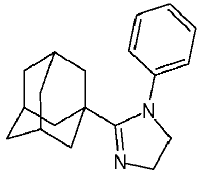 |
| *137* | 1-(adamantyl)-3a,4,5,6,7,7a-hexahydro-1*H*-benzo[d]imidazole | 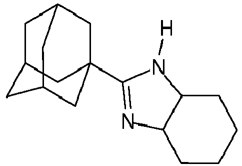 |
| *138* | *N*-(3-chlorobenzyl)adamantylamine | 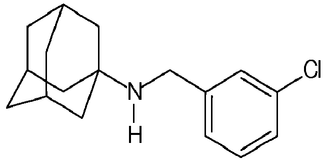 |
| *139* | *N*-(3-chlorobenzyl)-2-adamantylamine | 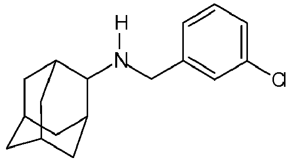 |
| *140* | *N*-(3-iodobenzyl)-2-adamantylamine | 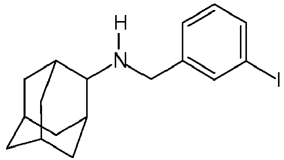 |
| *141* | *N*-(2,2-diphenylethyl)-2-adamantylamine | 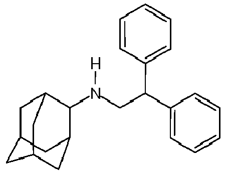 |
| *142* | *N*-(naphthalen-1-ylmethyl)-2-adamantylamine | 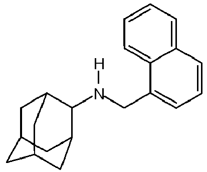 |
| *143* | *N*-(phenanthren-9-ylmethyl)-2-adamantylamine | 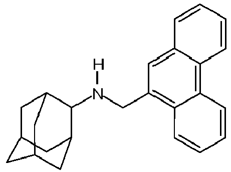 |
| *144* | 4-((adamantylamino)methyl)benzoic acid | 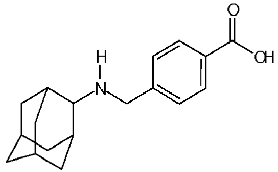 |
| *145* | adamantylamino-4-phenyl-1*H*-1,2,3-triazole | 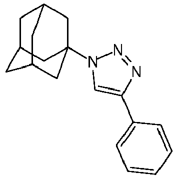 |
| *146* | adamantylamino-4-phenyl-1*H*-1,2,3-triazole | 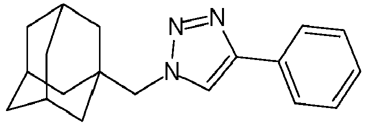 |
| *147* | 2-(adamantylamino-1*H*-1,2,3-triazol-4-yl)pyridine | 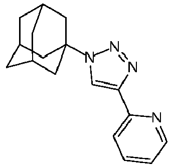 |
| *148* | *N*-(2-bromo-5-nitrobenzyl)adamantylamine | 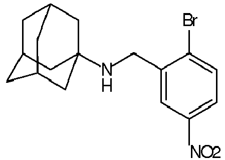 |
| *149* | 2-(3-bromobenzylideneamino)-*N-*phenylbenzamide | 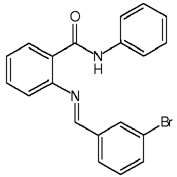 |
| *150* | 2-(3-bromobenzylamino)-*N-*phenylbenzamide | 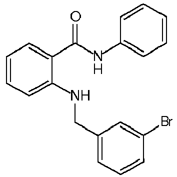 |
| *151* | 2-(3-fluorobenzylideneamino)-*N-*phenylbenzamide | 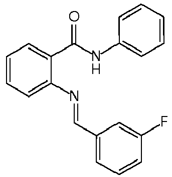 |
| *152* | (*E*)-2-((furan-2-yl)methyleneamino)-*N-*phenylbenzamide | 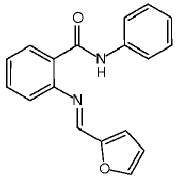 |
| *153* | (*E*)-2-((furan-3-yl)methyleneamino)-*N-*phenylbenzamide | 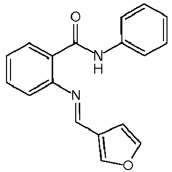 |
| *154* | (*E*)-2-((5-methylfuran-2-yl)methyleneamino)-*N*-phenylbenzamide | 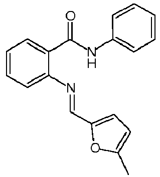 |
| *155* | (*E*)-2-(5-fluoro-2-nitrobenzylideneamino)-N-phenylbenzamide | 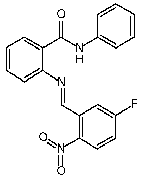 |
| *156* | (*E*)-2-(4-fluorobenzylideneamino)-*N-*phenylbenzamide | 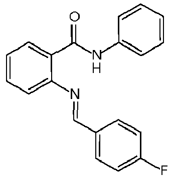 |
| *157* | (*E*)-2-(2-fluorobenzylideneamino)-*N-*phenylbenzamide | 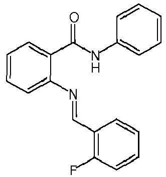 |
| *158* | (*E*)-2-((1-methyl-1*H*-indol-2-yl)methyleneamino)-*N*-phenylbenzamide | 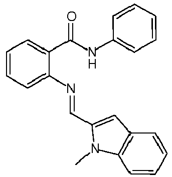 |
| *159* | (*E*)-2-(5-bromo-2-hydroxybenzylideneamino)-*N-*phenylbenzamide | 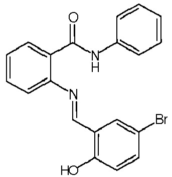 |
| *160* | 2-(2-fluorobenzylamino)-*N-*phenylbenzamide | 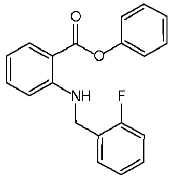 |
| *161* | (E)-2-(2-(5-methylthiophen-2-yl)vinyl)-N-phenylbenzamide | 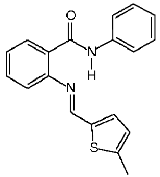 |
| *191* | *N*-cinnamyl-2-adamantylamine | 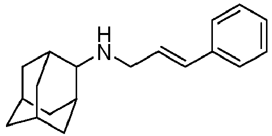 |
| *192* | N-(3-nitrobenzyl)-2-adamantylamine | 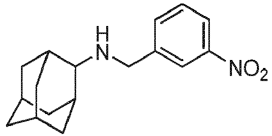 |

La présente invention se rapporte également aux composés de formule générale (I) telle que définie ci-dessus et leurs sels pharmaceutiquement acceptables en tant que tels à l'exclusion des composés 1, 6, 8, 11, 18, 29, 30, 34, 74, 78, 98, 100, 113, 114, 116, 121, 122, 124, 128, 135, 145 et 161.

Les composés dérivés d'aminoadamantanes ont été préparés par amination réductrice selon la réaction qui suit :

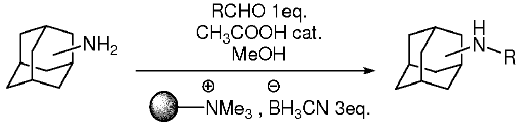

Plus spécifiquement, l'invention se rapporte au procédé de préparation des composés de formule générale (la) : 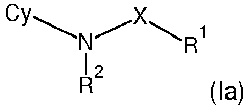 dans laquelle
- **Cy** est le noyau adamantyle avec l'atome d'azote en position 1 ou 2,
- **X** représente une chaîne alkyle en C₁-C₃, éventuellement insaturée, ramifiée, éventuellement interrompue par un atome d'oxygène ;
- **R¹** est un radical de 1 à 21 atomes de carbone éventuellement ramifié ou cyclique, saturé ou non dont un ou plusieurs des atomes de carbone peut être remplacé par un atome d'azote, d'oxygène et/ou de soufre et éventuellement mono ou bi-substitué par un atome d'halogène, une fonction -OH, -NO₂, un radical alcoxy en C₁-C₃ ou un radical acyloxy en C₁-C₃ ;
- **R²** est choisi parmi -H ou un radical alkyl en C₁-C₃,
caractérisé en ce qu'il comporte les étapes suivantes :
- ajout d'une suspension dans le méthanol de 1-adamantylamine ou de 2-adamantylamine sous agitation à un aldéhyde aromatique choisi en fonction du composé de formule générale (la) à préparer en présence de BH₃CN sur résine et d'acide acétique ;
- agitation du mélange pendant 2 jours à température ambiante.

De façon plus détaillée, les composés de formule générale (la) figurant dans les Tableaux A1 et A2 selon l'exemple 1 sont préparés dans le méthanol par traitement de la 1-adamantylamine (pour les composés du Tableau A1) ou de 2-adamantylamine (pour les composés du Tableau A2) en présence d'un aldéhyde aromatique (1 équiv.) en fonction du composé à préparer. Le cyanoborohydrure supporté est utilisé (BH₃CN sur résine, 1,5 équiv) comme réducteur en présence d'acide acétique (3 équiv.). L'ensemble est agité 2 jours à température ambiante, filtré, lavé avec du méthanol, évaporé puis purifié selon les méthodes connues de l'homme du métier.

Ce même procédé permet de préparer les dérivés de formule générale (Ib) : 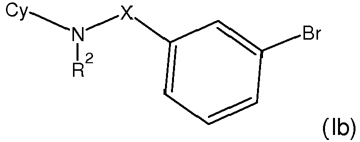 dans laquelle :
- Cy est 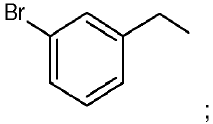
- **X** représente une chaîne alkyle en C₁-C₃, éventuellement insaturé, ramifié, éventuellement interrompu par un atome d'oxygène, -CO-, -CO-NH-, -CS-NH- ou 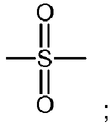
- **R²** est choisi parmi rien, -H, -CH₃ ou 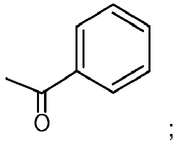 étant entendu que quand **R²** est rien alors l'atome d'azote et **X** sont liés par une double liaison.

L'invention se rapporte donc également au procédé de préparation des composés de formule générale (Ib) caractérisé en ce qu'il comporte les étapes suivantes :
- ajout d'une suspension dans le méthanol de 3-bromobenzylamine sous agitation à un aldéhyde aromatique choisi en fonction du composé de formule générale (Ib) à préparer en présence de BH₃CN sur résine et d'acide acétique ;
- agitation du mélange pendant 2 jours à température ambiante.

De façon plus détaillée, les composés (Ib) figurant dans le Tableau A3 et dans le Tableau A4 sont préparés en ajoutant sous agitation une suspension d'une amine (1 équiv.) choisie selon le composé à préparer (voir les Tableaux A3 et A4 selon l'exemple 1) dans du méthanol à un aldéhyde (1 équiv.) pour les composés du Tableau A3 ou de la 3-bromobenzylamine (1 équiv.) et un aldéhyde pour obtenir les composés du Tableau A4, en présence de 1,5 équiv de BH₃CN sur résine et d'acide acétique (3 équiv.). L'ensemble est agité 2 jours à température ambiante, filtré, lavé avec du méthanol, évaporé puis purifié selon les méthodes connues de l'homme du métier.

La formation des sels est réalisée par traitement du composé amine (correspondant au sel désiré) dans le CH₂Cl₂ avec une solution de l'acide correspondant dans un solvant (ex. HCl dans l'éther).

Le précipitat est filtré et séché sous vide pour donner le sel de l'acide (par exemple, le chlorhydrate dans le cas d'HCl). Ils figurent dans le Tableau B de l'exemple 1.

Les dérivés de 1-aminoadamantane, de 2-aminoadamantane ou de noradamantylamine de formule générale (Ic) dans laquelle : 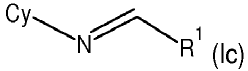 - **Cy** est 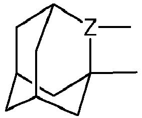 avec **Z** est un atome de carbone ou rien (noyau noradamantyle), avec l'atome d'azote en position 1 ou 2 lorsque Cy en le noyau adamantyle, - **R¹** est choisi parmi un radical phényl, un radical hétérocyclique tel que les radicaux pyridine, furane, thiophène, quinoline, indole, de préférence, le radical indole, ledit radical étant éventuellement mono ou bi-substitué par un atome d'halogène, une fonction -OH, - NO₂, un radical alcoxy en C₁-C₃ ou un radical acyloxy en C₁-C₃, un radical alkyl en C₁-C₃, de préférence, un radical méthyl ;
sont préparés comme suit: une suspension agitée dans du méthanol de 1-, 2- ou noradamantylamine (1 équiv.) est ajoutée à un aldéhyde (1 équiv.) choisi selon le composé à préparer (voir les Tableaux C1, C2 et C3 de l'exemple 1). L'ensemble est mélangé pendant deux jours à température ambiante puis évaporé.

Ainsi l'invention se rapporte au procédé de préparation des dérivés de 1-aminoadamantane, de 2-aminoadamantane ou de noradamantylamine de formule générale (Ic) caractérisé en ce qu'il comprend les étapes suivantes :
- agitation d'une suspension dans du méthanol de 1-, 2- ou noradamantylamine (1 équiv.) ;
- ajout à un aldéhyde (1 équiv.) choisi selon le composé à préparer ;
- mélange de l'ensemble pendant deux jours à température ambiante puis évaporation.

La préparation des imines est réalisée par ajout d'une solution de l'amine dans le MeOH à l'aldéhyde, l'amine et l'aldéhyde étant choisis selon l'imine à préparer, et le mélange est agité pendant 2 jours. Après évaporation et purification les imines sont obtenues.

Les imines sont réduites comme suit : à une solution de l'imine dans le MeOH est ajouté le BH₃CN sur résine (3 équiv.) et AcOH. Après 3 jours à température ambiante, le mélange est filtré, lavé au méthanol puis concentré sous vide. Le composé brut ainsi obtenu est purifié selon les méthodes classiques.

L'invention se rapporte également à procédé de préparation des imines de formule générale (I) caractérisé en ce qu'il comprend les étapes suivantes :
- ajout d'une solution d'amine dans le MeOH à un aldéhyde, ladite amine et ledit aldéhyde étant choisis en fonction du composé de formule générale (I), à préparer ;
- agitation du mélange pendant 2 jours ;
- évaporation et purification.

L'invention se rapporte encore à un procédé de préparation d'imine réduite de formule générale (I) caractérisé en ce qu'il comprend les étapes suivantes :
- ajout à une solution de l'imine obtenue selon le procédé ci-dessus dans le MeOH, le BH₃CN sur résine (3 équiv.) et AcOH ;
- traitement pendant 3 jours à température ambiante ;
- filtration du mélange ;
- lavage au méthanol ;
- concentration sous vide.

Les composés *N*-1-adamantylbenzamide et *N*-2-adamantylbenzamide sont préparés à partir des amines correspondantes (la 1- ou 2-adamantylamine (1 équiv.)) *via* le traitement par une base comme NaH (1,1 équiv.) dans le DMF puis ajout du chlorure de benzoyle (1,2 équiv.) à 0°C. Le mélange est agité 24 heures à température ambiante, évaporé puis lavé avec du cyclohexane.

L'invention se rapporte enfin à un procédé de préparation des composés *N-*1-adamantylbenzamide et *N*-2-adamantylbenzamide comprenant les étapes suivantes :
- ajout à une suspension de NaH dans le DMF de la 1- ou 2-adamantylamine et du chlorure de benzoyle à 0°C ;
- agitation du mélange pendant 24 heures à température ambiante.

### Formation d'urées

L'invention se rapporte encore à un procédé de préparation d'un dérivé d'urée de formule générale (I), caractérisé en ce qu'il comprend les étapes suivantes :
- à une suspension de l'amine dérivée de 1-adamantyle (1 équiv.) dans le THF est ajouté à 0°C, l'isocyanate, l'amine et l'isocyanate correspondants audit dérivé d'urée souhaité (1,1 équiv.)
- agitation du mélange 24 heures à température ambiante,
- évaporation et purification sur une petite cartouche de silice.

### Alkylation d'alcools (ex adamantanemethanol)

Les alcools sont traités dans un premier temps par l'iode (2,5 équiv) et une base telle que le carbonate de potassium dans le tert-butanol et sont chauffés 24h à 70°C. Le nucléophile (amine, alcool) est ensuite ajouté (1,5 équiv). Après traitement classique et purification, les composés alkylés sont obtenus.

### Formation des dérivés triazoliques par « click chemistry »

L'invention se rapporte aussi à un procédé de préparation de dérivés triazolique de formule générale (I) caractérisé en ce qu'il comprend les étapes suivantes :
- réaction d'un azoture avec un acétylénique (1,1 équiv), ces deux réactifs étant choisis en fonction dudit composé triazolique de formule générale (I) à synthétiser, en présence de cuivre sur charbon (catalytique) et triéthylamine (1,0 équiv) dans le dioxane pendant 24h à température ambiante ;
- filtration du mélange sur célite et évaporation ;
- purification.

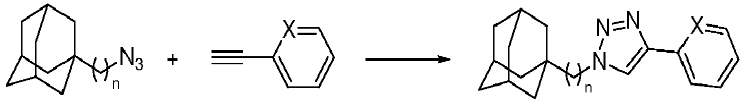

L'invention se rapporte également au 2-amino-N-phenylbenzamide comme intermédiaire de synthèse des amines de formule générale (I).

La présente invention se rapporte également à l'utilisation de composés dérivés de benzodiazépine de formule générale (II) : 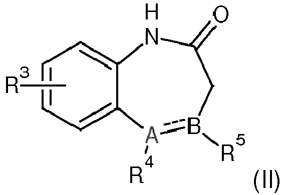 où
**A** et **B** représentent un atome de carbone ou un atome d'azote à la condition que si **A** = N alors **B** = C et si **A** = C alors **B** = N ;
**R³** est choisi parmi un atome d'hydrogène ou d'halogène ; un radical alkyl en C₁-C₆, un radical alcoxy en C₁-C₆ ou un radical acyloxy en C₁-C₆, ces radicaux étant éventuellement substitués par un radical alcoxy en C₁-C₆ ; un radical aryloxy ou un radical hétéroaryloxy ;
**R⁴** représente soit une liaison, soit est choisi parmi un atome d'hydrogène, un radical acyloxy en C₁-C₃, un radical alcoxy en C₁-C₃ ou un phényl ;
**R⁵** représente soit une liaison, soit est choisi parmi un atome d'hydrogène ; un radical alkyl en C₁-C₃ ; un radical alcoxy en C₁-C₃ ; un radical acyloxy en C₁-C₃ ou un radical phényle éventuellement substitué par une fonction -OH et/ou un atome d'halogène, un radical alkyl en C₁-C₃, un radical alcoxy en C₁-C₃, un radical acyloxy en C₁-C₃, une fonction -NO₂, -CF₃, ou un radical 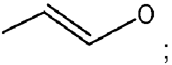 étant entendu que **R⁴** et **R⁵** ne peuvent simultanément représenter une liaison et que lorsque l'un des deux est une liaison alors **A** et **B** sont liés par une double liaison ; et
   que, lorsque **B** est un atome de carbone, **R⁵** peut également former avec l'atome d'hydrogène porté par le carbone adjacent à **B** un cycle de 5 ou 6 atomes éventuellement substitué par un radical phényl, éventuellement interrompu par un atome d'azote, de soufre ou d'oxygène ; de préférence, il s'agit d'un cycle à 5 atomes interrompu par un atome d'oxygène ;
   pour la préparation d'une composition pharmaceutique destinée à la prévention et/ou au traitement des intoxications à au moins une toxine à mode d'action intracellulaire ou à au moins un virus utilisant la voie d'internalisation pour infecter les cellules eucaryotes de mammifères.

L'invention se rapporte également aux sels pharmaceutiquement acceptables de ces composés

Le radical alkyle en C₁-C₆ désigne un radical hydrogénocarboné de 1 à 6 atomes de carbone, linéaire ou ramifié, on peut citer par exemple le méthyle, l'éthyle, le propyle ou l'isopropyle.

Par radical alcoxy en C₁-C₆, on entend un radical -OCₘH₂ₘ₊₁, m étant un nombre entier compris entre 1 et 6.

Par radical acyloxy en C₁-C₆, on entend un radical -O(CO)CₘH₂ₘ₊₁ ou - (CO)OCₘH₂ₘ₊₁, m étant un nombre entier compris entre 1 et 6.

Par radical aryloxy, on entend un groupement aryle relié par un atome d'oxygène au reste du composé.

Par radical hétéroaryloxy, on entend un groupement hétéroaryle relié par un atome d'oxygène au reste du composé.

De façon préférée, les composés de formule générale (II) sont tels que **R³** est une liaison et/ou **R⁴** représente un atome d'hydrogène et/ou **R⁵** représente un radical phényl et/ou quand **A** est un atome de carbone alors **R⁴** est un radical phényl et/ou quand **B** est un atome de carbone alors **R⁵** est un radical phényl.

Plus particulièrement, les composés de formule générale (II) sont choisis parmi :

| | | |
|---|---|---|
| 162 | 5-phenyl-2,3-dihydro-1*H*-1,4-benzodiazepin-2-one | 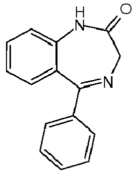 |
| 163 | 7-bromo-5-phenyl-2,3-dihydro-1*H-*1,4-benzodiazepin-2-one | 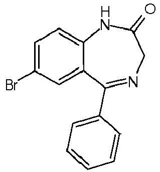 |
| 164 | 7-bromo-5-phenyl-2,3,4,5-tetrahydro-1*H*-1,4-benzodiazepin-2-one | 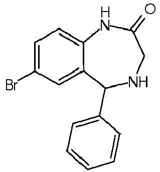 |
| 165 | 4-phenyl-2,3-dihydro-1*H*-1,5-benzodiazepin-2-one | 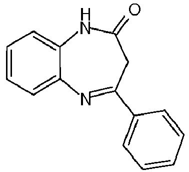 |
| 166 | 4-phenyl-2,3,4,5-tetrahydro-1*H*-1,5-benzodiazepin-2-one | 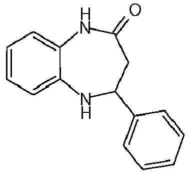 |
| 167 | 4,5-dihydro-7-methoxy-5-phenyl-1*H-*benzo[*e*][1,4]diazepin-2(3*H*)-one | 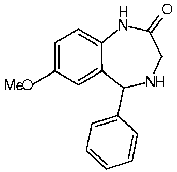 |
| 168 | Ethyl 4-oxo-2-phenyl-2,3,4,5-tetrahydro-1*H-*1,5-benzodiazepine-1-carboxylate | 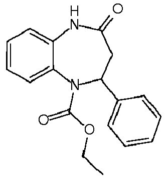 |
| 169 | 5-phenyl-2,3,4,5-tetrahydro-1*H*-1,4-benzodiazepin-2-one | 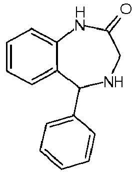 |
| 170 | 7-chloro-5-phenyl-2,3-dihydro-1*H*-1,4-benzodiazepin-2-one | 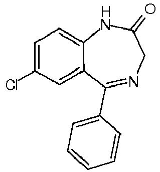 |
| 171 | 7-chloro-5-phenyl-2,3,4,5-tetrahydro-1*H*-1,4-benzodiazepin-2-one | 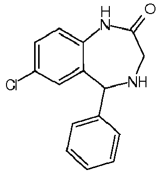 |
| 172 | 4-(2-hydroxyphenyl)-1*H-*benzo[*b*][1,4]diazepin-2(3*H*)-one | 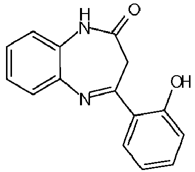 |
| 173 | 4-(5-bromo-2-hydroxyphenyl)-1*H-*benzo[b][1,4]diazepin-2(3*H*)-one | 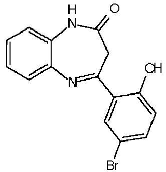 |
| 174 | 4-(5-fluoro-2-hydroxyphenyl)-1*H-*benzo[*b*][1,4]diazepin-2(3*H*)-one | 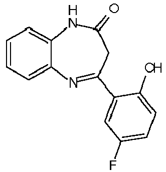 |
| 175 | 8-bromo-3-phenyl-3,3a,5,10-tetrahydrobenzo[*b*]pyrrolo[2,3-*e*][1,4]diazepin-4(2*H*)-one | 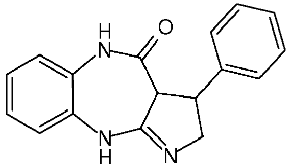 |
| 176 | 4-*m*-tolyl-1H-benzo[*b*][1,4]diazepin-2(3*H*)-one | 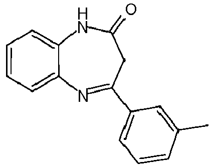 |
| 177 | 4-(3-methoxyphenyl)-1*H-*benzo[*b*][1,4]diazepin-2(3*H*)-one | 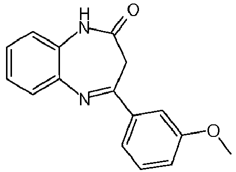 |
| 178 | 4-(3-nitrophenyl)-1*H*-benzo[*b*][1,4]diazepin-2(3*H*)-one | 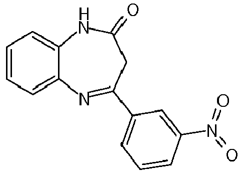 |
| 179 | 4-(3-chlorophenyl)-1*H*-benzo[*b*][1,4]diazepin-2(3*H*)-one | 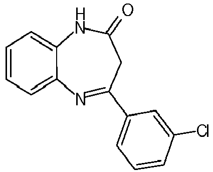 |
| 180 | 4-(3-bromophenyl)-1*H*-benzo[*b*][1,4]diazepin-2(3*H*)-one | 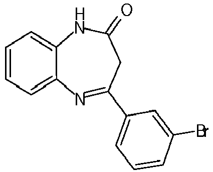 |
| 181 | 4-(3-(trifluoromethyl)phenyl)-1*H-*benzo[*b*][1,4]diazepin-2(3*H*)-one | 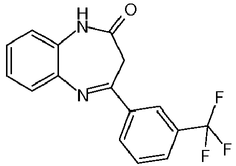 |
| 182 | 7-bromo-4-m-tolyl-1*H*-benzo[*b*][1,4]diazepin-2(3*H*)-one | 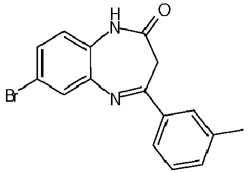 |
| 183 | 7-bromo-4-(3-methoxyphenyl)-1*H-*benzo[*b*][1,4]diazepin-2(3H)-one | 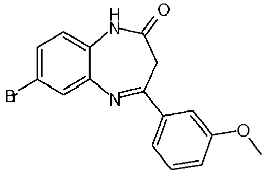 |
| 184 | 7-bromo-4-(3-nitrophenyl)-1*H-*benzo[*b*][1,4]diazepin-2(3*H*)-one | 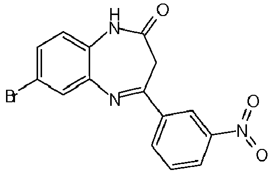 |
| 185 | 7-bromo-4-(3-chlorophenyl)-1*H-*benzo[*b*][1,4]diazepin-2(3*H*)-one | 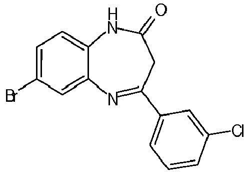 |
| 186 | 7-bromo-4-(3-bromophenyl)-1*H-*benzo[*b*][1,4]diazepin-2(3*H*)-one | 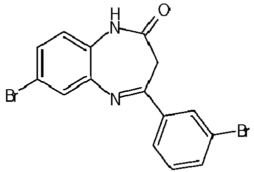 |
| 187 | 7-bromo-4-(3-(trifluoromethyl)phenyl)-1*H-*benzo[*b*][1,4]diazepin-2(3*H*)-one | 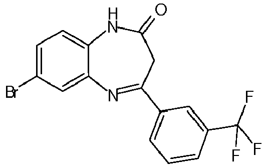 |
| 193 | 7-bromo-5-phenyl-4-propionyl-4,5-dihydro-1H-benzo[e][1,4]diazepin-2(3H)-one | 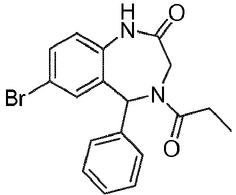 |

La présente invention se rapporte également aux composés de formule générale (II) et leurs sels pharmaceutiquement acceptables en tant que tels à l'exclusion des composés 162, 163, 164, 165, 166, 167, 169, 170, 171 et 193.

La synthèse des composés de formule générale (II) selon l'invention est décrite à l'exemple 1.

Plus particulièrement, les dérivés benzo[*e*][1,4]diazepine et les dérivés benzo[*b*][1,4]diazepine de formule générale (II) sont préparés comme suit :
- ajout à une suspension de diamine, choisie selon le composé à préparer, par exemple à partir de benzène-1,2-diamine, 4-bromobènzene-1,2-diamine (1 équiv.) dans le toluène (2 mL) avec un β-kétoester (1 équiv.) ;
- agitation du mélange à reflux (120°C) pendant 3 heures ;
- dilution du mélange dans l'EtOAc, acidification (pH 5) et extraction avec l'EtOAc ;
- filtration, évaporation et lavage avec Et₂O.

L'invention se rapporte également aux composés utiles comme intermédiaire de synthèse des composés de formule générale (II) choisis parmi : le *tert*-Butyl *N*-[(phenylcarbamoyl)methyl]carbamate, le *tert*-Butyl (4-methoxyphenylcarbamoyl)methylcarbamate, la 2-amino-*N*-phenylacetamide, la 2-amino-*N-*(4-methoxyphenyl)acetamide et la 2-benzoyl-4-bromoaniline.

Les composés selon l'invention sont des substances pharmacologiquement actives et trouvent leur intérêt grâce à leur effet inhibiteur des toxines à mode d'action intracellulaire, en particulier, de la ricine.

Selon un autre de ses objets, l'invention se rapporte aux composés de formule générale (I) et (II) et leurs sels pharmaceutiquement acceptables à l'exclusion des composés des composés 1, 6, 8, 11, 18, 29, 30, 34, 74, 78, 98, 100, 113, 114, 116, 121, 122, 124, 128, 135, 145, 161, 162, 163, 164, 165, 166, 167, 169, 170, 171 et 193, pour une utilisation comme médicament, en particulier comme principe actif.

L'invention se rapporte également à des compositions pharmaceutiques ou médicament comprenant un ou plusieurs composés de formule générale (I) ou (II) et leurs sels pharmaceutiquement acceptables à l'exclusion des composés des composés 1, 6, 8, 11, 18, 29, 30, 34, 74, 78, 98, 100, 113, 114, 116, 121, 122, 124, 128, 135, 145, 161, 162, 163, 164, 165, 166, 167, 169, 170, 171 et 193 dans un véhicule pharmaceutiquement acceptable.

Par pharmaceutiquement acceptable, on entend compatible avec une administration à un sujet, de préférence un mammifère, par toute voie d'administration.

L'homme du métier saura adapter la formulation des composés de formule générale (I) et (II) selon leurs propriétés physico-chimiques et leur voie d'administration.

Le médicament pourra être administré par voie orale, parentérale, pulmonaire, oculaire, nasale.... Les modes d'administration des composés (I) et (II) préférés sont ceux utilisant les voies aérienne (inhalation), orale (ingestion), parentérale ou locale (topique).

La quantité de composé de formule (I) ou (II) à administrer au mammifère dépend de l'activité propre de ce composé, activité qui peut être mesurée par des moyens qui sont exposés dans les exemples. Cette quantité dépend également de la gravité de la pathologie à traiter, notamment de la quantité de ricine absorbée et de la voie par laquelle elle l'a été, elle dépend enfin de l'âge et du poids de l'individu à traiter.

L'utilisation des composés de formule générale (I) ou (II) est particulièrement avantageuse pour prévenir et/ou traiter les désordres provoqués par les toxines AB à mode d'action intracellulaire et les virus utilisant la voie d'internalisation pour infecter les cellules.

Plus spécifiquement, les toxines AB ou toxines à mode d'action intracellulaire sont notamment : la ricine, les toxines botuliques, les toxines diphtériques, les toxines du charbon, la toxine cholérique, la toxine pertussique, la toxine de Shiga (vérotoxine-2), les toxines thermolabiles d'*Escherichia coli,* les grandes toxines clostridiales, les facteurs dermonecrotiques, des exemples de ces toxines sont listés dans le Tableau ci-dessous :

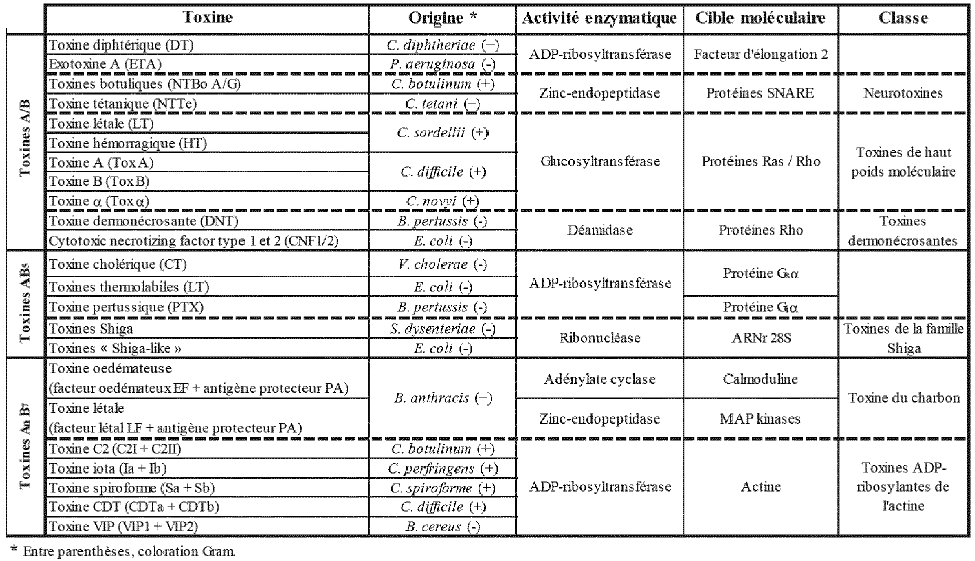

### Différentes activités, cibles moléculaires et structures des principales toxines bactériennes à action intracellulaire.

Les virus utilisant la voie d'internalisation pour infecter les cellules, ci-après également désignés virus, sont, par exemple, les virus à ARN, les *Flaviviridae* (comme la dengue, la fièvre jaune), les *Orthomyxoviridae* (comme la grippe) ou encore les *Rhabdoviridae* (comme la rage).

Cette utilisation s'avère efficace que le sujet touche, ingère ou inhale la toxine ou le virus, ou encore que la toxine ou le virus lui soit injectée.

Ainsi ces composés pourront être utilisés pour la préparation de composition pharmaceutique destinée aux traitements des effets des toxines AB ou toxines à mode d'action intracellulaire et des virus utilisant la voie d'internalisation pour infecter les cellules.

De façon concrète, une toxine comme la ricine, lorsqu'elle est inhalée, conduit à des signes d'irritation oculaire (sensation de brûlure, larmoiement, conjonctivite plus ou moins sévère) et pharyngée ainsi qu'une irritation respiratoire plus ou moins marquée : toux, dyspnée, oedème pulmonaire pouvant conduire à un syndrome de détresse respiratoire aigu (SDRA). Il est à noter qu'il existe un risque de réaction anaphylactique. La dose létale est de 1 mg/kg (Ministère de la santé, France).

Ainsi, l'invention se rapporte à l'utilisation d'un composé de formule générale (I) ou (II) pour la préparation d'une composition pharmaceutique destinée à la protection contre les effets de la ricine, d'autres toxines AB et de virus utilisant la voie d'internalisation pour infecter les cellules des cellules eucaryotes, notamment, épithéliales, oculaires, pharyngiennes, trachéales, bronchiques, cutanées, musculaires, en particulier, des cellules épithéliales pulmonaires et digestives, de préférence intestinales, de mammifères, de préférence de l'homme.

L'invention se rapporte plus spécifiquement à l'utilisation des composés de formule générale (I) et (II) et leurs sels pharmaceutiquement acceptables pour la préparation d'une composition pharmaceutique pour prévenir et/ou traiter une intoxication à la toxine de la ricine ; de préférence, les composés de formule générale (I) et (II) et leurs sels pharmaceutiquement acceptables sont choisis parmi les composés 1, 3, 5, 9, 15, 19, 24, 28, 34, 36, 39, 59, 65, 67, 68, 69, 75, 86, 89, 105, 106, 109, 110, 111, 112, 117, 118, 122, 128, 131, 138, 139, 140, 142, 143, 148, 154, 161 et 193.

Selon une variante préférée, l'invention se rapporte à l'utilisation de composés de formule générale (I) définis par la formule générale (I.1) 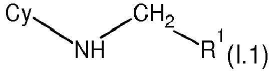 dans laquelle :

**Cy** représente un groupement choisi parmi : 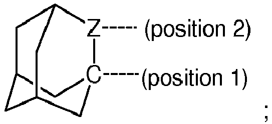 **Z** est un atome de carbone ou une liaison (Cy est alors un noyau noradamantyle) étant entendu que quand **Cy** est un noyau adamantyle, l'atome d'azote lui est attaché en position 1 ou 2,
**R¹** représente :
- un cycle phényl, éventuellement substitué par un radical -OCH₃ en para de l'atome de carbone lié à la chaine -CH₂-NH-Cy ; ledit cycle étant alternativement éventuellement substitué par un atome d'halogène en position méta de l'atome de carbone lié à la chaine - CH₂-NH-Cy et dans ce cas, ledit cycle porte éventuellement une seconde substitution en position para dudit atome d'halogène, ladite seconde substitution étant choisie parmi -NO₂ et -OCH₃ ;
- un cycle indole, imidazole ou furane substitué par un radical méthyl,
- un cycle benzo-(1,3)-dioxolo, un cycle naphthalènyle ou un cycle phénanthrényl ;
et leur sels pharmaceutiquement acceptables
pour la préparation d'une composition pharmaceutique pour prévenir et/ou traiter une intoxication à la toxine de la ricine.

De préférence, les composés de formule générale (I.1) sont choisis parmi les composés 1, 3, 5, 9, 15, 19, 24, 28, 34, 36, 39, 59, 65, 86, 109, 110, 111, 112, 138, 139, 140, 142, 143 et 148.

Selon une autre variante préférée, l'invention se rapporte à l'utilisation de composés de formule générale (I) définis par la formule générale (I.2) : 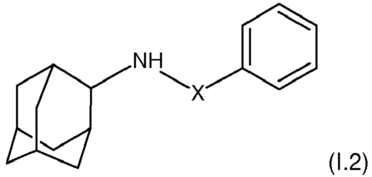 dans laquelle **X** représente -(CH₂)₂-O-CH₂-, -(CH₂)₃-, -CO-, -SO₂-
et leur sels pharmaceutiquement acceptables
pour la préparation d'une composition pharmaceutique pour prévenir et/ou traiter une intoxication à la toxine de la ricine.

De préférence, les composés de formule générale (I.2) sont choisis parmi les composés 68, 69, 122 et 128.

Selon encore une autre variante préférée, l'invention se rapporte à l'utilisation de composés de formule générale (I) définis par la formule générale (I.3) : 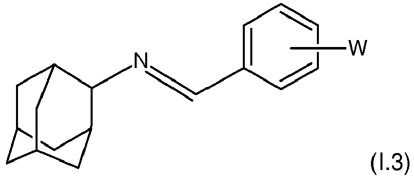 dans laquelle **W** représente un atome d'halogène
et leur sels pharmaceutiquement acceptables
pour la préparation d'une composition pharmaceutique pour prévenir et/ou traiter une intoxication à la toxine de la ricine.

De préférence, les composés de formule générale (I.3) sont choisis parmi les composés 117 et 118.

Une autre variante préférée de l'invention se rapporte à l'utilisation des composés de formule générale (I.4) : 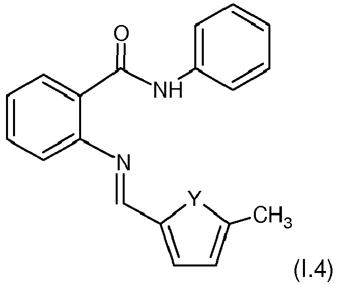 dans laquelle **Y** est O ou S
et leur sels pharmaceutiquement acceptables
pour la préparation d'une composition pharmaceutique pour prévenir et/ou traiter une intoxication à la toxine de la ricine.

De préférence, les composés de formule générale (I.4) sont choisis parmi les composés 154 et 161.

La présente invention se rapporte encore à l'utilisation des composés de formule générale (I) et (II) et leurs sels pharmaceutiquement acceptables pour la préparation d'une composition pharmaceutique pour prévenir et/ou traiter une intoxication à la toxine diphtérique.

Les composés apdatés à la prévention et/ou au traitement des intoxications à la toxine diphtérique sont notamment ceux de formule générale (I.5) 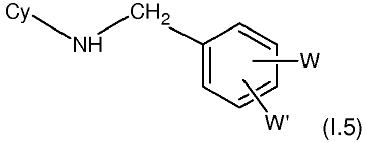 dans laquelle :

**Cy** représente un groupement: 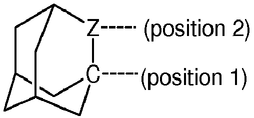 auquel l'atome d'azote est attaché en position 1 ou 2,

**W et W**' sont, indépendament l'un de l'autre, choisis parmi un atome d'hydrohène, un atome d'halogène et un radical alcoxy en C₁-C₃
et leur sels pharmaceutiquement acceptables.

De préférence, les composés de formule générale (I.5) sont choisis parmi les composés 5, 9, 39, 110, 111, 112, 139 et 140.

Outre les dispositions qui précèdent, l'invention comprend encore d'autres dispositions qui ressortiront de la description qui va suivre, qui se réfèrent à des exemples de mise en oeuvre de la présente invention, ainsi qu'aux figures annexées dans lesquelles :
La **Figure 1** est une représentation schématique dutest cellulaire à haut débit.
La **Figure 2** représente les résultats du criblage. Les points jaunes (nuage de points horizontal de la partie haute du graphe) représentent les contrôles positifs (cellules avec ricine + lactose), les points verts (nuage de points horizontal de la partie basse du graphe) représentent les contrôles négatifs (cellules traitées avec la ricine seule) et, en rouge, les composés selon l'invention testés en présence de ricine.

### Exemple 1 - Exemples de synthèse de composés selon l'invention

Les réactifs commerciaux ont été achetés auprès de Sigma-Aldrich et ont été utilisés sans purification préalable. Toutes les réactions ont été effectuées sous azote avec des solvants secs fraîchement distillés et de la verrerie séchée à l'étuve.

Les méthodes de purification utilisées pour la préparation des composés sont précisées dans la colonne « Méthode de purification » des tableaux et codées comme suit :
1 : Filtration
2 : Short pad (petite colonne avec silice déjà conditionnée)
3 : Colonne de chromatographie sur gel de silice
4 : HPLC
5 : Cristallisation
6 : Traitement aqueux puis séparation

La RMN ¹H a été réalisée avec un appareil Brucker Advance 400 MHz avec une sonde BBO. Les solvants sont précisés pour chaque expérience. Les déplacements chimiques sont donnés en parties par million (ppm), par rapport à la référence interne (TMS). Les données sont listées dans l'ordre suivant : δ, déplacement chimique ; multiplicité (avec s singulet, d doublet, t triplet, q quadruplet, m multiplet), intégration, constantes de couplages (*J* en Hertz, Hz).

Les analyses de LC/MS ont été réalisées par HPLC (High Pressure Liquid Chromatography) couplée avec un spectromètre de masse WATERS^{®} AUTOPURIF. L'ionisation est obtenue soit par collision électronique, soit par ionisation électrochimique. Les données sont obtenues sous forme m/z.
Colonne : Xbridge C18 3-5 µM, 4,6 mm*100 mm
Débit : 1.0 mL/min
Détecteurs :
- Photodiode array detector Waters 2996 : UV (200-400 nm),
- PL-ELS 1000,
- MS ZQ 2000.
Volume d'injection : 1 µL avec l'autosampler Waters 2767
Methode : 95 % solution A (99.99 % eau, 0.01 % acide formique), 5 %B (100 % acétonitrile) jusque 0 % A, 100 % B sur un gradient de 8 minutes puis palier de 5 minutes.

Les chromatographies sur colonne ont été effectuées avec un gel de silice Merck (taille des particules : 230-400 mesh). Toutes les réactions ont été suivies par chromatographie sur couche mince avec des plaques préenduites de gel de silice d'épaisseur 0.2 mm 60G-264 (Merck). La révélation a été effectuée avec une lampe à UV ou avec du diiode.

### Procédé A

Les dérivés de 1-aminoadamantane et de 2-aminoadamantane de formule générale (la) telle que décrite ci-dessus et figurant, respectivement, dans les Tableaux A1 et A2 sont préparés comme suit : une suspension dans le méthanol de 1-adamantylamine ou de 2-adamantylamine (0,5 mmol ; 75 mg ; 1 équiv.) est ajoutée sous agitation à un aldéhyde aromatique (1 équiv.) en fonction du composé à en présence de BH₃CN sur résine (0,75 mmol ; 1,5 équiv) et d'acide acétique (1,5 mmol ; 84 µL ; 3 équiv.). L'ensemble est agité 2 jours à température ambiante, filtré, lavé avec du méthanol, évaporé puis purifié.

Les Tableaux A1 et A2 présentent le numéro du composé, l'aldéhyde utilisé, le nom du composé obtenu, les caractéristiques du composé ainsi que le code de la méthode de purification utilisée, l'aspect du composé obtenu et son rendement.

**Tableau A1 : composés dérivés d'1-aminoadamantane préparés par le procédé A à partir du 1-adamantylamine**

| **Composé** | **aldéhyde** | **Nom du composé** | **Caractéristiques du composé** | **Méthode de purification / aspect / rendement** |
|---|---|---|---|---|
| 1 | Benzaldehyde | *N*-benzyladamantylamine | ¹H NMR (CDCl₃, 400MHz): δ 7.39 (m, 2H), 7.32 (m, 3H), 3.86 (s, 2H), 3.66 (bs, 1H), 2.12 (s, 3H), 1.84-1.63 (m, 12H). | 1 |
| | | | | solide blanc |
| | | | | 100% |
| | | | ESI+MS: calcd for C₁₇H₂₃N: 241.18; found: 242.2 (MH⁺) | |
| 2 | 2-bromobenzaldehyde | *N*-(2-bromobenzyl)adamantyla mine | | 1 |
| | | | | solide jaune |
| | | | | 24% |
| 3 | 3-bromobenzaldehyde | *N*-(3-bromobenzyl)adamantyla mine | ¹H NMR (CDCl₃, 400MHz): δ 7.56 (s, 1H), 7.39 (d, 1H, *J* = 6.7 Hz), 7.33 (d, 1H, *J* = 7.6 Hz), 7.18 (t, 1H, *J* = 8 Hz), 6.09 (bs, 1H), 3.81 (s, 2H), 2.12 (s, 3H), 1.79-1.63 (m, 12H). | 1 |
| | | | | solide blanc |
| | | | | 35% |
| | | | ¹³C NMR (CDCl₃, 100MHz): δ 139.7, 132.2, 130.7, 130.0, 127.8, 122.4, 53.7, 43.7, 40.9, 36.2, 29.3. | |
| 4 | 4-bromobenzaldehyde | *N-*(4-bromobenzyl)adamantyla mine | ESI+MS: calcd for C₁₇H₂₂BrN: 319.09; found: 320.1 (MH⁺) | 1 |
| | | | | solide vert |
| | | | | 70% |
| 5 | 3-fluorobenzaldehyde | *N-*(3-fluorobenzyl)adamantylam ine | ¹H NMR (CDCl₃, 400MHz): δ 7.26 (m, 1H), 7.15 (m, 2H), 6.95 (m, 1H), 4.10 (bs, 1H), 3.85 (s, 2H), 2.12 (s, 3H), 1.79-1.62 (m, 12H). | 1 |
| | | | | solide blanc |
| | | | | 100% |
| | | | ESI+MS: calcd for C₁₇H₂₂FN: 259.17; found: 260.2 (MH⁺) | |
| 6 | 3-hydroxybenzaldehyde | *N*-(3-hydroxybenzyl)adamantyl amine | ¹H NMR (CDCl₃, 400MHz): δ 7.06 (t, 1H, *J* = 7.6 Hz), 6.83 (s, 1H), 6.75 (d, 1H, *J* = 7.6 Hz), 6.63 (d, 1H, *J* = 8 Hz), 3.83 (s, 2H), 3.49 (bs, 1H), 2.18 (s, 3H), 1.93-1.67 (m, 12H). | 1 |
| | | | | solide jaune pale |
| | | | | 98% |
| | | | ¹³C NMR (DMSO-d6, 100MHz): δ 156.3, 137.2, 127.7, 117.9, 114.6, 113.2, 52.0, 42.3, 38.8, 34.6,27.6. | |
| | | | ESI+MS: calcd for C₁₇H₂₃NO: 257.18; found: 258.1 (MH⁺) | |
| 7 | 2-methoxybenzaldehyde | *N-*(2-methoxybenzyl)adamantyl amine | ESI+MS: calcd for C₁₈H₂₅NO: 271.19; found: 272.2 (MH⁺) | 1 |
| | | | | solide jaune pale |
| | | | | 32% |
| 8 | 3-methoxybenzaldehyde | *N*-(3-methoxybenzyl)adamantyl amine | ESI+MS: calcd for C₁₈H₂₅NO: 271.19; found: 272.2 (MH⁺) | 1 |
| | | | | huile verte |
| | | | | 40% |
| 9 | 4-methoxybenzaldehyde | *N-*(4-methoxybenzyl)adamantyl amine | ¹H NMR (CDCl₃, 400MHz): δ 7.19 (d, 2H, *J* = 8.4 Hz), 6.77 (d, 2H, *J* = 8.4 Hz), 3.70 (s, 3H), 3.64 (s, 2H), 2.01 (s, 3H), 1.65-1.54 (m, 12H). | 1 |
| | | | | solide vert |
| | | | | 42% |
| | | | ¹³C NMR (CDCl₃, 100MHz): δ 158.5, 129.6, 113.8, 55.2, 51.4, 44.3, 42.4, 36.6, 29.6. ESI+MS: calcd for C₁₈H₂₅NO: 271.19; found: 272.2 (MH⁺) | |
| 10 | 2-nitrobenzaldehyde | *N*-(2-nitrobenzyl)adamantylami ne | ¹H NMR (CDCl₃, 400MHz): δ 7.85 (d, 1H, *J* = 8.0 Hz), 6.68 (d, 1H, *J* = 7.6 Hz), 7.52 (t, 1H, *J* = 7.2 Hz), 7.34 (t, 1H, *J* = 7.6 Hz), 3.96 (s, 2H), 2.04 (s, 3H), 1.69-1.56 (m, 12H). | 1 |
| | | | | solide orange |
| | | | | 44% |
| | | | ¹³C NMR (CDCl₃, 100MHz): δ 149.3, 133.1, 131.8, 127.7, 124.5, 51.2, 42.7, 42.2, 36.7, 29.6. ESI+MS: calcd for C₁₇H₂₂N₂O₂: 286.17; found: 287.2 (MH⁺) | |
| 11 | 4-nitrobenzaldehyde | *N*-(4-nitrobenzyl)adamantylami ne | ESI+MS: calcd for C₁₇H₂₂N₂O₂: 286.17; found: 287.2 (MH⁺) | 1 |
| | | | | solde orange |
| | | | | 58% |
| 12 | 4-methyl 4-formylbenzoate | *N*-(4-carbethoxybenzyl)adaman tylamine | ¹H NMR (CDCl₃, 400MHz): δ 7.99 (d, 2H, *J* = 8.4 Hz), 7.46 (d, 2H, *J* = 8.4 Hz), 6.37 (bd, 1H), 3.90 (s, 5H), 2.12 (s, 3H), 1.81-1.62 (m, 12H). | 1 |
| | | | | solide blanc |
| | | | | 100% |
| | | | ¹³C NMR (CDCl₃, 100MHz): δ 176.1, 166.4, 139.4, 129.7, 129.3, 55.5, 51.9, 43.1, 39.2, 35.6, 28.9. | |
| | | | ESI+MS: calcd for C₁₉H₂₅NO₂: 299.19; found: 300.1 (MH⁺) | |
| 13 | 5-bromo-2-hydroxybenzaldehyde | 4-bromo-2-((1 - adamantylino)methyl)phen ol | ¹H NMR (CDCl₃, 400MHz): δ 7.28 (dd, 1H, *J* = 2.4 and 8.8 Hz), 7.14 (dd, 1H, *J* = 2.4 and 25.6 Hz), 6.73 (dd, 1H, *J* = 8.4 and 34 Hz), 5.17 (bs, 2H), 3.95 (s, 2H), 2.12 (s, 3H), 1.84-1.61 (m, 12H). | 2 |
| | | | | solide blanc |
| | | | | 15% |
| | | | ESI+MS: calcd for C₁₇H₂₂BrNO: 335.09; found: 336.2 (MH⁺) | |
| 14 | 2-bromo-5-methoxybenzaldehyde | *N*-(2-bromo-5-methoxybenzyl)adamantyl amine | ¹H NMR (CDCl₃, 400MHz): δ 7.46 (d, 1H, *J* = 2.4 Hz), 7.31 (dd, 1H, *J* = 2.4 and 8.8 Hz), 6.71 (d, 1H, *J* = 8.8 Hz), 3.82 (s, 3H), 3.76 (s, 2H), 3.19 (bs, 1H), 2.10 (s, 3H), 1.74-1.62 (m, 12H). | 1 |
| | | | | solide blanc |
| | | | | 55% |
| | | | ¹³C NMR (CDCl₃, 100MHz): δ 156.5, 132.6, 130.7, 111.8, 55.5, 51.8, 42.1, 39.6, 36.6, 29.5. | |
| | | | ESI+MS: calcd for C₁₈H₂₄BrNO: 349.10; found: 350.1 (MH⁺) | |
| 15 | 2-methoxy-5-bromobenzaldehyde | *N*-[(2-methoxy-5-bromo)benzyl]adamantyla mine | | 1 Solide blanc 100% |
| 16 | picolinaldehyde | *N*-((pyridin-2-yl)methyl)adamantylamine | ¹H NMR (CDCl₃, 400MHz): δ 8.52 (d, 1H, *J* = 4.8 Hz), 7.67 (dt, 1H, *J* = 1.6 and 7.6 Hz), 7.38 (d, 1H, *J* = 7.6 Hz), 7.21 (dd, 1H, *J* = 4.8 and 6.8 Hz), 4.17 (s, 3H), 3.76 (s, 2H), 2.14 (s, 3H), 1.92-1.64 (m, 12H). | 1 |
| | | | | solide brun |
| | | | | 55% |
| | | | ¹³C NMR (CDCl₃, 100MHz): δ 148.7, 137.1, 122.8, 114.0, 54.5, 43.9, 40.4, 36.0, 29.2. | |
| | | | ESI+MS: calcd for C₁₆H₂₂N₂: 242.18; found: 243.2 (MH⁺) | |
| 17 | nicotinaldehyde | *N*-((pyridin-3-yl)methyl)adamantylamine | ¹H NMR (CDCl₃, 400MHz): δ 8.59 (s, 1H), 8.52 (d, 1H, *J* = 6.6 Hz), 7.92 (d, 1H, *J* = 8.0 Hz), 7.31 (m, 1H), 4.00 (s, 2H), 2.16 (s, 3H), 1.90-1.63 (m, 12H). | 1 |
| | | | | solide blanc |
| | | | | 93% |
| | | | ¹³C NMR (CDCl₃, 100MHz): δ 150.1, 149.1, 138.3, 129.6, 123.7, 56.4, 40.9, 39.2, 35.7, 29.1. | |
| | | | ESI+MS: calcd for C₁₆H₂₂N₂: 242.18; found: 243.2 (MH⁺) | |
| 18 | isonicotinaldehyde | *N*-((pyridin-4-yl)methyl)adamantylamine | ¹H NMR (CDCl₃, 400MHz): δ 8.53 (d, 1H, *J* = 4.4 Hz), 7.38 (d, 1H, *J* = 5.2 Hz), 3.91 (s, 2H), 2.13 (s, 3H), 1.80-1.61 (m, 12H). | 1 |
| | | | | solide jaune pale |
| | | | | 61% |
| | | | ESI+MS: calcd for C₁₆H₂₂N₂: 242.18; found: 243.2 (MH⁺) | |
| 19 | 5-methylfuran-2-carbaldehyde | *N*-((5-methylfuran-2-yl)methyl)adamantylamine | ¹H NMR (CDCl₃, 400MHz): δ 6.22 (d, 1H, *J* = 2.8 Hz), 5.83 (d, 1H, *J* = 2.0 Hz), 5.80 (s, 1H), 3.89 (s, 2H), 2.19 (s, 3H), 2.07 (s, 3H), 1.79 (m, 6H), 1.60 (m, 6H). | 1 |
| | | | | solide jaune |
| | | | | 84% |
| | | | ¹³C NMR (CDCl₃, 100MHz): δ 152.6, 145.4, 111.3, 106.6, 55.5, 39.0, 36.1, 35.6, 28.9, 13.2. | |
| | | | ESI+MS: calcd for C₁₆H₂₃NO: 245.18; found: 246.2 (MH⁺) | |
| 20 | 5-methylthiophene-2-carbaldehyde | *N*-((5-methylthiophen-2-yl)methyl)cyclohexanamin e | ¹H NMR (CDCl₃, 400MHz): δ 6.80 (d, 1H, *J* = 3.2 Hz), 6.57 (d, 1H, *J* = 3.2 Hz), 5.33 (bs, 1H), 3.98 (s, 2H), 2.43 (s, 3H), 2.11 (s, 3H), 1.78 (m, 6H), 1.66 (m, 6H). | 1 |
| | | | | solide jaune |
| | | | | 89% |
| | | | ¹³C NMR (CDCl₃, 100MHz): δ 139.7, 138.3, 126.5, 124.9, 53.3, 41.1, 39.0, 36.3, 29.4, 15.3. ESI+MS: calcd for C₁₆H₂₃NS: 261.16; found: 262.2 (MH⁺) | |
| 21 | Furan-3-carbaldehyde | *N*-[(3-furyl)methyl]adamantylami ne | ¹H NMR (CDCl₃, 400MHz): δ 7.51 (s, 1H), 7.36 (s, 1H), 6.52 (s, 1H), 3.84 (s, 2H), 2.15 (s, 3H), 1.89-1.64 (m, 12H). | 1 |
| | | | | solide jaune |
| | | | | 70% |
| | | | ESI+MS: calcd for C₁₅H₂₁NO: 231.16; found: 232.2 (MH⁺) | |
| 22 | 1-methyl-1*H*-imidazole-5-carbaldehyde | *N*-((1-methyl-1H-imidazol-5-yl)methyl)adamantylamine | ¹H NMR (CDCl₃, 400MHz): δ 7.53 (s, 1H), 6.97 (s, 1H), 6.71 (bs, 1H), 3.81 (s, 2H), 3.70 (s, 3H), 2.12 (s, 3H), 1.76-1.62 (m, 12H). | 1 |
| | | | | solide blanc |
| | | | | 100% |
| 23 | 1-methyl-1*H*-imidazole-2-carbaldehyde | *N-[(5-N-*methylimidazolyl)methyl]a damantylamine | ¹H NMR (CDCl₃, 400MHz): δ 7.75 (bs, 1H), 6.96 (s, 1H), 6.837 (s, 1H), 4.15 (s, 2H), 3.72 (s, 3H), 2.14 (s, 3H), 1.83-1.64 (m, 12H). | 1 |
| | | | | solide blanc |
| | | | | 100% |
| | | | ¹³C NMR (CDCl₃, 100MHz): δ 131.8, 126.2, 121.8, 54.3, 40.1, 36.1, 29.3. | |
| | | | ESI+MS: calcd for C₁₅H₂₃N₃: 245.18; found: 246.2 (MH⁺) | |
| 24 | Benzo[*d*][1,3]dioxole-4-carbaldehyde | Benzo[*d*][1,3]dioxol-4-yl)methyl)adamantylamine | ¹H NMR (CDCl₃, 400MHz): δ 6.87 (d, 1H, *J* = 8.4 Hz), 6.79 (t, 1H, *J* = 7.6 Hz), 6.73 (dd, 1H, *J* = 1.2 and 7.6 Hz), 5.97 (s, 2H), 3.82 (s, 2H), 3.22 (bs, 1H), 2.11 (s, 3H), 1.78-1.62 (m, 12H). | 1 |
| | | | | solide blanc |
| | | | | 100% |
| | | | ESI+MS: calcd for C₁₈H₂₃NO₂: 285.17; found: 286.2 (MH⁺) | |
| 25 | 6-nitro[d][1,3]dioxole-5-carbaldehyde | *N*-((5-nitrobenzo[*d*][1,3]dioxol-6-yl)methyl)adamantylamine | ¹H NMR (CDCl₃, 400MHz): δ 7.56 (s, 1H), 7.25 (s, 1H), 6.36 (bs, 1H), 6.13 (s, 2H), 4.18 (s, 2H), 2.19 (s, 3H), 1.99-1.67 (m, 12H). | 1 |
| | | | | solide brun |
| | | | | 54% |
| | | | ESI+MS: calcd for C₁₈H₂₂N₂O₄: 330.16; found: 331.2 (MH⁺) | |
| 26 | Quinoline-3-carbaldehyde | *N*-((quinolin-3-yl)methyl)adamantylamine | ¹H NMR (CDCl₃, 400MHz): δ 8.91 (s, 1H), 8.22 (s, 1H), 8.08 (d, 1H, *J* = 8.4 Hz), 7.81 (d, 1H, *J* = 8.4 Hz), 7.68 (t, 1H, *J* = 7.2 Hz), 7.53 (t, 1H, *J* = 7.6 Hz), 4.02 (s, 2H), 3.31 (bs, 1H), 2.11 (s, 3H), 1.88-1.62 (m, 12H). | 1 |
| | | | | solide jaune |
| | | | | 100% |
| | | | ESI+MS: calcd for C₂₀H₂₄N₂: 292.19; found: 293.3 (MH⁺) | |
| 27 | Quinoline-4-carbaldehyde | *N*-((quinolin-4-yl)methyl)adamantylamine | ESI+MS: calcd for C₂₀H₂₄N₂: 292.19; found: 293.2 (MH⁺) | 1 |
| | | | | solide orange 100% |
| 28 | 1-methyl-1*H*-indole-2-carbaldehyde | *N*-((1-methyl-1*H*-indol-2-yl)methyl)adamantylamine | ¹H NMR (CDCl₃, 400MHz): δ 7.54 (d, 1H, *J* = 7.6 Hz), 7.28 (d, 1H, *J* = 8.0 Hz), 7.17 (t, 1H, *J* = 7.2 Hz), 7.06 (t, 1H, *J* = 7.6 Hz), 6.38 (s, 1H), 3.92 (s, 2H), 3.79 (s, 3H), 2.11 (s, 3H), 1.75-1.63 (m, 12H). | 1 |
| | | | | solide jaune |
| | | | | 100% |
| | | | ESI+MS: calcd for C₂₀H₂₆N₂: 294.21; found: 295.2 (MH⁺) | |
| 29 | 2-phenylacetaldehyde | *N-*phenethyladamantylamine | ESI+MS: calcd for C₁₈H₂₅N₂: 255.20; found: 256.3 (MH⁺) | 2 |
| | | | | huile incolore |
| | | | | 5% |
| 30 | 3-phenylpropanal | *N*-(3-phenylpropyl)adamantyla mine | ¹H NMR (CDCl₃, 400MHz): δ 8.02 (bs, 1H), 7.26 (m, 2H), 7.16 (m, 3H), 2.78 (t, 2H, *J* = 7.6 Hz), 2.62 (t, 2H, *J* = 8.4 Hz), 2.06 (s, 2H), 1.85 (s, 6H), 1.62 (m, 6H). | 1 |
| | | | | solide blanc |
| | | | | 100% |
| | | | ¹³C NMR (CDCl₃, 100MHz): δ 128.5, 128.4, 128.3, 126.0, 55.2, 38.9, 38.8, 35.8, 33.2, 28.9, 28.3. | |
| | | | ESI+MS: calcd for C₁₉H₂₇N: 269.22; found: 270.3 (MH⁺) | |
| 31 | 2-(benzyloxy)acetaldehyd e | *N*-(2-(benzyloxy)ethyl)adamant ylamine | ¹H NMR (CDCl₃, 400MHz): δ 7.33 (m, 5H), 4.53 (s, 2H), 3.69 (t, 2H, *J* = 5.2 Hz), 2.96 (t, 2H, *J* = 5.2 Hz), 2.12 (s, 3H), 1.78 (m, 5H), 1.66 (m, 7H). | 1 |
| | | | | solide orange |
| | | | | 100% |
| | | | ESI+MS: calcd for C₁₉H₂₇NO: 285.21; found: 286.3 (MH⁺) | |
| 32 | cinnamaldehyde | *N-*cinnamyladamantylamine | ¹H NMR (CDCl₃, 400MHz): δ 8.81 (bs, 1H), 7.36-7.11 (m, 5H), 6.59 (d, 1H, *J* = 16 Hz), 6.35 (dt, 1H, *J* = 6.8 and 15.6 Hz), 3.54 (d, 2H, *J* = 6.4 Hz), 2.00 (s, 3H), 1.84-1.55 (m, 12H). | 1 |
| | | | | solide jaune |
| | | | | 100% |
| | | | ESI+MS: calcd for C₁₉H₂₅N: 267.20; found: 268.3 (MH⁺) | |
| 33 | paraformaldehyde | *N*-methyl(3-bromobenzyl)adamantyla mine | ¹H NMR (CDCl₃, 400MHz): δ 7.51 (s, 1H), 7.34 (d, 1H, *J* = 7.6 Hz), 7.25 (d, 1H, *J* = 6.8 Hz), 7.17 (t, 1H, *J* = 7.6 Hz), 3.53 (s, 2H), 2.13 (s, 6H), 1.77 (s, 6H), 1.66 (m, 6H). | 2 |
| | | | | huile incolore |
| | | | | 23% |
| | | | ESI+MS: calcd for C₁₈H₂₄BrN: 333.11; found: 334.0 (MH⁺) | |

**Tableau A2 : composes dérivés de 2-aminoadamantane préparés par le procédé A à partir du 2-adamantylamine**

| **Composé** | **aldéhyde** | **Nom du composé** | **caractéristiques du composé** | **Méthode de purification / aspect/ rendement** |
|---|---|---|---|---|
| *34* | Benzaldehyde | *N*-benzyl-2-adamantylamine | ¹H NMR (CDCl₃, 400MHz): δ 7.55 (m, 2H), 7.37 (m, 3H), 6.33 (bs, 1H), 3.05 (s, 1H), 2.28-1.59 (m, 14H). | 1 |
| | | | | huile blanche |
| | | | | 100% |
| | | | ¹³C NMR (CDCl₃, 100MHz): δ 131.5, 130. 1, 128.9, 60.2, 48.4, 37.1, 36.8, 30.4, 29.2, 26.9, 26.6, 22.8. | |
| | | | ESI+MS: calcd for C₁₇H₂₃N: 241.18; found: 242.2 (MH⁺) | |
| *35* | 2-bromobenzaldehyde | *N*-(2-bromobenzyl)-2-adamantylamine | LC-MS (ES⁺) *m*/*z* 320.1 (M+H)⁺ | 1 |
| | | | ¹H NMR (CDCl₃, 400MHz): δ 7.70 (d, 1H, *J* = 7.6 Hz), 7.56 (d, 1H, *J* = 7.6 Hz), 7.33 (t, 1H, *J* = 7.2 Hz), 7.18 (m, 1H), 5.42 (bs, 1H), 4.11 (s, 2H), 3.02 (s, 1H), 2.20-1.59 (m, 14H). | huile incolore |
| | | | | 62% |
| | | | ESI+MS: calcd for C₁₇H₂₂BrN: 319.09; found: 320.1 (MH⁺) | |
| *36* | 3-bromobenzaldehyde | *N*-(3-bromobenzyl)-2-adamantylamine | ¹H NMR (CDCl₃, 400MHz): δ 7.66 (s, 1H), 7.53 (d, 1H, *J* = 6.8 Hz), 7.46 (d, 1H, *J* = 8.4 Hz), 7.26 (m, 1H), 4.02 (s, 2H), 2.98 (s, 1H), 2.22-1.61 (m, 14H). | 1 |
| | | | | solide blanc |
| | | | | 60% |
| | | | ¹³C NMR (CDCl₃, 100MHz): δ 132.7, 131.8, 128.5, 122.8, 60.8, 48.3, 37.3, 36.9, 30.7, 29.8, 27.1, 26.9. | |
| | | | ESI+MS: calcd for C₁₇H₂₂BrN: 319.09; found: 320.1 (MH⁺) | |
| *37* | 4-bromobenzaldehyde | *N*-(4-bromobenzyl)-2-adamantylamine | ¹H NMR (CDCl₃, 400MHz): δ 7.50 (d, 1H, *J* = 8.4 Hz), 7.43 (d, 1H, *J* = 8.0 Hz), 5.65 (bs, 1H), 4.00 (s, 2H), 2.97 (s, 1H), 2.22-1.60 (m, 14H). | 1 |
| | | | | solide blanc |
| | | | | 27% |
| | | | ESI+MS: calcd for C₁₇H₂₂BrN: 319.09; found: 320.1 (MH⁺) | |
| *38* | 2-fluorobenzaldehyde | *N*-(2-fluorobenzyl)-2-adamantylamine | ¹H NMR (CDCl₃, 400MHz): δ 7.42 (td, 1H, *J* = 1.2 and 7.2 Hz), 7.22 (m, 1H), 7.33 (td, 1H, *J* = 0.8 and 7.2 Hz), 7.03 (t, 1H, *J* = 9.6 Hz), 3.87 (s, 2H), 2.81 (s, 1H), 2.33 (bs, 1H), 2.04 (d, 2H, *J* = 12.4 Hz), 1.87 (s, 2H), 1.84 (m, 4H), 1.70 (m, 4H), 1.52 (d, 2H, *J* = 12 Hz). ESI+MS: calcd for C₁₇H₂₂FN: 259.17; found: 260.2 (MH⁺) | 2 |
| | | | | solide jaune |
| | | | | 9,6% |
| *39* | 3-fluorobenzaldehyde | *N*-(3-fluorobenzyl)-2-adamantylamine | ¹H NMR (CDCl₃, 400MHz): δ 7.34 (m, 3H), 7.03 (m, 1H), 4.08 (s, 2H), 3.01 (s, 1H), 2.22-1.61 (m, 14H). | 1 |
| | | | | solide blanc |
| | | | | 100% |
| | | | ESI+MS: calcd for C₁₇H₂₂FN: 259.17; found: 260.2 (MH⁺) | |
| *40* | 4-fluorobenzaldehyde | *N*-(4-fluorobenzyl)-2-adamantylamine | ¹H NMR (CDCl₃, 400MHz): δ 7.33 (dd, 2H, *J* = 5.6 and 8.4 Hz), 7.00 (t, 2H, *J* = 8.8 Hz), 3.77 (s, 2H), 2.79 (s, 1H), 2.04 (d, 2H, *J* = 12.4 Hz), 1.85 (m, 6H), 1.70 (m, 4H), 1.52 (d, 2H, *J* = 12.4 Hz). | Short pad |
| | | | | solide jaune |
| | | | | 5,2% |
| | | | ESI+MS: calcd for C₁₇H₂₂FN: 259.17; found: 260.2 (MH⁺) | |
| *41* | 3-hydroxybenzaldehyde | *N*-(3-hydroxybenzyl)-2-adamantylamine | ¹H NMR (CDCl₃, 400MHz): δ 7.25 (s, 1H), 7.18 (t, 1H, J = 7.6 Hz), 6.87 (dd, 1H, *J* = 2.0 and 8.4 Hz), 6.80 (d, 1H, *J* = 7.2 Hz), 4.04 (s, 2H), 3.12 (s, 2H), 2.24-1.62 (m, 14H). | 1 |
| | | | | mousse blanche |
| | | | | 88% |
| | | | ¹³C NMR (CDCl₃, 100MHz): δ 157.7, 131.7, 129.9, 121.1, 116.9, 116.9, 116.9, 60.6, 48.4, 36.9, 36.7, 30.3, 29.1, 26.7, 26.5. | |
| | | | ESI+MS: calcd for C₁₇H₂₃NO: 257.18; found: 258.1 (MH⁺) | |
| *42* | 2-methoxybenzaldehyde | *N*-(2-methoxybenzyl)-2-adamantylamine | ¹H NMR (CDCl₃, 400MHz): δ 7.42 (dd, 1H, *J* = 1.2 and 7.6 Hz), 7.33 (m, 1H), 6.96 (t, 1H, *J* = 8 Hz), 5.66 (bs, 1H), 4.09 (s, 2H), 3.88 (s, 3H), 3.10 (s, 1H), 2.19-1.63 (m, 14H). | 1 |
| | | | | solide blanc |
| | | | | 54% |
| | | | ESI+MS: calcd for C₁₈H₂₅NO: 271.19; found: 272.2 (MH⁺) | |
| *43* | 3-methoxybenzaldehyde | *N*-(3-methoxybenzyl)-2-adamantylamine | ¹H NMR (CDCl₃, 400MHz): δ 7.32-6.83 (m, 4H), 3.97 (s, 2H), 3.83 (s, 3H), 2.94 (s, 1H), 2.18-1.58 (m, 14H). | 1 |
| | | | | Solide blanc |
| | | | | 48% |
| | | | ESI+MS: calcd for C₁₈H₂₅NO: 271.19; found: 272.2 (MH⁺) | |
| *44* | 4-methoxybenzaldehyde | N-(4-methoxybenzyl)-2-adamantylamine | ¹H NMR (CDCl₃, 400MHz): δ 7.39 (d, 2H, *J* = 8.8 Hz), 6.89 (d, 2H, *J* = 8.4 Hz), 5.62 (bs, 1H), 3.96 (s, 2H), 3.79 (s, 3H), 2.97 (s, 1H), 2.15-1.59 (m, 14H). ESI+MS: calcd for C₁₈H₂₅NO: 271.19; found: 272.2 (MH⁺) | 1 |
| | | | | Solide blanc |
| | | | | 45% |
| *45* | 2-nitrobenzaldehyde | *N*-(2-nitrobenzyl)-2-adamantylamine | ESI+MS: calcd for C₁₇H₂₂N₂O₂: 286.17; found: 287.2 (MH⁺) | 1 |
| | | | | Huile jaune |
| | | | | 59% |
| *46* | 4-nitrobenzaldehyde | *N*-(4-nitrobenzyl)-2-adamantylamine | ¹H NMR (CDCl₃, 400MHz): δ 8.20 (d, 2H, *J* = 8.4 Hz), 7.63 (d, 2H, *J =* 8.4 Hz), 3.99 (s, 2H), 2.82 (s, 1H), 2.20-1.54 (m, 14H). | 1 |
| | | | | Huile jaune |
| | | | | 56% |
| | | | ESI+MS: calcd for C₁₇H₂₂N₂O₂: 286.17; found: 287.2 (MH⁺) | |
| *47* | 4-methyl-4-formylbenzoate | *N*-(4-carbethoxybenzyl)-2-adamantylamine | ¹H NMR (CDCl₃, 400MHz): δ 8.86 (bs, 1H), 7.97 (d, 2H, *J* = 8.4 Hz), 7.57 (d, 2H, *J* = 8.4 Hz), 4.11 (s, 2H), 3.85 (s, 3H), 3.02 (s, 1H), 2.17-1.54 (m, 14H). | 1 |
| | | | | Solide blanc |
| | | | | 100% |
| | | | ¹³C NMR (CDCl₃, 100MHz): δ 166.3, 136.6, 130.5, 130.0, 129.9, 60.8, 52.1, 48.1, 37.0, 36.7, 30.3, 29.2, 26.7, 26.5. | |
| | | | ESI+MS: calcd for C₁₉H₂₅NO₂: 299.19; found: 300.1 (MH⁺) | |
| *48* | 5-bromo-2-hydroxybenzaldehyde | 4-bromo-2-((2-adamantylamino)methyl)p henol | ESI+MS: calcd for C₁₇H₂₂BrNO: 335.09; found: 336.1 (MH⁺) | 2 |
| | | | | Solide jaune pâle |
| | | | | 18% |
| 50 | 5-bromo-2-methoxybenzaldehyde | *N*-(5-bromo-2-methoxybenzyl)-2-adamantylamine | ESI+MS: calcd for C₁₈H₂₄BrNO₂: 349.10; found: 350.1 (MH⁺) | 1 |
| | | | | solide blanc |
| | | | | 56% |
| 51 | 5-fluoro-2-nitrobenzaldehyde | *N*-(5-fluoro-2-nitrobenzyl)-2-adamantylamine | ESI+MS: calcd for C₁₇H₂₁FN₂O₂: 304.16; found: 305.2 (MH⁺) | 4 |
| | | | | huile incolore |
| | | | | 2,3% |
| 52 | 2,5-difluorobenzaldehyde | *N*-(2,5-difluorobenzaldehyde)-2-adamantylamine | ¹H NMR (CDCl₃, 400MHz): δ 7.67-7.62 (m, 1H), 7.19-7.15 (m, 1H), 7.11-6.93 (m, 1H), 4.24 (s, 2H), 3.11 (s, 1H), 2.24 (bs, 2H), 1.95-1.89 (m, 6H), 1.75 (m, 4H), 1.67 (m, 2H). | 6,2 |
| | | | | Solide jaune |
| | | | | 28% |
| | | | ESI+MS: calcd for C₁₇H₂₁F₂N: 277.16; found: 278.2 (MH⁺) | |
| *53* | picolinaldehyde | *N*-((pyridin-2-yl)methyl)-2-adamantylamine | ESI+MS: calcd for C₁₆H₂₂N₂: 242.18; found: 243.2 (MH⁺) | 1 |
| | | | | huile jaune |
| | | | | 71% |
| *54* | nicotinaldehyde | *N*-((pyridin-3-yl)methyl)-2-adamantylamine | ¹H NMR (CDCl₃, 400MHz): δ 8.62 (s, 1H), 8.55 (d, 1H, *J* = 4.4 Hz), 8.00 (s, 1H), 7.32 (m, 1H), 3.99 (s, 2H), 2.91 (s, 1H), 2.17-1.57 (m, 14H). | 1 |
| | | | | solide jaune 79% |
| | | | ¹³C NMR (CDCl₃, 100MHz): δ 150.0, 149.1, 137.8, 130.7, 123.8, 60.9, 46.5, 37.3, 37.0, 30.6, 30.1, 27.1, 26.9, 22.0. | |
| | | | ESI+MS: calcd for C₁₆H₂₂N₂: 242.18; found: 243.2 (MH⁺) | |
| *55* | isonicotinaldehyde | *N*-((pyridin-4-yl)methyl)-2-adamantylamine | ¹H NMR (CDCl₃, 400MHz): δ 8.58 (m, 2H), 7.44 (d, 1H, *J* = 4.0 Hz), 7.31 (d, 1H, *J* = 5.6 Hz), 3.95 (s, 2H), 2.86 (s, 1H), 2.17-1.56 (m, 14H). | 1 |
| | | | | solide jaune |
| | | | | 46% |
| | | | ¹³C NMR (CDCl₃, 100MHz): δ 150.0, 149.1, 137.8, 130.7, 123.8, 60.9, 46.5, 37.3, 37.0, 30.6, 30.1, 27.1, 26.9, 22.0. | |
| | | | ESI+MS: calcd for C₁₆H₂₂N₂: 242.18; found: 243.2 (MH⁺) | |
| *56* | 5-methylfuran-2-carbaldehyde | *N*-((5-methylfuran-2-yl)methyl)-2-adamantylamine | ¹H NMR (CDCl₃, 400MHz): δ 6.33 (d, 1H, *J* = 2.8 Hz), 5.93 (d, 1H, *J* = 2.0 Hz), 4.80 (bs, 1H), 4.01 (s, 2H), 3.00 (s, 1H), 2.28 (s, 3H), 2.16-1.59 (m, 14H). | 1 |
| | | | | huile jaune |
| | | | | 59% |
| | | | ¹³C NMR (CDCl₃, 100MHz): δ 152.8, 146.3, 111.7, 106.7, 60.7, 41.4, 37.4, 37.1, 30.7, 29.9, 27.2, 26.9, 13.6. | |
| | | | ESI+MS: calcd for C₁₆H₂₃NO: 245.18; found: 246.2 (MH⁺) | |
| *57* | 5-methylthiophene-2-carbaldehyde | *N*-((5-methylthiophen-2-yl)methyl)-2-adamantylamine | ¹H NMR (CDCl₃, 400MHz): δ 6.86 (d, 1H, *J* = 3.2 Hz), 6.62 (d, 1H, *J* = 2.4 Hz), 5.39 (bs, 1H), 4.10 (s, 2H), 3.00 (s, 1H), 2.46 (s, 3H), 2.14-1.57 (m, 14H). | 1 |
| | | | | Huile jaune |
| | | | | 55% |
| | | | ESI+MS: calcd for C₁₆H₂₃NS: 261.16; found: 262.1 (MH⁺) | |
| *58* | furan-3-carbaldehyde | *N*-((furan-3-yl)methyl)-2-adamantylamine | ¹H NMR (CDCl₃, 400MHz): δ 7.58 (d, 1H, *J* = 6.8 Hz), 7.42 (s, 1H), 6.74 (d, 1H, *J* = 13.6 Hz), 4.01 (s, 2H), 3.07 (s, 1H), 2.28-1.42 (m, 14H). | 1 |
| | | | | Solide orange |
| | | | | 61% |
| | | | ESI+MS: calcd for C₁₅H₂₁NO: 231.16; found: 232.1 (MH⁺) | |
| 59 | 1-methyl-1*H*-imidazole-5-carbaldehyde | *N*-((1-methyl-1*H*-imidazol-5-yl)methyl)-2-adamantylamine | ¹H NMR (CDCl₃, 400MHz): δ 7.62 (s, 1H), 7.01 (s, 1H), 5.75 (bs, 1H), 3.95 (s, 2H), 3.80 (s, 3H), 2.99 (s, 1H), 2.14-1.58 (m, 14H). | 1 |
| | | | | Solide blanc |
| | | | | 100% |
| | | | ¹³C NMR (CDCl₃, 100MHz): δ 138.9, 129.0, 124.8, 61.2, 37.6, 37.2, 36.9, 32.1, 30.6, 30.0, 29.9, 27.0, 26.8. ESI+MS: calcd for C₁₅H₂₃N₃: 245.19; found: 246.1 (MH⁺) | |
| 60 | 1-methyl-1*H*-imidazole-2-carbaldehyde | *N-[(5-N-*methylimidazolyl)methyl]-2-adamantylamine | ¹H NMR (CDCl₃, 400MHz): δ 7.08 (bs, 1H), 7.02 (d, 1H, *J* = 1.2 Hz), 6.88 (d, 1H, *J* = 1.2 Hz), 4.12 (s, 2H), 3.80 (s, 3H), 3.14 (s, 1H), 2.13-1.58 (m, 14H). | 1 |
| | | | | solide blanc |
| | | | | 100% |
| | | | ¹³C NMR (CDCl₃, 100MHz): δ 141.0, 126.2, 122.4, 62.3, 39.7, 37.2, 36.9, 33.6, 30.5, 29.8, 27.0,26.8. | |
| | | | ESI+MS: calcd for C₁₅H₂₃N₃: 245.19; found: 246.2 (MH⁺) | |
| *61* | Benzo[d][1,3]dioxole-4-carbaldehyde | Benzo[d][1,3]dioxol-4-yl)methyl)-2-adamantylamine | ¹H NMR (CDCl₃, 400MHz): δ 6.84 (d, 1H, *J* = 7.6 Hz), 6.80 (t, 1H, *J =* 7.6 Hz), 6.74 (dd, 1H, *J =* 1.2 and 7.6 Hz), 5.96 (s, 2H), 3.79 (s, 2H), 2.78 (s, 1H), 2.11 (s, 1H), 2.05-1.49 (m, 14H). | 2 |
| | | | | Solide blanc |
| | | | | 9% |
| | | | ESI+MS: calcd for C₁₈H₂₃NO₂: 285.17; found: 286.2 (MH⁺) | |
| 62 | 6-nitro[d][1,3]dioxole-5-carbaldehyde | *N*-((5-nitrobenzo[d][1,3]dioxol-6-yl)methyl)-2-adamantylamine | ESI+MS: calcd for C₁₈H₂₂N₃O₄: 330.16; found: 331.2 (MH⁺) | 1 |
| | | | | Solide marron |
| | | | | 62% |
| *63* | Quinoline-3-carbaldehyde | *N*-((quinolin-3-yl)methyl)-2-adamantylamine | ¹H NMR (CDCl₃, 400MHz): δ 8.87 (d, 1H, *J* = 4.4 Hz), 8.15 (m, 2H), 7.41 (m, 1H), 7.56 (m, 2H), 6.99 (bs, 1H), 4.30 (s, 2H), 2.92 (s, 1H), 2.21-1.54 (m, 14H). | 2 |
| | | | | solide jaune |
| | | | | 100% |
| | | | ESI+MS: calcd for C₂₀H₂₄N₂: 292.19; found: 293.2 (MH⁺) | |
| *64* | Quinoline-4-carbaldehyde | *N*-((quinolin-4-yl)methyl)-2-adamantylamine | ¹H NMR (CDCl₃, 400MHz): δ 8.87 (d, 1H, *J* = 4.4 Hz), 8.15 (m, 2H), 7.41 (m, 1H), 7.56 (m, 2H), 6.99 (bs, 1H), 4.30 (s, 2H), 2.92 (s, 1H), 2.21-1.54 (m, 14H). | 1 |
| | | | | Solide jaune |
| | | | | 100% |
| | | | ESI+MS: calcd for C₂₀H₂₄N₂: 292.19; found: 293.2 (MH⁺) | |
| *65* | 1-methyl-1*H*-indole-2-carbaldehyde | *N*-((1-methyl-1*H*-indol-2-yl)methyl)-2-adamantylamine | ¹H NMR (CDCl₃, 400MHz): δ 7.56 (d, 1H, *J* = 8.0 Hz), 7.31 (d, 1H, *J* = 8 Hz), 7.22 (t, 1H, *J* = 7.6 Hz), 7.08 (t, 1H, *J* = 7.6 Hz), 6.59 (bs, 1H), 4.14 (s, 2H), 3.83 (s, 3H), 3.07 (s, 1H), 2.26-1.58 (m, 14H). | 1 |
| | | | | solide jaune |
| | | | | 100% |
| | | | ESI+MS: calcd for C₂₀H₂₆N₂: 294.21; found: 295.2 (MH⁺) | |
| *66* | 1-(3-bromophenyl)ethanone | *N*-(1-(3-bromophenyl)ethyl)-2-adamantylamine | ¹H NMR (CDCl₃, 400MHz): δ 7.54 (m, 1H), 7.41 (m, 2H), 7.26 (m, 2H), 4.88 (q, 1H, *J* = 6.4 Hz), 2.74 (bs, 1H), 2.08-1.53 (m, 14H), 1.49 (d, 3H, *J* = 6.4 Hz). | 4 |
| | | | | huile incolore |
| | | | | 18% |
| | | | ¹³C NMR (CDCl₃, 100MHz): δ 147.6, 130. 1, 130.0, 129.9, 125.4, 122.5, 59.2, 54.7, 32.5, 31.3, 31.1, 30.6, 27.6, 27.4, 24.2. | |
| | | | ESI+MS: calcd for C₁₈H₂₄BrN: 333.11; found: 334.2 (MH⁺) | |
| *67* | benzophenone | *N*-benzhydryl-2-adamantylamine | ¹H NMR (CDCl₃, 400MHz): δ 7.73 (bs, 1H), 7.29 (m, 10H), 4.57 (t, 1H, *J* = 7.6 Hz), 3.48 (dd, 1H, *J* = 1.2 and 7.6 Hz), 3.06 (s, 1H), 2.08 (s, 4H), 1.83 (m, 4H), 1.71-1.64 (m, 6H). | 4 |
| | | | | solide blanc |
| | | | | 3% |
| | | | ¹³C NMR (CDCl₃, 100MHz): δ 140.7, 129.0, 127.9, 127.3, 61.8, 49.5, 48.1, 37.0, 36.9, 30.5, 29.4, 26.9, 26.5. | |
| | | | ESI+MS: calcd for C₂₄H₂₉N: 331.23; found: 331.9 (MH⁺) | |
| *68* | 2-(benzyloxy)acetaldehyd e | *N*-(2-(benzyloxy)ethyl)-2-adamantylamine | ¹H NMR (CDCl₃, 400MHz): δ 7.34 (m, 5H), 4.58 (s, 2H), 3.85 (m, 2H), 3.15 (m, 3H), 2.20-1.64 (m, 14H). | 1 |
| | | | | solide blanc |
| | | | | 79% |
| | | | ESI+MS: calcd for C₁₉H₂₇NO: 285.21; found: 286.3 (MH⁺) | |
| *69* | 3-phenylpropanal | *N*-(phenylpropyl)-2-adamantylamine | ¹³C NMR (CDCl₃, 100MHz): δ 140.8, 128.4, 128.3, 126.0, 62.5, 45.7, 37.3, 37.1, 33.3, 30.5, 29.9, 28.5, 27.2, 26.8. | 1 |
| | | | | solide blanc |
| | | | | 100% |
| | | | ESI+MS: calcd for C₁₉H₂₇N: 269.22; found: 270.2 (MH⁺) | |
| *70* | acetophenone | *N*-(1-phenylethyl)-2-adamantylamine | ¹H NMR (CDCl₃, 400MHz): δ 7.57 (d, 2H, *J* = 11.2 Hz), 7.38 (m, 3H), 4.91 (q, 1H, *J* = 6.4 Hz), 2.87 (s, 1H), 2. 14-1.54 (m, 14H), 1.51 (d, 3H, J = 5.1 Hz). | 2 |
| | | | | huile incolore |
| | | | | 18% |
| | | | ESI+MS: calcd for C₁₈H₂₅N: 255.20; found: 256.3 (MH⁺) | |
| *71* | 1-(pyridin-2-yl)ethanone | *N*-(1-(pyridin-2-yl)ethyl)-2-adamantylamine | ¹H NMR (CDCl₃, 400MHz): δ 8.58 (d, 1H, *J* = 4.4 Hz), 7.73 (m, 1H), 7.48 (m, 1H), 7.26 (m, 1H), 6.51 (bs, 1H), 4.28 (m, 1H), 3.00 (bs, 1H), 2.18-1.51 (m, 14H), 1.62 (d, 3H, *J* = 6.4 Hz). ESI+MS: calcd for C₁₇H₂₄N: 256.19; found: 257.2 (MH⁺) | 1 |
| | | | | huile incolore 100% |
| *72* | 1-adamantyl methyl ketone | *N*-(-2-adamantylmethyl)-1-(adamantyl)ethanamine | ¹H NMR (CDCl₃, 400MHz): δ 3.25 (s, 1H), 2.50 (q, 1H, *J* = 5.1 Hz), 2.36 (s, 1H), 2.06-1.65 (m, 28H), 1.27 (d, 3H, *J* = 6.8 Hz). | 4 |
| | | | | solide blanc |
| | | | | 4,7% |
| | | | ESI+MS: calcd for C₂₂H₃₅N: 313.28; found: 314.2 (MH⁺) | |
| *73* | paraformaldehyde | *N*-methyl(-3bromobenzyl)-2-adamantylamine | ¹H NMR (CDCl₃, 400MHz): δ 7.50 (s, 1H), 7.35 (d, 1H, *J* = 8 Hz), 7.27 (d, 1H, *J* = 9.2 Hz), 7.17 (t, 1H, *J* = 7.6 Hz), 3.48 (s, 2H), 2.22-2.14 (m, 5H), 1.86 (m, 4H), 1.72 (m, 4H), 1.47 (m, 2H). | 2 |
| | | | | huile blanche |
| | | | | 35% |
| | | | ¹³C NMR (CDCl₃, 100MHz): δ 143.6, 131.5, 129.7, 129.5, 127.2, 122.3, 67.6, 57.5, 38.7, 37.8, 37.3, 31.6, 29.9, 27.6, 27.3. | |
| | | | ESI+MS: calcd for C₁₈H₂₄BrN: 333.11; found: 334.0 (MH⁺) | |
| *191* | cinnamaldehyde | *N*-cinnamyl-2-adamantylamine | ¹H NMR (CDCl₃, 400MHz): δ 7.42-7.27 (m, 5H), 6.63 (d, 1H, *J* = 15.6 Hz), 6.42 (dt, 1H, *J* = 6.8 and 16 Hz), 3.70 (d, 2H, *J* = 6.8 Hz), 3.18 (s, 1H), 2.20-1.43 (m, 14H). | Solide blanc |
| | | | | 100% |
| | | | ESI+MS: calcd for C₁₉H₂₅N: 267.20; found: 268.3 (MH⁺) | |

De même, les dérivés et les alkylamines de formule générale (Ib) telle que décrite ci-dessus et figurant dans le Tableau A3 et dans le Tableau A4 sont préparés en ajoutant sous agitation une suspension d'une amine (1 équiv.) choisie selon le composé à préparer (voir les Tableaux A3 et A4) dans du méthanol à du 3-bromobenzaldehyde ou du 3-fluorobenzaldehyde (1 équiv.) pour les composés du tableau A3 ou du 3-bromobenzylamine ou du 3-fluorobenzylamine (1 équiv.) pour obtenir les composés du tableau A4, en présence de BH₃CN sur résine (0,75 mmol ; 1,5 équiv) et d'acide acétique (1,5 mmol ; 84 µL; 3 équiv.). L'ensemble est agité 2 jours à température ambiante, filtré, lavé avec du méthanol, évaporé puis purifié selon les méthodes connues de l'homme du métier.

Les Tableaux A3 et A4 présentent le numéro du composé, les réactifs utilisés, le nom du composé obtenu, les caractéristiques du composé ainsi que le code de la méthode de purification utilisée, l'aspect du composé obtenu et son rendement.

**Tableau A3 : composés alkyle amine de formule générale (I) préparés par le procédé A à partir de 3-halogenobenzaldéhydes**

| **Composé** | **Réactif 1** | **Réactif 2** | **Nom du composé** | **structure du composé** | **Méthode de purification / aspect / rendement** |
|---|---|---|---|---|---|
| *74* | 3-bromobenzaldehyde | *tert*-Butylamine | *N*-(3-bromobenzyl)-2-methylpropan-2-amine | ¹H NMR (CDCl₃, 400MHz): δ 7.59 (s, 1H), 7.40 (d, 1H, *J* = 8 Hz), 7.36 (d, 1H, *J* = 7.6 Hz), 7.19 (t, 1H, *J* = 7.6 Hz), 4.78 (bs, 1H), 3.82 (s, 2H), 1.26 (s, 9H). | 1 |
| | | | | | Solide jaune |
| | | | | | 83% |
| | | | | ESI+MS: calcd for C₁₁H₁₆BrN: 241.05; found: 242.1 (MH⁺) | |
| *75* | 3-bromobenzaldehyde | 2,4,4-trimethylpenta n-2-amine | *N*-(3-bromobenzyl)-2,4,4-trimethylpentan-2-amine | ¹H NMR (CDCl₃, 400MHz): δ 7.54 (s, 1H), 7.39 (d, 1H, *J* = 8 Hz), 7.33 (d, 1H, *J* = 7.6 Hz), 7.18 (t, 1H, *J* = 8 Hz), 3.81 (s, 2H), 1.60 (s, 2H), 1.31 (s, 6H), 1.04 (s, 9H). | 2 |
| | | | | | solide jaune |
| | | | | | 26% |
| | | | | ¹³C NMR (CDCl₃, 100MHz): δ 140.0, 132.0, 130.6, 130.0, 127.6, 122.4, 57.2, 51.8, 45.3, 31.7, 27.5. | |
| | | | | ESI+MS: calcd for C₁₅H₂₄BrN: 297.11; found: 298.1 (MH⁺) | |
| *76* | 3-bromobenzaldehyde | 2-amino-3-hydroxy-2-methylpropano ic acid | 2-(3-bromobenzylamino) -3-hydroxy-2-methylpropanoic acid | ESI+MS: calcd for C₁₁H₁₄BrNO₃: 287.02; found: 288.1 (MH⁺) | 2 |
| | | | | | Solide blanc |
| | | | | | 18% |
| *77* | 3-bromobenzaldehyde | 2-am ino-2-(hydroxymethyl )propane-1,3-diol | 2-(3-bromobenzylamino) -2-(hydroxymethyl)pro pane-1,3-diol | ¹H NMR (CDCl₃, 400MHz): δ 7.54 (s, 1H), 7.41 (d, 1H, *J* = 8 Hz), 7.30 (d, 1H, *J* = 8 Hz), 7.21 (t, 1H, *J* = 7.6 Hz), 3.77 (s, 2H), 3.66 (s, 6H), 1.98 (bs, 4H). | 2 |
| | | | | | Huile incolore |
| | | | | | 10% |
| | | | | ESI+MS: calcd for C₁₁H₁₆BrNO₃: 289.03; found: 290.1 (MH⁺) | |
| *78* | 3-bromobenzaldehyde | cyclohexylamin e | *N*-(3-bromobenzyl)cyclo hexanamine | ¹H NMR (CDCl₃, 400MHz): δ 7.52 (s, 1H), 7.40 (d, 1H, *J* = 7.6 Hz), 7.29 (d, 1H, *J* = 7.6 Hz), 7.20 (t, 1H, *J* = 8 Hz), 3.86 (s, 2H), 2.56 (s, 1H), 2.02 (m, 2H), 1.95 (m, 2H), 1.75 (m, 2H), 1.64 (m, 2H), 1.20 (m, 2H). ¹³C NMR (CDCl₃, 100MHz): δ 138.9, 132.0, 130.8, 130.1, 127.6, 122.6, 55.8, 48.5, 31.3, 25.6, 24.8. ESI+MS: calcd for C₁₃H₁₈BrN: 267.06; found: 268.0 (MH⁺) | 1 |
| | | | | | solide jaune pale |
| | | | | | 100% |
| *79* | 3-bromobenzaldehyde | 4-aminocyclohex anol | 4-(3-bromobenzylamino) cyclohexanol | ¹H NMR (CDCl₃, 400MHz): δ 7.54 (s, 1H), 7.43 (d, 1H, *J* = 7.6 Hz), 7.32 (d, 1H, *J* = 7.6 Hz), 7.22 (t, 1H, *J* = 7.6 Hz), 5.28 (bs, 2H), 3.88 (s, 2H), 3.64 (m, 1H), 2.64 (m, 1H), 2.02 (m, 4H), 1.43-1.23 (m, 2H). | 1 |
| | | | | | solide blanc |
| | | | | | 100% |
| | | | | ¹³C NMR (CDCl₃, 100MHz): δ 137.6, 132.2, 131.3, 130.2, 127.7, 125.3, 69.6, 54.9, 48.8, 33.4, 28.7. | |
| | | | | ESI+MS: calcd for C₁₃H₁₈BrNO: 283.06; found: 284.0 (MH⁺) | |
| *80* | 3-fluorobenzaldéhyde | cyclohexylamin e | N-(3-fluorobenzyl)cycloh exylamine | ESI+MS: calcd for C₁₃H₁₈FN: 207.14; found: 208.12 (MH⁺) | 1 |
| | | | | | Solide blanc |
| | | | | | 100% |
| *81* | 3-fluorobenzaldéhyde | 4-aminocyclohex anol | 4-(3-fluorobenzylamino) cyclohexanol | ESI+MS: calcd for C₁₃H₁₈FNO: 223.14; found: 224.10 (MH⁺) | 1 |
| | | | | | Solide blanc |
| | | | | | 100% |
| *82* | 3-bromobenzaldehyde | (1-aminocyclopen tyl)methanol | (1-(3-bromobenzylamino) cyclopentyl)methan ol | ¹H NMR (CDCl₃, 400MHz): δ 7.55 (s, 1H), 7.40 (d, 1H, *J* = 8 Hz), 7.31 (d, 1H, *J* = 7.6 Hz), 7.18 (t, 1H, *J* = 7.6 Hz), 6.14 (bs, 2H), 3.77 (s, 2H), 3.47 (s, 2H), 1.72 (m, 4H), 1.57 (m, 4H). | 1 |
| | | | | | solide jaune pale |
| | | | | | 100% |
| | | | | ESI+MS: calcd for C₁₃H₁₈BrNO: 283.06; found: 284.1 (MH⁺) | |
| *83* | 3-bromobenzaldehyde | (1-aminocyclopen tyl)methanol | 1-(3-bromobenzylamino) cyclopentanecarbo xylic acid | ESI+MS: calcd for C₁₃H₁₆BrNO₂: 297.04; found: 298.1 (MH⁺) | 4 |
| | | | | | huile incolore |
| | | | | | 6,6% |
| *84* | 3-bromobenzaldehyde | bicyclo[2.2.1]h eptan-2-amine | N-(3-bromobenzyl)bicycl o[2.2.1]heptan-2-amine | ESI+MS: calcd for C₁₄H₁₈BrN: 279.06; found: 280.2 (MH⁺) | 2 |
| | | | | | huile jaune |
| | | | | | 23% |
| *85* | 3-bromobenzaldehyde | 2-aminobicyclo[2 .2.1]heptane-2-carboxylic acid | 2-(3-bromobenzylamino) bicyclo[2.2.1]hepta ne-2-carboxylic acid | ESI+MS: calcd for C₁₅H₁₈BrNO₂: 323.05; found: 324.1 (MH⁺) | 1 |
| | | | | | solide blanc |
| | | | | | 8% |
| *86* | 3-bromobenzaldehyde | noradamantyla mine | *N*-(3-bromobenzyl)-noradamantylamine | ¹H NMR (CDCl₃, 400MHz): δ 10.01 (bs, 1H), 7.48 (m, 2H), 7.31 (m, 3H), 3.93 (s, 2H), 2.27 (s, 2H), 2.11 (t, 1H, *J* = 6.4 Hz), 2.05 (d, 2H, *J* = 10 Hz), 1.78 (m, 4H), 1.57 (d, 1H, *J* = 12.8 Hz), 1.46-1.37 (m, 3H). | 2 |
| | | | | | solide blanc |
| | | | | | 88% |
| | | | | ¹³C NMR (CDCl₃, 100MHz): δ 132.1, 130.2, 128.7, 128.6, 69.9, 47.6, 45.4, 42.8, 42.0, 37.1, 34.2. | |
| | | | | ESI+MS: calcd for C₁₆H₂₀BrN: 305.08; found: 306.1 (MH⁺) | |
| *87* | 3-bromobenzaldehyde | 3-Am ino-1-adamantanol | N-(3-bromobenzyl)-1-hydroxy-2-adamantylamine | ¹H NMR (CDCl₃, 400MHz): δ 7.53 (s, 1H), 7.37 (d, 1H, *J* = 8 Hz), 7.26 (m, 1H), 7.18 (t, 1H, *J* = 7.6 Hz), 3.75 (s, 2H), 2.56 (s, 1H), 2.29 (s, 2H), 1.77-1.54 (m, 12H). | 2 |
| | | | | | huile jaune |
| | | | | | 17% |
| | | | | ¹³C NMR (CDCl₃, 100MHz): δ 138.9, 132.0, 130.8, 130.1, 127.6, 122.6, 55.8, 48.5, 31.3, 25.6, 24.8. | |
| | | | | ESI+MS: calcd for C₁₇H₂₂BrNO: 335.09; found: 336.1 (MH⁺) | |
| *88* | 3-bromobenzaldehyde | benzo[d][1,3]di oxol-5-amine | *N*-(3-bromobenzyl)-*N-*((benzo[*d*][1,3]diox ol-6-yl)methyl)(3-bromophenyl)meth anamine | | 2 |
| | | | | | 5% |
| *89* | 3-bromobenzaldehyde | 2-(4-hydroxybenzyl) -2-aminopropanoi c acid | 2-(3-bromobenzylamino) -2-(4-hydroxybenzyl)prop anoic acid | ESI+MS: calcd for C₁₇H₁₈BrNO₃: 363.05; found: 364.0 (MH⁺) | 2 |
| | | | | | solide blanc |
| | | | | | 2% |
| *90* | 3-bromobenzaldehyde | 2-(3,4-dihydroxybenz yl)-2-aminopropanoi c acid | 2-(3,4-dihydroxybenzyl)-2-(3-bromobenzylamino) propanoic acid | ESI+MS: calcd for C₁₇H₁₈BrNO₄: 379.04; found: 380.1 (MH⁺) | 4 |
| | | | | | huile jaune |
| | | | | | 20% |
| *91* | 3-bromobenzaldehyde | 2-amino-2-phenylbutanoic acid | 2-(3-bromobenzylamino) -2-phenylbutanoic acid | ESI+MS: calcd for C₁₇H₁₈BrNO₂: 347.05; found: 348.1 (MH⁺) | 2 |
| | | | | | solide blanc |
| | | | | | 9% |
| *92* | 3-bromobenzaldehyde | 2-am ino-2,2-diphenylacetic acid | 2-(3-bromobenzylamino) -2,2-diphenylacetic acid | ESI+MS: calcd for C₂₁H₁₈BrNO₂: 395.05; found: 396.1 (MH⁺) | 2 |
| | | | | | solide blanc |
| | | | | | 1,4% |
| *93* | 3-bromobenzaldehyde | methyl 2-amino-2-benzylpropano ate | methyl 2-(3-bromobenzylamino) -2-methyl-3-phenylpropanoate | ESI+MS: calcd for C₁₈H₂₀BrNO₂: 361.07; found: 362.1 (MH⁺) | 2 |
| | | | | | huile incolore |
| | | | | | 15,5% |
| *94* | 3-bromobenzaldehyde | | methyl 3-(3-bromobenzylamino) -8-azabicyclo[3.2.1]oct ane-8-carboxylate | | 6 |
| *95* | 3-bromobenzaldehyde | 8-methyl-8-aza-bicyclo[3.2.1]o ctan-3-one | *N-(3-*bromobenzyl)-9-methyl-9-azabicyclo[3.3.1] ] nonan-3-amine | ¹H NMR (CDCl₃, | 4 |
| | | | | 400MHz): δ 8.57 (bs, 1H), 7.49 (s, 1H), 7.39 (d, 1H, *J* = 7.6 Hz), 7.25 (d, 1H, *J* = 7.6 Hz), 7.20 (t, 1H, *J* = 7.6 Hz), 3.78 (s, 2H), 3.44 (m, 3H), 2.75 (s, 3H), 2.64 (m, 2H), 2.10 (m, 2H), 1.61-1.44 (m, 6H). | huile incolore |
| | | | | | 25% |
| | | | | ¹³C NMR (CDCl₃, 100MHz): δ 141.1, 131.4, 130.5, 130.1, 127.0, 122.6, 52.0, 50.6, 46.2, 38.3, 31.5, 24.2, 12.8. | |
| *96* | 3-bromobenzaldehyde | benzo[d][1,3]di oxol-5-amine | N-(3-bromobenzyl)benzo [d][1,3]dioxol-5-amine | ESI+MS: calcd for C₁₅H₁₄BrNO₂: 319.02; found: 320.1 (MH⁺) | 2 |
| | | | | | huile incolore |
| | | | | | 39% |
| *97* | 3-bromobenzaldehyde | 2-(3,4-dihydroxybenz yl)-2-aminopropanoi c acid | 2-(3-bromobenzylidenea mino)-2-(3,4-dihydroxyphenyl)pr opanoic acid | | 4 |
| | | | | | 10% |

**Tableau A4 : composés alkyle amine de formule générale (I) préparés par le procédé A à partir de 3-halogenobenzylamines**

| **Compo** | **Réactif 1** | **Réactif 2** | **Nom du composé** | **structure du composé** | **Méthode de purification / aspect / rendement** |
|---|---|---|---|---|---|
| *98* | 3-bromobenzylamine | benzaldehyde | *N*-benzyl(3-bromophenyl)methanamine | ¹H NMR (CDCl₃, 400 MHz): δ 7.52 (s, 1H), 7.46-7.23 (m, 9H), 5.62 (bs, 1H), 3.93 (s, 2H), 3.34 (s, 2H). ESI+MS: calcd for C₁₄H₁₄BrN: 275.03; found: 276.0 (MH⁺) | 1 |
| | | | | | solide blanc 78% |
| *99* | 3-bromobenzylamine | 3-bromobenzaldeh yde | bis(3-bromobenzyl)amine | ESI+MS: calcd for C₁₄H₁₃Br₂N: 352.94; found: 353.8 (MH⁺) | 1 |
| | | | | | solide blanc |
| | | | | | 75% |
| *100* | 3-bromobenzylamine | 3-fluorobenzaldehy de | *N*-(3-fluorobenzyl)(3-bromophenyl)meth anamine | ¹H NMR (CDCl₃, 400 MHz): δ 7.53 (s, 1H), 7.45 (d, 1H, *J* = 8 Hz), 7.33 (m, 3H), 7.13 (dd, 2H, *J* = 7.6 and 16 Hz), 7.02 (td, 1H, *J* = 2 and 8 Hz), 3.89 (s, 2H), 3.86 (s, 2H), 3.50 (bs, 1H). | 1 |
| | | | | | solide blanc |
| | | | | | 86% |
| | | | | ESI+MS: calcd for C₁₄H₁₃BrFN: 293.02; found: 293.9 (MH⁺) | |
| *101* | 3-bromobenzylamine | 1-methyl-1*H-*indole-2-carbaldehyde | *N*-(3-bromobenzyl)(1-methyl-1*H*-indol-2-yl)methanamine | ESI+MS: calcd for C₁₇H₁₇BrN₂: 328.06; found: 328.9 (MH⁺) | 1 |
| | | | | | solide jaune |
| | | | | | 27% |
| *102* | 3-bromobenzylamine | acetophenone | *N*-(3-bromobenzyl)-1-phenylethanamine | ¹H NMR (CDCl₃, 400 MHz): δ 7.47-7.18 (m, 9H), 3.94 (q, 1H, *J* = 6.4 Hz), 3.75 (d, 1H, *J* = 13.2 Hz), 3.61 (d, 1H, *J* = 13.6 Hz), 1.55 (d, 3H, *J* = 6.4 Hz). | 1 |
| | | | | | solide blanc |
| | | | | | 34% |
| *103* | 3-bromobenzylamine | 1-(3-bromophenyl)eth anone | *N*-(3-bromobenzyl)-1-(3-bromophenyl)ethan amine | ¹H NMR (CDCl₃, 400 MHz): δ 7.54-7.19 (m, 8H), 3.87 (q, 1H, *J* = 6.8 Hz), 3.73 (d, 1H, *J* = 13.6 Hz), 3.60 (d, 1H, *J* = 13.6 Hz), 1.50 (d, 3H, *J* = 6.8 Hz). | 1 |
| | | | | | solide blanc |
| | | | | | 43% |
| | | | | ESI+MS: calcd for C₁₅H₁₅Br₂N: 366.96; found: 367.8 (MH⁺) | |
| *104* | 3-bromobenzylamine | 1-(pyridin-2-yl)ethanone | N-(3-bromobenzyl)-1-(pyridin-2-yl)ethanamine | ¹H NMR (CDCl₃, 400 MHz): δ 8.63 (d, 1H, *J* = 4.4 Hz), 7.75 (t, 2H, *J* = 2 and 8 Hz), 7.47-7.18 (m, 5H), 4.21 (q, 1H, *J* = 6.8 Hz), 3.86 (d, 1H, *J* = 13.2 Hz), 3.76 (d, 1H, *J* = 13.2 Hz), 1.58 (d, 3H, *J* = 6.8 Hz). | 1 |
| | | | | | huile incolore |
| | | | | | 100% |
| | | | | ESI+MS: calcd for C₁₄H₁₅BrN₂: 290.04; found: 291.0 (MH⁺) | |
| *105* | 3-bromobenzylamine | quinuclidin-3-amine | *N*-(3-bromobenzyl)quinu clidin-3-amine | ¹³C NMR (CDCl₃, 100MHz): δ 141.5, 131.0, 130.4, 130.1, 126.7, 122.6, 63.8, 55.7, 53.4, 51.6, 50.6, 46.4, 46.2, 45.5, 45.3, 26.5, 24.2, 22.2, 17.4, 16.6. | 1 |
| | | | | | huile incolore |
| | | | | | 63% |
| | | | | ESI+MS: calcd for C₁₄H₁₉BrN₂: 294.07; found: 295.0 (MH⁺) | |
| *106* | 3-bromobenzylamine | 9-Methyl-9-azabicyclo[3.3.1] nonan-3-one | (1R*,5S*)-*N*-(3-bromobenzyl)bicycl o[3.3.1]nonan-9-amine | ¹H NMR (CDCl₃, 400MHz): δ 7.66 (s, 1H), 7.55 (d, 1H, *J* = 8 Hz), 7.47 (d, 1H, *J* = 8 Hz), 7.26 (m, 1H,), 4.02 (s, 2H), 2.80 (s, 1H), 2.08 (m, 2H), 1.88-1.47 (m, 12H). | 1 |
| | | | | | solide blanc |
| | | | | | 100% |
| | | | | ¹³C NMR (CDCl₃, 100MHz): δ 134.3, 133.0, 132.0, 130.4, 128.7, 122.7, 59.1, 47.7, 31.9, 29.2, 24.0, 21.3, 20.4. | |
| | | | | ESI+MS: calcd for C₁₆H₂₂BrN: 307.09; found: 308.0 (MH⁺) | |
| *107* | 3-bromobenzylamine | 8-methyl-8-aza-bicyclo[3.2.1]octa n-3-amine | *N*-(3-bromobenzyl)-8-methyl-8-aza-bicyclo[3.2.1]octan-3-amine | ¹³C NMR (CDCl₃, 100MHz): δ 142.1, 130.9, 130.2, 130.1, 126.6, 122.6, 62.0, 51.8, 49.0, 47.9, 46.3, 43.2, 24.9. | 1 |
| | | | | | huile incolore |
| | | | | | 100% |
| | | | | ESI+MS: calcd for C₁₅H₂₁BrN₂: 308.09; found: 309.0 (MH⁺) | |
| *108* | 3-bromobenzylamine | 1-adamantyl methyl ketone | *N*-(1-(3-bromophenyl)ethyl) adamantylamine | ¹³C NMR (CDCl₃, 100MHz): δ 137.4, 132.3, 131.4, 130.4, 127.9, 122.7, 60.9, 49.4, 38.0, 37.7, 37.2, 36.7, 35.5, 28.3, 28.1. | 1 |
| | | | | | solide blanc |
| | | | | | 84% |
| | | | | ESI+MS: calcd for C₁₉H₂₆BrN: 347.12; found: 348.0 (MH⁺) | |
| *109* | 3-fluorobenzylamine | noradamantylami ne | *N*-(3-fluorobenzyl)norad amantylamine | ESI+MS: calcd for C₁₆H₂₀FN: 245.16; found: 246.09 (MH⁺) | 1 |
| | | | | | 100% |

Les composés du Tableau A5 sont réalisés selon le procédé A en ajoutant une étape de chauffage au micro-onde.

**Tableau A5**

| **Composé** | **Nom du composé** | **caractéristiques du composé** | **Méthode de purification / aspect / rendement** |
|---|---|---|---|
| *138* | *N*-(3-chlorobenzyl)adamantylamine | ¹H NMR (CDCl₃, 400MHz): δ 7.38 (s, 1H), 7.26 (m, 3H), 6.86 (bs, 1H), 3.82 (s, 2H), 2.13 (s, 3H), 1.79-1.63 (m, 12H). ¹³C NMR (CDCl₃, 100MHz): δ 139.2, 134.2, 129.7, 129.3, 127.8, 127.4, 53.9, 43.6, 40.7, 40.6, 36.2, 29.3. | 1 |
| | | | solide blanc |
| | | | 88% |
| | | ESI+MS: calcd for C₁₇H₂₂ClN: 275.14; found: 276.1 (MH⁺) | |
| *139* | *N*-(3-chlorobenzyl)-2-adamantylamine | ¹H NMR (CDCl₃, 400MHz): δ 7.47 (s, 1H), 7.41-7.28 (m, 3H), 3.99 (s, 2H), 2.97 (s, 1H), 2.13 (m, 4H), 1.87 (m, 4H), 1.73-1.59 (m, 6H). | 1 solide blanc |
| | | ¹³C NMR (CDCl₃, 100MHz): δ 135.3, 134.6, 130.1, 129.8, 128.8, 127.9, 60.8, 48.3, 37.3, 37.0, 30.6, 29.8, 27.1, 26.8. | 88% |
| | | ESI+MS: calcd for C₁₇H₂₂ClN: 275.14; found: 276.2 (MH⁺) | |
| *140* | *N*-(3-iodobenzyl)-2-adamantylamine | ¹H NMR (CDCl₃, 400MHz): δ 7.80 (s, 1H), 7.65 (d, 1H, *J* = Hz), 7.49 (d, 1H, *J* = 7.2 Hz), 7.11 (t, 1H, *J=* 7.6 Hz), 3.93 (s, 2H), 2.94 (s, 1H), 2.15 (m, 4H), 1.89 (m, 4H), 1.73-1.59 (m, 6H). | 1 |
| | | | solide blanc |
| | | | 100% |
| | | ¹³C NMR (CDCl₃, 100MHz): δ 138.6, 137.7, 135.7, 130.5, 129.0, 94.4, 60.9, 48.2, 37.3, 37.0, 30.6, 29.8, 27.1, 26.8. | |
| | | ESI+MS: calcd for C₁₇H₂₂IN: 367.08; found: 367.6 (MH⁺) | |
| *141* | N-(2,2-diphenylethyl)-2-adamantylamine | ¹H NMR (CDCl₃, 400MHz): δ 7.73 (bs, 1H), 7.29 (m, 10H), 4.57 (t, 1H, *J* = 7.6 Hz), 3.48 (dd, 1H, *J=* 1.2 and 7.6 Hz), 3.06 (s, 1H), 2.08 (s, 4H), 1.83 (m, 4H), 1.71-1.64 (m, 6H). | 1 |
| | | | Solide blanc |
| | | | 100% |
| | | ¹³C NMR (CDCl₃, 100MHz): δ 140.7, 129.0, 127.9, 127.3, 61.8, 49.5, 48.1, 37.0, 36.9, 30.5, 29.4, 26.9, 26.5. | |
| | | ESI+MS: calcd for C₂₄H₂₉N: 331.23; found: 331.9 (MH⁺) | |
| *142* | N-(naphthalen-1-ylmethyl)-2-adamantylamine | ¹H NMR (CDCl₃, 400MHz): δ 8.14 (d, 1H, *J* = 8.4 Hz), 7.88 (d, 1H, *J* = Hz), 7.84 (d, 1H, *J* = 8.4 Hz), 7.76 (bs, 1H), 7.68 (d, 1H, *J* = 7.2 Hz), 7.59 (t, 1H, *J* = 7.2 Hz), 7.49 (m, 2H), 4.51 (s, 2H), 3.13 (s, 1 H), 2.14 (m, 4H), 1.86 (m, 4H), 1.72-1.60 (m, 6H). | 1 |
| | | | solide blanc |
| | | | 100% |
| | | ¹³C NMR (CDCl₃, 100MHz): δ 133.7, 131.7, 129.5, 129.2, 128.9, 128.8, 126.9, 126.0, 125.4, 122.9, 61.6, 46.1, 37.2, 37.0, 30.6, 30.1, 27.0, 26.8. | |
| | | ESI+MS: calcd for C₂₁H₂₅N: 291.2; found: 291.8 (MH⁺) | |
| *143* | N-(phenanthren-9-ylmethyl)-2-adamantylamine | ¹H NMR (CDCl₃, 400MHz): δ 8.74 (m, 1H), 8.65 (d, 1H, *J* = 8 Hz), 8.20 (bs, 1H), 7.91 (m, 2H), 7.70-7.57 (m, 4H), 4.48 (s, 2H), 3.14 (s, 1H), 2.14 (m, 2H), 2.05 (m, 2H), 1.87 (m, 4H), 1.74-1.60 (m, 6H). | 1 |
| | | | solide blanc |
| | | | 100% |
| | | ¹³C NMR (CDCl₃, 100MHz): δ 131.0, 130.7, 130.5, 130.2, 129.8, 128.9, 127.9, 127.3, 127.2, 127.0, 126.9, 126.8, 123.7, 123.4, 122.4, 61.9, 46.8, 37.3, 37.1, 30.7, 30.2, 30.2, 27.1, 26.9. | |
| | | ESI+MS: calcd for C₂₅H₂₇N: 341.21; found: 341.8 (MH⁺) | |
| 144 | 4-((adamantylamino)methyl)benzoic acid | ¹H NMR (CDCl₃, 400MHz): δ 7.83 (d, 2H, *J* = 8.4 Hz), 7.49 (d, 2H, *J* = 8 Hz), 4.16 (s, 2H), 3.27 (s, 1H), 2.32-1.67 (m, 14H). ¹³C NMR (CDCl₃, 100MHz): δ 170.4, 134.5, 133.5, 130.2, 129.8, 61.2, 48.2, 36.9, 36.7, 30.1, 29.0, 26.7, 26.5. | 1 |
| | | | Solide blanc |
| | | | 100% |
| | | ESI+MS: calcd for C₁₈H₂₃NO₂: 285.17; found:286.1 (MH⁺) | |

| | | | |
|---|---|---|---|
| **Procédé B : Formation des sels d'acides** | | | |

A une solution dans le CH₂Cl₂ de l'amine (correspondant au sel du chlorhydrate désiré) est ajouté une solution de HCl dans l'Et₂O (2N). Le sel est obtenu après filtration et séchage sous vide du précipitât.

**Tableau B1 : sels dérivés des 1 ou 2-aminoadamantane**

| **Composé** | **Nom du composé** | **caractérisitiques du composé** | **Méthode de purification / aspect / rendement** |
|---|---|---|---|
| *110* | *N*-(3-bromobenzyl)adamantylamine hydrochloryde salt | ¹H NMR (CDCl₃, 400MHz): δ 9.55 (bs, 1H), 7.81 (s, 1H), 7.66 (d, 1H, *J* = 7.6 Hz), 7.39 (d, 1H, *J* = 8 Hz), 7.23 (t, 1H, *J* = Hz), 3.86 (t, 2H, *J* = 6 Hz), 2.12 (s, 3H), 1.95 (s, 5H), 1.65 (s, 4H), 1.55 (s, 3H). | 1 Solide blanc |
| *111* | *N*-(5-bromo-2-methoxybenzyl)adamantylamine hydrochloryde salt | ¹H NMR (CDCl₃, 400MHz): δ 8.94 (bs, 1H), 7.66 (d, 1H, *J* = 2.4 Hz), 7.38 (dd, 1H, *J* = 2.4 and 8.8 Hz), 6.75 (d, 1H, *J* = 8.4 Hz), 3.91 (s, 5H), 2.13 (s, 3H), 1.89, 1.67 and 1.53 (3s, 12H). | 1 |
| | | | Solide blanc |
| | | ¹³C NMR (DMSO-d⁶, 100MHz): δ 155.7, 133.2, 131.8, 121.2, 111.2, 110.5, 56.1, 54.4, 36.6, 36.0, 34.1, 27.5. | |
| *112* | *N*-(2-bromobenzyl)-2-adamantylamine hydrochloryde salt | ¹H NMR (CDCl₃, 400MHz): δ 9.84 (bs, 1H), 7.78 (s, 1H), 7.51 (dd, 1H, *J* = 2.4 and 10.8 Hz), 7.30 (m, 2H), 4.18 (s, 2H), 3.05 (s, 1H), 2.48-1.55 (s, 14H). | 1 |
| | | | Solide blanc |
| | | ¹³C NMR (CDCl₃, 100MHz): δ 1133.3, 132.5, 132.4, 130.7, 129.1, 122.8, 60.2, 47.4, 37.1, 36.7, 30.4, 29.0, 26.8, 26.6. | |

### Procédé C

Les dérivés de 1-aminoadamantane, de 2-aminoadamantane ou de noradamantylamine de formule générale (Ic) telle que décrite ci-dessus et figurant, respectivement dans les Tableaux C1, C2 et C3 sont préparés comme suit : une suspension agitée dans du méthanol de 1-, 2- aminoadamantane ou noradamantylamine (1 équiv.) est ajoutée à un aldéhyde (1 équiv.) choisi selon le composé à préparer (voir les Tableaux C1, C2 et C3). L'ensemble est mélangé pendant deux jours à température ambiante puis évaporé.

**Tableau C1 : composés dérivés d'1-aminoadamantane préparés par le procédé C à partir de 1-adamantylamine**

| **Composé** | **aldéhyde** | **Nom du composé** | **caractérsitiques du composé** | **Méthode de purification / aspect / rdt** |
|---|---|---|---|---|
| *113* | 3-bromobenzaldehyde | (*E*)-*N*-(3-bromobenzylidene)adamantyla mine | ¹H NMR (DMSO, 400MHz): δ 8.30 (s, 1H), 7.94 (s, 1H), 7.74 (d, 1H, *J* = 7.6 Hz), 7.62 (d, 1H, *J* = 7.6 Hz), 7.40 (t, 1H, *J* = 7.6 Hz), 2.12 (s, 3H), 1.74-1.64 (m, 12H). | Solide blanc |
| *114* | 3-fluorobenzaldehyde | (*E*)-*N*-(3-fluorobenzylidene)adamantyla mine | ¹H NMR (DMSO, 400MHz): δ 8.32 (s, 1H), 7.59 (d, 1H, *J* = 8 Hz), 7.55-7.45 (m, 2H), 7.27 (m, 1H), 2.12 (s, 3H), 1.75-1.64 (m, 12H). | Solide jaune 90% |
| *115* | 1-methyl-1*H*-indole-2-carbaldehyde | (*E*)-*N*-((1-methyl-1*H*-indol-2-yl)methylene)adamantylamine | ¹H NMR (DMSO, 400MHz): δ 8.41 (s, 1H), 7.58 (d, 1H, *J* = 7.6 Hz), 7.46 (dd, 1H, *J* = 2.4 and 8.4 Hz), 7.23 (t, 1H, *J* = 7.6 Hz), 7.05 (t, 1H, *J* = 7.6 Hz), 6.86 (s, 1H), 4.09 (s, 3H), 2.12 (s, 3H), 1.77-1.64 (m, 12H). | Solide brun 55% |
| | | | ¹³C NMR (CDCl₃, 100MHz): δ 147.8, 139.8, 136.4, 127.0, 126.9, 123.3, 121.3, 119.7, 109.6, 108.2, 57.8, 43.1, 36.6, 29.6. | |
| | | | ESI+MS: calcd for C₂₀H₂₄N₂: 292.19; found: 293.2 (MH⁺) | |
| *116* | benzaldehyde | (*E*)-*N-*benzylideneadamantylamine | ¹H NMR (DMSO, 400MHz): δ 8.31 (s, 1H), 7.75 (m, 2H), 7.42 (m, 3H), 2.12 (s, 3H), 1.75-1.64 (m, 12H). | Solide brun 99% |

**Tableau C2 : composés dérivés de 2-aminoadamantane préparés par le procédé C à partir de 2-adamantylamine**

| **Composé** | **aldéhyde** | **Nom du composé** | **caractéristiques du composé** | **Méthode de purification/ aspect / rdt** |
|---|---|---|---|---|
| *117* | 3-bromobenzaldehyde | (*E*)*-N-*(3-bromobenzylidene)adamantyla mine | ¹H NMR (CDCl₃, 400MHz): δ 8.52 (bs, 1H), 7.62 (s, 1H), 7.46 (d, 1H, *J* = 8 Hz), 7.38 (d, 1H, *J* = Hz),7.23 (d, 1H, *J=* 7.6 Hz), 3.52 (s, 1H), 2.23 (s, 5H), 1.96-1.66 (m, 9H). | Solide blanc 60% |
| *118* | 3-fluorobenzaldehyde | (*E*)-*N*-(3-fluorobenzylidene)adamantyla mine | ¹H NMR (CDCl₃, 400MHz): δ 8.52 (bs, 1H), 7.34 (m, 1H), 7.23 (d, 1H, *J* = 7.6 Hz), 7.18 (d, 1H, *J* = 9.6 Hz),7.02 (dt, 1H, *J* = 2 and 8 Hz), 3.51 (s, 1H), 2.23 (s, 3H), 1.96-1.66 (m, 11H). | solide blanc 74% |

**Tableau C3 : composés préparés par le procédé C à partir de noradamantylamine**

| **Composé** | **Réactif 2** | **Nom du composé** | **caractéristiques du composé** | **rendement** |
|---|---|---|---|---|
| *119* | 3-bromobenzaldehyde | (*E*)-*N-*(3-bromobenzylidene)noradaman tylamine | | 98% |
| *120* | 1-methyl-1*H*-indole-2-carbaldehyde | (*E*)-*N*-((1-methyl-1*H*-indol-2-yl)methylene)noradamantylami ne | | 100% |

### Procédé E : Benzoylation

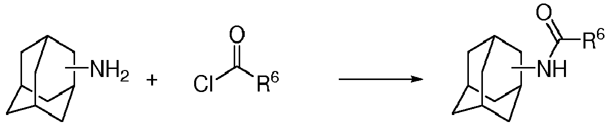

Les composés *N-*1-adamantylbenzamide et *N*-2-adamantylbenzamide sont préparés comme suit : à une suspension, de NaH (1,1 équiv.) dans le DMF est ajouté la 1-ou 2-adamantylamine (1 équiv.) et du benzoyle chloride (1,2 équiv.) à 0°C. Le mélange est agité 24 heures à température ambiante, évaporé puis lavé avec du cyclohexane.

**Tableau D1 : composés dérivés d'1-aminoadamantane préparés par le procédé E préparés à partir de 1-adamantylamine**

| **Numéro du Compo** | **Réactif 2** | **Nom du composé** | **caractéristiques du composé** | **Méthode de purification/ aspect / rendement** |
|---|---|---|---|---|
| *121* | benzoyle chloride | *N*-1-adamantylbenzamide | ¹H NMR (CDCl₃, 400MHz): δ 7.72 (dd, 2H, *J* = 1.2 and 7.2 Hz), 7.46 (m, 3H), 5.79 (bs, 1H), 2.13 (s, 9H), 1.73 (m, 6H). | 6 |
| | | | | solide blanc 74% |
| | | | ¹³C NMR (CDCl₃, 100MHz): δ 166.6, 136.0, 131.0, 128.4, 126.7, 52.3, 41.7, 36.4, 29.5. | |
| *122* | benzoyle chloride | *N*-2-adamantylbenzamide | ¹H NMR (CDCl₃, 400MHz): δ 7.78 (dd, 2H, *J* = 1.6 and 6.8 Hz), 7.49 (m, 3H), 6.42 (bs, 1H), 4.27 (m, 1H), 2.06-1.55 (m, 14H). | 6 |
| | | | | Solide blanc 100% |
| | | | ESI+MS: calcd for C₁₇H₂₁NO: 255.16; found: 256.2 (MH⁺) | |

La sulfonylation est réalisée selon le procédé suivant:

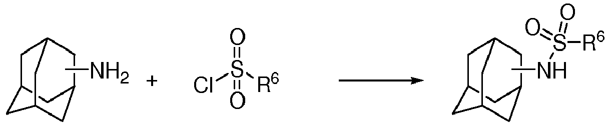

A une solution de chlorhydrate de 1-adamantylamine ou de 2-adamantylamine (0.2 mmol; 1 équiv.) in CH₂Cl₂ (1 mL) est ajouté du Et₃N (138µL; 1 mmol; 5 équiv.) et du benzenesulfonyl chloride (76µL; 0.6 mmol; 3 équiv.). Le mélange est agité à température ambiante pendant 24h, hydrolyse avec NH₄Cl. La phase organique est lavée 3 fois avec de l'eau, séchée sur MgSO₄, filtrée et évaporée pour donner le produit attendu.

### Préparation du N-1-adamantylsulfonamide (composé 124)

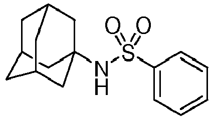

à partir du 1-adamantylamine, le produit obtenu est un solide brun (86%).
¹H NMR (CDCl₃, 400MHz): δ 7.91 (dd, 2H, *J* = 1.6 and 7.2 Hz), 7.53 (m, 3H), 2.01 (s, 3H), 1.79 (m, 6H), 1.58 (m, 6H).
¹³C NMR (CDCl₃, 100MHz): δ 143.9, 132.0, 128.8, 126.8, 55.2, 43.0, 35.0, 29.4.

### Préparation du N-2-adamantylsulfonamide (composé 128)

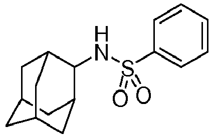

à partir du 2-adamantylamine, le produit obtenu est un solide jaune (86%)
¹H NMR (CDCl₃, 400MHz): δ 7.89 (dd, 2H, *J* = 1.9 and 7.2 Hz), 7.55 (m, 3H), 3.43 (s, 1 H), 1.79-1.53 (m, 14H).
¹³C NMR (CDCl₃, 100MHz): δ 141.2, 132.3, 129.0, 126.8, 57.9, 37.3, 37.1, 32.7, 31.1, 26.8, 26.7.

### Addition nucléophile

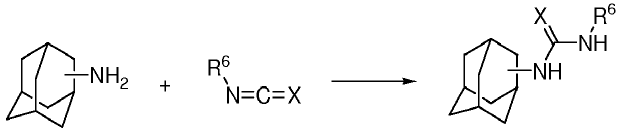

à une suspension agitée de 1-adamantylamine (0.5 mmol; 1 équiv.) dans le THF (3 mL) est ajouté à 0°C du phénylisocyanate ou du phénylthioisocyanate (0.75 mmol; 1.5 équiv.). Le mélange est agité 24 heures à température ambiante puis évaporé ;
ou
à une suspension agitée de chlorhydrate de 2-adamantylamine (0.5 mmol; 1 équiv.) dans le DMF (3 mL) est ajouté à 0°C du phénylisocyanate ou du phénylthioisocyanate (0.75 mmol; 1.5 équiv.) et de la triéthylamine (1.05 équiv.). Le mélange est agité 24 heures à température ambiante, puis évaporé, mis en solution dans Et₂O, filtré puis le filtrat est évaporé.

Ce procédé permet de préparer les composés suivants :

### Préparation du 1-Adamantan-1-yl-3-phenyl-urea (composé 129)

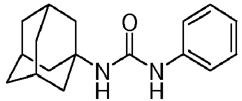

à partir de 1-adamantylamine et de phenylisocyanate, on obtient un solide blanc (95%)
¹H NMR (CDCl₃, 400MHz): δ 7.35 (m, 3H), 7.11 (m, 2H), 2.18 (s, 3H), 2.00 (s, 6H), 1.68 (s, 6H).
¹³C NMR (CDCl₃, 100MHz): δ 160.5, 138.7, 129.2, 123.7, 121.0, 51.5, 42.2, 36.3, 29.5.
ESI+MS: calcd for C₁₇H₂₂N₂O: 270.17; found: 271.1 (MH⁺)

### Préparation de 1-Adamantan-2-yl-3-phenyl-urea (composé 132)

À une suspension agitée de chlorhydrate de 2-adamantylamine (0.5 mmol; 1 équiv.) dans le DMF (3 mL) est ajouté à 0°C du phenylisocyanate ou du phenylthioisocyanate (0.75 mmol; 1.5 équiv.) et de la triéthylamine (1.05 équiv.). Le mélange est agité 24 h à température ambiante, évaporé, mis en solution dans Et₂O, filtré puis le filtrat est évaporé. 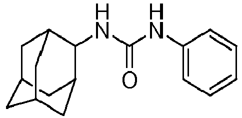 on obtient un solide blanc (69%).
¹H NMR (CDCl₃, 400MHz): δ 7.34 (m, 4H), 7.11 (t, 1 H, *J* = 6.8 Hz), 3.96 (s, 1 H), 1.96 (s, 2H), 1.84-1.61 (m, 12H).
ESI+MS: calcd for C₁₇H₂₂N₂O: 270.17; found: 271.1 (MH⁺)

Le procédé qui suit permet de préparer les composés ci-dessous : à une suspension agitée de chlorhydrate de 1-adamantylamine ou de 2-adamantylamine (0.5 mmol; 1 équiv.) dans le CH₂Cl₂ (4 mL) est ajouté à température ambiante du phenyl chloroformate (0.6 mmol; 1.2 équiv.) et de la triéthylamine (2.5 mmol; 5 équiv.). Le mélange est agité 24 h à température ambiante, lavé à l'eau, mis en solution saturée de NH₄Cl, séché sur Na₂SO₄, filtré, évaporé et purifié sur short pad.

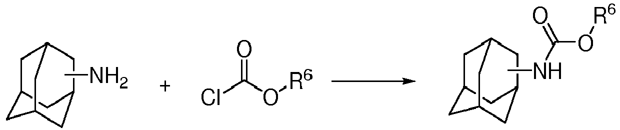

### Préparation de Adamantan-1-yl-carbamic acid phenyl ester (composé 123)

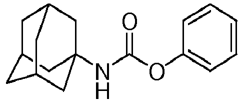

à partir de1-adamantylamine et de phenyl chloroformate, on obtient un solide blanc (13%).
¹H NMR (CDCl₃, 400MHz): δ 7.35 (t, 2H, *J* = 8 Hz), 7.18 (t, 1 H, *J* = 7.2 Hz), 7.12 (d, 2H, *J* = 7.6 Hz), 4.88 (bs, 1 H), 2.11 (s, 3H), 2.01 (s, 6H), 1.69 (s, 6H).
¹³C NMR (CDCl₃, 100MHz): δ 150.9, 129.1, 125.0, 121.7, 51.2, 41.7, 36.3, 29.4.
ESI+MS: calcd for C₁₇H₂₁NO₂: 271.16; found: 272.1 (MH⁺)

### Préparation de Adamantan-2-yl-carbamic acid phenyl ester (composé 126)

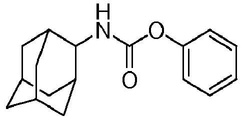

à partir de chlorhydrate de 2-adamantylamine et de phenyl chloroformate, on obtient un solide blanc (17%).
¹H NMR (CDCl₃, 400MHz): δ 7.36 (t, 2H, *J* = 7.6 Hz), 7.20 (t, 1 H, *J=* 7.6 Hz), 7.14 (d, *J* = 8 Hz), 5.36 (bs, 1 H), 3.88 (s, 1 H), 2.03 (s, 2H), 1.87-1.67 (m, 12H).
¹³C NMR (CDCl₃, 100MHz): δ 129.2, 125.1, 121.5, 114.9, 55.1, 37.4, 37.0, 32.0, 31.7, 27.1, 27.0.
ESI+MS: calcd for C₁₇H₂₁NO₂: 271.16; found: 272.1 (MH⁺)

### Préparation de N-Adamantan-1-yl-N-(3-bromo-benzyl)-benzamide (composé 125)

À une suspension agitée de 1-adamantylamine (0.156 mmol; 50 mg; 1 équiv.) dans le CH₂Cl₂ (3 mL), est ajouté à température ambiante le chlorure de benzoyle (0.47 mmol; 55 µL; 3 équiv.) et la triéthylamine (109 µL; 5 équiv.). Le mélange est agité 24 h à température ambiante, lavé à l'eau, mis en solution saturée de NH₄Cl, séché sur Na₂SO₄, filtré, évaporé et purifié sur short pad. 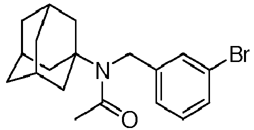 à partir de chlorure d'acétyle, on obtient une huile incolore (26%).
¹H NMR (CDCl₃, 400MHz): δ 7.39 (dd, 1 H, *J* = 1.2 and 10.8 Hz), 7.25 (t, 1 H, *J* = 7.6 Hz), 7.17 (d, 1 H, *J* = 7.6 Hz), 4.58 (s, 2H), 2.20 (s, 6H), 2.07 (s, 6H), 1.63 (m, 6H).
¹³C NMR (CDCl₃, 100MHz): δ 172.2, 142.2, 130.3, 130.1, 128.7, 124.2, 123.0, 59.3, 48.0, 39.9, 36.3, 30.1, 25.7.
ESI+MS: calcd for C₁₉H₂₄BrNO: 361.10; found: 362.0 (MH⁺)

### Préparation de 1-Adamantan-1-yl-1-(3-bromo-benzyl)-3-phenyl-urea (composé 131)

À une suspension agitée de 1-adamantylamine (0.156 mmol; 50 mg; 1 équiv.) dans le THF (3 mL), est ajouté à 0°C du phenylisocyanate (19 µL; 1.1 équiv.). Le mélange est agité 24 h à température ambiante, évaporé et purifié sur short pad.

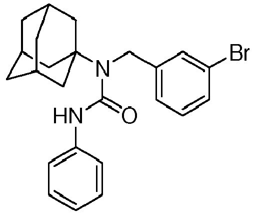

A partir de phenylisocyanate, on obtient une huile incolore (51%)
¹H NMR (CDCl₃, 400MHz): δ 7.52-6.98 (m, 9H), 6.13 (bs, 1 H), 4.64 (s, 2H), 2.25 (s, 6H), 2.11 (s, 3H), 1.68 (m, 6H).
¹³C NMR (CDCl₃, 100MHz): δ 156.5, 141.1, 138.9, 130.8, 130.6, 128.9, 128.7 124.2, 123.4, 122.9, 120.0, 58.2, 47.2, 40.4, 36.3, 30.1.
ESI+MS: calcd for C₂₄H₂₇BrN₂O: 438.13; found: 439.0 (MH⁺)

### Préparation de N-Adamantan-2-yl-N-(3-bromo-benzyl)-benzamide (composé 127)

À une suspension agitée de 2-adamantylamine (0.156 mmol; 50 mg; 1 équiv.) dans le CH₂Cl₂ (2 mL) est ajouté à température ambiante du chlorure de benzoyle (0.47 mmol; 55 µL; 3 équiv.) et de la triéthylamine (109 µL; 5 équiv.). Le mélange est agité 24 h à température ambiante, lavé à l'eau, mis en solution saturée de NH₄Cl, séché sur Na₂SO₄, filtré, évaporé et purifié sur short pad.

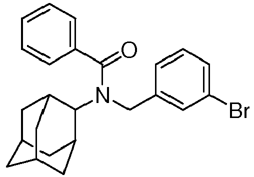

A partir du chlorure de benzoyle, on obtient une huile blanche (17%)
¹H NMR (CDCl₃, 400MHz): δ 7.43 (m, 2H), 7.35 (m, 4H), 7.19 (s, 1H), 7.16 (t, 1H, *J* = 8 Hz), 7.05 (d, 1 H, *J=* 7.6 Hz), 4.78 (s, 2H), 4.17 (s, 1 H), 2.32 (s, 2H), 2.02-1.66 (m, 12H).
¹³C NMR (CDCl₃, 100MHz): δ 175.4, 142.1, 137.8, 130.2, 130.0, 129.7, 128.8, 128.4, 127.1, 124.2, 122.8, 60.2, 49.5, 38.0, 37.4, 32.8, 30.0, 27.3, 26.9.
ESI+MS: calcd for C₂₄H₂₆BrNO: 423.12; found: 424.8 (MH⁺)

### Synthèse par "Click chemistry"

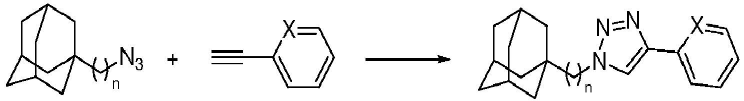

A une suspension de Cu/C (50 mg) dans le 1,4-dioxane (1.5 mL) est ajouté l'Et₃N (0.83 mmol; 1.1 équiv.), le phenylacetylene (0.83 mmol; 1.1 équiv.) ou l'ethynylpyridine (0.83 mmol; 1.1 équiv.) et l'azoture dérivé de la 1-adamantylamine (0.75 mmol; 1 équiv). Le mélange est agité à 60°C pendant 2 jours, filtré sur short pad, lavé avec AcOEt puis évaporé. Ce procédé permet de préparer les composés suivants :

### Préparation du 1-Adamantan-1-yl-4-phenyl-1H-[1,2,3]triazole (composé 145)

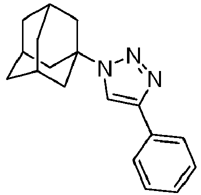

A partir de 1-Azidoadamantane et de phénylacétylène, on obtient une huile brune (96%).
¹H NMR (CDCl₃, 400MHz): δ 7.84 (dd, 3H, *J* = 1.2 and 8.4 Hz), 7.42 (t, 2H, *J* = 8 Hz), 7.32 (t, 1 H, *J* = 7.2 Hz), 2.30 (s, 9H), 1.82 (s, 6H), 1.43 (m, 4H).
¹³C NMR (CDCl₃, 100MHz): δ 146.5, 140.0, 128.6, 127.6, 125.4, 116.0, 59.4, 42.8, 35.7, 29.3.
ESI+MS: calcd for C₁₈H₂₁N₃: 279.17; found: 280.2 (MH⁺)

### Préparation de 1-Adamantan-1-ylmethyl-4-phenyl-1H-[1,2,3]triazole

### (composé 146)

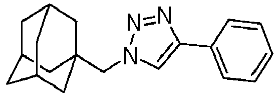

A partir de 1-Azidomethyladamantane et de phénylacétylène, on obtient une huile brune (68%).
¹H NMR (CDCl₃, 400MHz): δ 7.86 (dd, 2H, *J* = 1.2 and 8.4 Hz), 7.68 (s, 1H), 7.44 (t, 2H, *J=* 7.2 Hz), 7.34 (t, 1 H, *J* = 7.6 Hz), 4.08 (s, 2H), 2.02 (s, 3H), 1.58 (m, 12H).
¹³C NMR (CDCl₃, 100MHz): δ 147.0, 130.7, 128.7, 127.9, 125.6, 120.9, 62.2, 40.2, 36.4, 34.1, 28.0.
ESI+MS: calcd for C₁₉H₂₃N₃: 293.19; found: 294.2 (MH⁺)

### Préparation de 2-(1-Adamantan-1-yl-1H-[1,2,3]triazol-4-yl)-pyridine (composé 147)

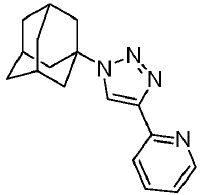

à partir de 1-Azidoadamantane et de 2-éthynylpyridine, on obtient une huile brune (39%)
¹H NMR (CDCl₃, 400MHz): δ 8.58 (d, 1H, *J* = 4.4 Hz), 8.22 (m, 2H), 7.79 (t, 1H, *J* = 7.6 Hz), 7.23 (t, 1 H, *J* = 6 Hz), 2.30 (s, 8H), 1.81 (m, 7H).
¹³C NMR (CDCl₃, 100MHz): δ 150.7, 149.2, 147.3, 136.9, 122.6, 120.1, 118.6, 59.8, 42.9, 35.9, 29.4.
ESI+MS: calcd for C₁₇H₂₀N₄: 280.17; found: 281.2 (MH⁺)

### Imidazolines and oxazoline

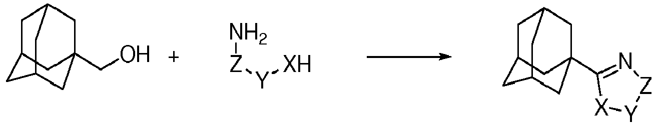

A une solution de 1-adamantanemethanol (166 mg; 1 mmol) dans le *tert*-butanol (8 mL) est ajouté du carbonate de potassium (415 mg; 3 mmol; 3 équiv.) et de l'iodine (635 mg; 2.5 mmol; 2.5 équiv.). Le mélange est agité à 70°C pendant 16 h et l'amine (1.5 équiv) diluée dans le *tert*-butanol est ajoutée. Le mélange est traité à reflux à 70°C pendant 2 h, dilué avec une solution saturée de Na₂SO₃ (5 mL), extrait avec du chloroforme (20 mL). Les phases organiques sont lavées avec NaOH 2N(20 mL), brine (20 mL), séchées sur Na₂SO₄, filtrées et évaporées.

### Préparation de 2-Adamantan-1-yl-4,5-dihydro-1H-imidazole (composé 134)

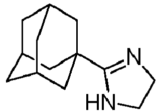

A partir de 1-adamantanemethanol et d'éthylènediamine, on obtient une huile incolore (66%). ¹H NMR (CDCl₃, 400MHz): δ 3.59 (s, 4H), 2.04 (s, 3H), 1.89 (s, 6H), 1.73 (m, 6H).

### Préparation de 2-Adamantan-1-yl-4,5-dihydro-oxazole (composé 135)

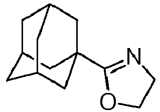

A partir de 1-adamantanemethanol et de 2-aminoéthanol, on obtient une huile incolore (2.4%)
¹H NMR (CDCl₃, 400MHz): δ 4.23 (t, 2H, *J* = 9.6 Hz), 3.83 (t, 2H, *J* = 9.6 Hz), 2.02 (s, 3H), 1.91 (s, 6H), 1.73 (m, 6H).

### Préparation de 2-Adamantan-1-yl-1-phenyl-4,5-dihydro-1H-imidazole (composé 136)

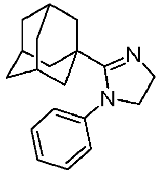

A partir de 1-adamantanemethanol et de *N*-phényléthylènediamine, on obtient une huile jaune (15%)
¹H NMR (CDCl₃, 400MHz): δ 7.40-7.18 (m, 5H), 3.82 (t, 2H, *J* = 9.1 Hz), 3.68 (t, 2H, *J* = 9.6 Hz), 1.85 (s, 3H), 1.81 (s, 6H), 1.54 (m, 6H).
¹³C NMR (CDCl₃, 7100MHz): δ 172.0, 145.0, 129.2, 127.1, 58.2, 52.1, 40.5, 37.5, 36.4, 28.2. ESI+MS: calcd for C₁₉H₂₄N₂: 280.19; found: 281.2 (MH⁺)

### Préparation de 2-Adamantan-1-yl-4,5-dicyclohexyl-4,5-dihydro-1H-imidazole (composé 137)

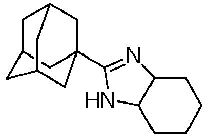

A partir de 1-adamantanemethanol et de (1*R*,2*R*)-1,2-cyclohexanediamine, on obtient une huile jaune (10%)
¹H NMR (CDCl₃, 400MHz): δ 4.67 (bs, 1 H), 2.84 (bs, 2H), 2.03 (s, 3H), 1.87 (s, 6H), 1.72 (m, 6H).
ESI+MS: calcd for C₁₇H₂₆N₂: 258.21; found: 259.2 (MH⁺)

La synthèse des composés amines nécessite la préparation préalable de l'intermédiaire de synthèse suivant :

### - 2-amino-N-phenylbenzamide

La 2-nitro-N-phenylbenzamide (1 éq., 5 mmol, *m attendue* = *1.06 g*) est dissout dans 12 mL de méthanol. Quelques gouttes d'acide acétique sont ajoutées au milieu réactionnel. Le décaborane (0.3 éq., 1.5 mmol, 183 mg) est ensuite additionné, avec le palladium sur charbon (100 mg, 10 % de la masse de la2-nitro-N-phenylbenzamide). Après 3 heures à reflux, le mélange est filtré puis purifié par chromatographie sur silice (cyclohexane / acétate d'éthyle dans des proportions 70 : 30), ce qui donne le composé 2-amino-N-phenylbenzamide avec un rendement de 88 % (933 mg).
**¹H NMR** (400MHz, DMSO d⁶)
*δ :* 9.95 (s, 1 H, N**H**), 7.70-7.68 (d, *J* = *7.6Hz,* 2H, H_{Ar} 2'), 7.62-7.59 (d, *J* = *8Hz,* 1 H, H_{Ar} 3), 7.33-7.29 (t, *J* =*7.6Hz,* 2H, H_{Ar} 3'), 7.20-7.16 (t, *J* = *8.4Hz,* 1 H, H_{Ar} 4), 7.08-7.04 (t, *J* = *7.2Hz,* 1 H, H_{Ar} 4'), 6.75-6.73 (d, *J* =*8.4 Hz,* 1 H, H_{Ar} 6), 6.60-6.56 (t, *J* = *8Hz,* 1 H, H_{Ar} 5), 6.29 (2H, N**H₂**).
**MS (ES)** *m*/*z* 213 (M + H⁺), 120.

### Préparation du 2-(3-bromobenzylideneamino)-N-phenylbenzamide (compose 149)

La 2-amino-N-phenylbenzamide (1 éq., 42 mg, 0.2 mmol) est dissoute dans 2mL de méthanol avec le 3-bromobenzaldehyde (1 éq., 23 µL, 0.2 mmol)). Au bout d'une nuit, le mélange réactionnel est évaporé (on constate la disparition de la 2-amino-N-phenylbenzamide par RMN) et l'imine 149 est formée quantitativement.
**¹H NMR** (400MHz, DMSO d⁶) δ *:* 8.40 (s, 1 H, C**H**), 7.70-6.60 (m, 13H, H_{Ar})

### Préparation du 2-(3-fluorobenzylideneamino)-N-phenylbenzamide (composé 151)

La 2-amino-N-phenylbenzamide (1 éq., 21 mg, 0.1 mmol) est dissoute dans 1 mL de méthanol avec le 3-fluorobenzaldehyde (1 éq., 10 µL, 0.1 mmol)). Au bout d'une nuit, le mélange réactionnel est évaporé (on constate la disparition de la 2-amino-N-phenylbenzamide par RMN) et l'imine 151 est formée quantitativement.
**¹H NMR** (400MHz, DMSO d⁶) *δ* : 9.43 (s, 1 H, CH), 7.62-6.72 (m, 13H, H_{Ar})

### Amination

À une solution de nitroacide (8g; 47.9 mmol; 1 équiv.) dans le CH₂Cl₂ (100mL), sont ajoutés du DCC (10.9g; 52.7 mmol; 1.1 équiv.), du DMAP (1.17g, 9.6 mmol, 0.2 équiv.) et de l'aniline (6.1 mL; 67.1 mmol; 1.4 équiv.) à température ambiante. Le mélange est agité 4 jours et évaporé. Le solide est alors mis en solution dans l'acétone puis filtré.

Le filtrat brun est évaporé pour donner un solide brun qui est lavé avec Et₂O pour donner le 2-nitro-*N*-phenylbenzamide sous forme de solide orange (11.6g; 71%).

Par ailleurs, à une solution de 2-nitro-*N*-phenylbenzamide (5g; 20.7 mmol; 1 équiv.) dans le MeOH (65 mL) est ajouté du palladium sur charcoal (550 mg) et le decaborane (757 mg; 6.2 mmol; 0.3 équiv.) à température ambiante. Le mélange est chauffé à 60°C pendant 6 h, filtré sur célite, lavé avec AcOEt et évaporé pour donner le 2-amino-*N-*phenylbenzamide (solide brun, 4.2g; 96%) utilisé sans purification.

À une solution de 2-amino-*N-*phenylbenzamide (42 mg; 0.2 mmol; 1 équiv.) dans le MeOH (2 mL) est ajouté un aldéhyde (0.2 mmol; 1 équiv.), le mélange est agité 2 jours à température ambiante, évaporé et purifié par une méthode appropriée si nécessaire.

Ce procédé permet de préparer les composés suivants :

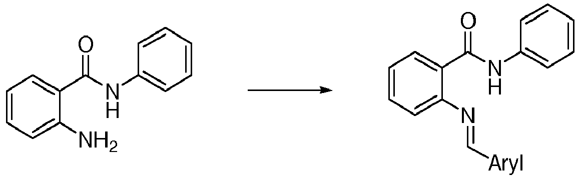

### Préparation de (E)-2-((furan-2-yl)methyleneamino)-N-phenylbenzamide (composé 152)

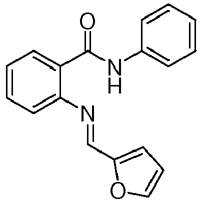

à partir de furan-2-carbaldehyde, on obtient un solide jaune (99%)
¹H NMR (CDCl₃, 400MHz): δ 8.02 (dd, 1 H, *J* = 1.2 and 8 Hz), 7.40-7.24 (m, 7H), 6.91 (t, 1 H, *J* = 8 Hz), 6.70 (d, 1 H, *J* = 8 Hz), 6.33 (d, 1 H, *J =* 3.2 Hz), 6.25 (dd, 1 H, *J* = 2 and 3.2 Hz), 6.06 (d, 1 H, *J* = 2 Hz), 4.94 (bs, 1 H).
¹³C NMR (CDCl₃, 100MHz): δ 162.6, 152.2, 145.1, 142.6, 140.6, 133.7, 128.9, 128.8, 126.7, 126.1, 119.7, 117.1, 115.1, 110.3, 109.0, 68.4.
ESI+MS: calcd for C₁₈H₁₄N₂O₂: 290.11; found: 291.1 (MH⁺)

### Préparation de (E)-2-((5-methylfuran-2-yl)methyleneamino)-N-phenylbenzamide (composé 154)

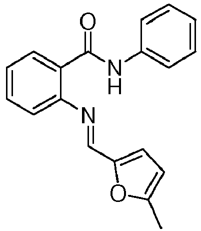

à partir de 5-methylfuran-2-carbaldehyde, on obtient un solide jaune (100%) ESI+MS: calcd for C₁₉H₁₆N₂O₂: 304.12; found: 305.0 (MH⁺)

### Préparation de (E)-2-((furan-3-yl)methyleneamino)-N-phenylbenzamide (compose 153)

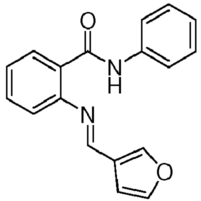

à partir de furan-3-carbaldehyde, on obtient un solide jaune (97%)
¹H NMR (CDCl₃, 400MHz): δ 8.02 (d, 1 H, *J* = 7.6 Hz), 7.39-7.23 (m, 7H), 6.93 (t, 1H, *J* = 7.6 Hz), 6.70 (d, 1 H, *J* = 8 Hz), 6.28 (s, 1 H), 6.04 (s, 1 H), 4.70 (bs, 1 H).
¹³C NMR (CDCl₃, 100MHz): δ 162.7, 145.5, 143.6, 140.8, 140.4, 133.8, 129.0, 128.9, 126.9, 126.7, 125.5, 119.7, 117.1, 115.2, 108.7, 67.7.
ESI+MS: calcd for C₁₈H₁₄N₂O₂: 290.11; found: 291.1 (MH⁺)

### Préparation de (E)-2-(2-fluorobenzylideneamino)-N-phenylbenzamide (composé 157)

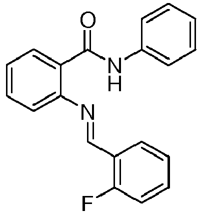

à partir de 2-fluorobenzaldehyde, on obtient un solide jaune (53%) ESI+MS: calcd for C₂₀H₁₅FN₂O: 318.12; found: 319.1 (MH⁺)

### Préparation de (E)-2-(3-bromobenzylideneamino)-N-phenylbenzamide (composé 151)

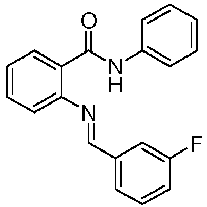

à partir de 3-fluorobenzaldehyde, on obtient un rendement de 100%.
ESI+MS: calcd for C₂₀H₁₅FN₂O: 318.12; found: 319.1 (MH⁺)

### Préparation de (E)-2-(3-fluorobenzylideneamino)-N-phenylbenzamide (composé 149)

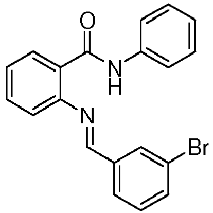

à partir de 3-bromobenzaldehyde, , on obtient un rendement de 100%.
¹H NMR (DMSO-d⁶, 400MHz): δ 8.40 (s, 1H), 7.70-6.60 (m, 13H, H).

### Préparation du (E)-2-(4-fluorobenzylideneamino)-N-phenylbenzamide (omposé 156)

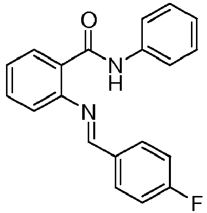

à partir de 4-fluorobenzaldehyde, on obtient une huile jaune (8%).
Mode de purification : Short pad
¹H NMR (DMSO d⁶, 400MHz): δ 9.43 (s, 1 H), 7.62-6.72 (m, 13H)
ESI+MS: calcd for C₂₀H₁₅BrN₂O: 318.12; found: 319.2 (MH⁺)

### Préparation de (E)-2-(5-fluoro-2-nitrobenzylideneamino)-N-phenylbenzamide (composé 155)

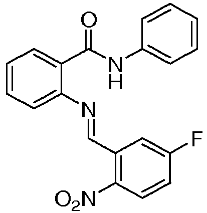

à partir de 5-fluoro-2-nitrobenzaldehyde, on obtient une huile jaune (2.9%) Mode de purification : Short pad
ESI+MS: calcd for C₂₀H₁₄FN₃O₃: 363.34; found: 364.1 (MH⁺)

### Préparation de (E)-2-(5-bromo-2-hydroxybenzylideneamino)-N-phenylbenzamide (composé 159)

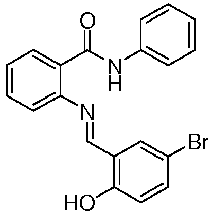

à partir de 5-bromo-2-hydroxybenzaldehyde, on obtient une huile jaune (92%).
ESI+MS: calcd for C₂₀H₁₅BrN₂O₂: 394.03; found: 395.0 (MH⁺)

### Préparation de (E)-2-((1-methyl-1H-indol-2-yl)methyleneamino)-N-phenylbenzamide (composé 158)

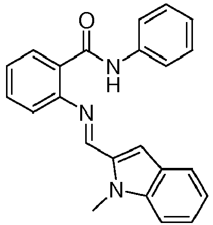

à partir de 1-methyl-1*H-*indole-2-carbaldehyde, on obtient une huile jaune (12%).
Mode de purification : Short pad
ESI+MS: calcd for C₂₃H₁₉N₃O: 353.15; found: 354.2 (MH⁺)

### Amination réductive

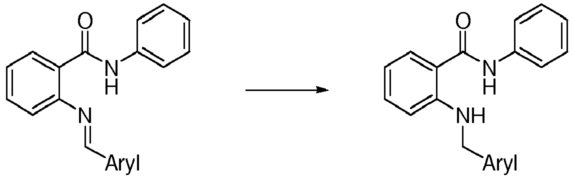

### Préparation du 2-(3-bromobenzylamino)-N-phenylbenzamide (composé 150)

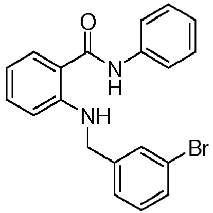

à partir du (*E*)-2-(3-fluorobenzylideneamino)-*N*-phenylbenzamide.
Rendement = 100%

### Préparation du 2-(2-fluorobenzylamino)-N-phenylbenzamide (composé 160)

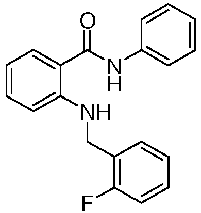

à partir du (*E*)-2-(2-fluorobenzylideneamino)-*N*-phenylbenzamide, on obtient une huile incolore (1 %)
Mode de purification: Short pad
ESI+MS: calcd for C₂₀H₁₇FN₂O: 320.13; found: 321.0 (MH⁺)

### BENZODIAZEPINES

La synthèse des composés de formule générale (II) nécessite au préalable la préparation des **intermédiaires de synthèse** suivants :

### - tert-Butyl N-[(phenylcarbamoyl)methyl]carbamate

La glycine *N*-bocquée (1 éq, 5,71 mmol, 1,0 g) est dissoute dans 10 mL de dichlorométhane à 0°C. Le DCC (1,1 éq, 6,28 mmol, 1,3 g) est ensuite ajouté par petites portions. Enfin, l'aniline (1,4 éq, 8,0 mmol, 0,73 mL) est additionnée à température ambiante. Après deux jours à température ambiante, le contenu du ballon est filtré, puis évaporé. La purification par colonne sur gel de silice (mélange d'acétate d'éthyle / *N*-hexane dans un gradient 50 : 50 à 80 : 20) permet d'obtenir 740 mg de *tert*-Butyl *N-*[(phenylcarbamoyl)methyl]carbamate purifié (51 %).
**¹H NMR** (400MHz, CDCl₃)
*δ :* 9.89 (s, 1 H, N**H**), 7.57-7.55 (d, *J* = *7,6Hz,* 2H, H_{Ar}), 7.30-7.26 (t, *J*=*7,6Hz,* 2H, H_{Ar}), 7.02-7.00 (d, *J*=*7,2Hz*, 1 H, H_{Ar}), 5.19 (s, 1H, N**H**), 3.70-3.69 (d, *J*=*6Hz*, 2H, C**H**₂), 1.38 (s, 9H, (C**H**₃)₃).
**MS (ES)** *m*/*z* 251 (M + H⁺), 195, 151, 94.

### - tert-Butyl (4-methoxyphenylcarbamoyl)methylcarbamate

La glycine *N*-bocquée (1 éq, 7,2 mmol, 1,26 g) est dissoute dans 10 mL de dichlorométhane à 0° C. Le DCC (1,1 éq, 8,0 mmol, 1,65 g) est ensuite ajouté par petites portions. Enfin, la para-anisidine (1,4 éq, 10 mmol, 1,23 g) est additionnée à température ambiante. Après deux jours à température ambiante, le contenu du ballon est filtré, puis évaporé. La purification par colonne de gel de silice (mélange d'acétone / dichlorométhane 98 : 2) permet d'obtenir 1,154 g de composé *tert*-Butyl (4-methoxyphenylcarbamoyl)methylcarbamate purifié (57 %).
**¹H NMR** (400MHz, CDCl₃)
*δ :* 9.74 (s, 1 H, N**H**), 7.48-7.45 (d, *J* = *9,2Hz,* 2H, H_{Ar}), 7.00 (s, 1 H, N**H**), 6.87-6.84 (d, *J*=*8,8Hz,* 2H, H_{Ar}), 3.70 (s, 3H, OCH₃), 3.67-3.66 (d, *J*=*6Hz,* 2H, C**H₂**), 1.38 (s, 9H, (C**H₃**)₃). **MS (ES)** *m*/*z* 281 (M + H⁺), 225, 181, 124.

### - 2-amino-N-phenylacetamide

Le composé *tert*-Butyl *N*-[(phenylcarbamoyl)methyl]carbamate (1 éq, 3 mmol, 450 mg) est dissout dans un mélange 2 : 1 dichlorométhane / acide trifluoroacétique. Après une heure, le produit 2-amino-*N*-phenylacetamide est obtenu après évaporation de manière quantitative et engagé directement dans les essais de réaction de Pictet-Spengler.
**MS (ES)** *m*/*z* 151 (M + H⁺), 94.

### - 2-amino-N-(4-methoxyphenyl)acetamide

Le composé *tert*-Butyl (4-methoxyphenylcarbamoyl)methylcarbamate (1 éq, 3 mmol, 540 mg) est dissout dans un mélange 2: 1 dichlorométhane / acide trifluoroacétique. Après une heure, la 2-amino-*N*-(4-methoxyphenyl)acetamide est obtenue après évaporation de manière quantitative et engagée directement dans les essais de réaction de Pictet-Spengler.
**MS (ES)** *m*/*z* 181 (M + H⁺), 124.

### - 2-benzoyl-4-bromoaniline

Voie 1 : La 4-bromoaniline (1 éq, 0.05 mol, 8.6 g) est dissoute dans le chlorure de benzoyle (2,7 éq, 0,135 mol, 15,7 mL). Le mélange réactionnel est chauffé à 180° C, puis le chlorure de zinc (1,25 éq, 0,063 mol, 8,5 g) est ajouté. Après deux heures de chauffage à 205° C, le mélange est refroidi à 120°C, puis 60 mL d'acide chlorhydrique 3N sont ajoutés. Après chauffage à reflux et décantation de la couche acide chaude, le résidu insoluble dans l'eau est dissout dans 80 mL d'acide sulfurique à 70 %, porté à reflux pendant 8 heures, puis versé dans une grande quantité d'eau glacée. Après ajout d'acétate d'éthyle (3×50 mL), les phases organiques sont rassemblées, puis évaporées. Après purification par HPLC, le rendement est inférieur à 1 % (la masse obtenue était d'environ 200 mg).

Voie 2 : La 2-aminobenzophénone (1 éq, 5 mmol, 0,986 g) est dissoute dans du dichlorométhane à 0°C. Puis le *N*-bromosuccinimide (1 éq, 5 mmol, 0,890 g) est additionné par petites portions. On laisse remonter la température du mélange réactionnel jusqu'à température ambiante sur deux heures environ. Le mélange réactionnel est ensuite évaporé et le 2-benzoyl-4-bromoaniline est obtenu de manière quantitative.
**¹H NMR** (400MHz, CDCl₃)
*δ :* 7.64-7.63 (d, *J* = *7,2 Hz,* 2H, **2'**), 7.56 (s, 1 H, **6**), 7.55-7.54 (d, *J*=*2.4 Hz,* 1 H, **4'**), 7.50-7.47 (t, *J*=*7.6 Hz,* 2H, **3'**), 7.38-7.35 (q, *J*=*8.8 - 2.4 Hz,* 1 H, **4**), 6.66-6.64 (d, *J*=*8.4 Hz,* 1 H, **3**), 6.09 (s, 2H, N**H₂**).
**MS (ES)** *m*/*z* 276 (M + H⁺), 198, 105.

### Préparation du 4,5-dihydro-7-methoxy-5-phenyl-1H-benzo[e][1,4]diazepin-2(3H)-one (composé 167)

La 2-amino-*N*-(4-methoxyphenyl)acetamide (1 éq, 0,5 mmol, 140 mg) est dissoute dans 2 mL d'acétonitrile. Le benzaldéhyde (1,5 éq, 0,75 mmol, 76 µL) est ajouté au milieu réactionnel, puis 1 mL d'acide trifluoroacétique. Après 3 heures à reflux, le mélange est évaporé puis purifié par chromatographie sur gel de silice (mélange cyclohexane/acétate d'éthyle dans un gradient de 80 : 20 à 50 : 50), ce qui permet d'obtenir 16 mg de composé **167** soit un rendement de 12 %.
**¹H NMR** (400MHz, CDCl₃)
*δ :* 7.50-6.60 (m, 8H, H_{Ar}), 6.44 (s, 1 H, N**H**), 5.30 (s, 1 H, CH), 3.82-3.72 (dd, *J*= *10 et 15.6 Hz*, 2H, C**H₂**), 3.73 (s, 3H, OC**H₃**), 3.49 (s, 1 H, N**H**).
**MS (ES)** *m*/*z* 269(M + H⁺), 222, 204, 145, 106.

### Préparation du 5-phenyl-2,3-dihydro-1H-1,4-benzodiazepin-2-one (composé 162)

Dans un ballon équipé d'un Dean-Stark et d'un condenseur, l'aminobenzophénone (2,85 éq, 5 mmol, 1,0 g) est dissoute dans 15 mL de pyridine contenant du tamis moléculaire 4Å. Le chlorhydrate de glycinate d'éthyle (1 éq, 1,75 mmol, 244 mg) est ensuite ajouté, puis le mélange est porté à reflux pendant 3 heures. Environ 5 mL de pyridine sont retirés du Dean-Stark et remplacés par 5 mL de pyridine fraîche. Après une nuit à 110° C, la pyridine est évaporée, en ajoutant 10 mL de toluène, puis le résidu est dissout dans 30 mL de dichlorométhane et 30 mL de Na₂CO₃ à 2.5 %. Les phases organiques regroupées sont lavées avec une solution saturée en chlorure de sodium puis séchées avec Na₂SO₄ anhydre et enfin évaporées pour donner le produit brut. La purification sur colonne de gel de silice (mélange dichlorométhane / acétone dans un gradient de concentration de 95 : 5 à 80 : 20) permet d'obtenir 217 mg de produit **161** purifié soit un rendement de 52,5 %.
**¹H NMR** (400MHz, CDCl₃)
δ *:* 8.45 (s, 1 H, NH), 7.64-7.26 (m, 9H, H_{Ar}), 4.34 (s, 2H, C**H₂**).
**MS (ES)** *m*/*z* 237 (M + H⁺).

### Préparation du : 7-chloro-5-phenyl-2,3-dihydro-1H-1,4-benzodiazepin-2-one (composé 170)

Dans un ballon équipé d'un Dean-Stark et d'un condenseur, la 4-chloro-2-aminobenzophénone (2,85 éq, 5 mmol, 1,16 g) est dissoute dans 15 mL de pyridine contenant du tamis moléculaire 4À. Le chlorhydrate de glycinate de méthyle (1 éq, 1,75 mmol, 220 mg) est ensuite ajouté, puis le mélange est porté à reflux pendant 3 heures. Après une nuit à 110° C, la pyridine est évaporée, en ajoutant 10 mL de toluène, puis le résidu est dissout dans 30 mL de dichlorométhane et 30 mL de Na₂CO₃ à 2.5 %. Les phases organiques regroupées sont lavées avec une solution saturée en chlorure de sodium puis séchées avec Na₂SO₄ anhydre et enfin évaporées pour donner le produit brut. La purification sur colonne de gel de silice (mélange cyclohexane / acétate d'éthyle dans un gradient de 90 : 10 à 50 : 50) permet d'obtenir 80 mg de produit **170** (17 %).
**¹H NMR** (400MHz, CDCl₃)
*δ :* 9.42 (s, 1 H, NH), 7.78-7.16 (m, 8H, H_{Ar}), 4.33 (s, 2H, C**H₂**).
**MS (ES)** *m*/*z* 271 (M + H⁺).

### Préparation du 7-bromo-5-phenyl-2,3-dihydro-1H-1,4-benzodiazepin-2-one (composé 163)

Dans un ballon équipé d'un Dean-Stark et d'un condenseur, la 2-benzoyl-4-bromoaniline (2 éq, 0,47 mmol, 130 mg) est dissoute dans 5 mL de pyridine. Le chlorhydrate de glycinate de méthyle (1 éq, 1,24 mmol, 30 mg) est ensuite ajouté, puis le mélange est porté à reflux pendant 3 heures. Environ 2 mL de pyridine sont retirés du Dean-Stark et remplacés par 2 mL de pyridine fraîche. Après une nuit à 110° C, la pyridine est évaporée, en ajoutant 5 mL de toluène, puis le résidu est dissout dans 20mL de dichlorométhane et 20 mL de Na₂CO₃ à 2.5 %. Les phases organiques regroupées sont lavées avec une solution saturée en chlorure de sodium puis séchées sur du Na₂SO₄ anhydre et enfin évaporées pour donner le produit brut. La purification sur colonne de gel de silice (mélange cyclohexane / acétate d'éthyle dans un gradient de concentration de 90 : 10 à 50 : 50) permet d'obtenir 100 mg de produit **163** soit 26 %.
**¹H NMR** (400MHz, CDCl₃)
*δ :* 9.26 (s, 1 H, N**H**), 7.90-7.32 (m, 8H, H_{Ar}), 4.37 (s, 2H, C**H₂**).
**MS (ES)** *m*/*z* 315 (M + H⁺).

### Préparation du 5-phenyl-2,3,4,5-tetrahydro-1H-1,4-benzodiazepin-2-one (composé 169)

Le composé **162** (1 éq, 0,42 mmol, 100 mg) est dissout dans 2 mL de méthanol. Le réducteur sur billes de polymères (3 éq, 1,27 mmol, loading : 4,4 mmol/g, 290 mg) est ajouté, ainsi que quelques gouttes d'acide acétique (50 µL). La réaction se déroule à température ambiante pendant 3 jours. Après filtration puis évaporation, le produit obtenu est purifié par chromatographie sur gel de silice (cyclohexane / acétate d'éthyle dans un gradient 80 : 20 à 1 : 2), et le produit désiré **169** est obtenu avec un rendement de 60 %.
**¹H NMR** (400MHz, CDCl₃)
*δ :* 9.12 (s, 1 H, N**H**), 7.35-6.67 (m, 9H, H_{Ar}), 5.21 (s, 1H, C**H**), 3.35-3.25 (q, *J*=*10 - 14.8Hz,* 2H, C**H₂**), 3.21 (s, 1 H, N**H**).
**MS (ES)** *m*/*z* 239 (M + H⁺), 182, 132, 91.

### Préparation du 7-chloro-5-phenyl-2,3,4,5-tetrahydro-1H-1,4-benzodiazepin-2-one (composé 171)

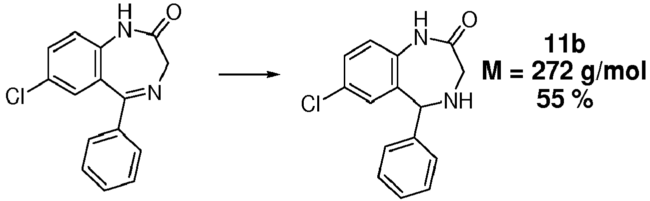

Le composé **170** (1 éq, 0,22 mmol, 60 mg) est dissout dans 2 mL de méthanol. Le réducteur sur billes de polymères (3 éq, 0,66 mmol, loading : 4,4 mmol/g, 150 mg) est ajouté, ainsi que quelques gouttes d'acide acétique (50 µL). La réaction se déroule à température ambiante pendant 5 jours. Après filtration puis évaporation, le produit obtenu est purifié par chromatographie sur gel de silice (cyclohexane / acétate d'éthyle dans un gradient 80 : 20 à 1 : 2), et le produit désiré **171** est obtenu avec un rendement de 55 %.
**¹H NMR** (400MHz, CDCl₃)
*δ :* 9.15 (s, 1 H, **NH**), 7.40-6.86 (m, 8H, H_{Ar}), 5.20 (s, 1 H, CH), 3.45-3.36 (q, *J*=*10 - 13.6Hz,* 2H, C**H₂**), 3.10 (s, 1 H, N**H**).
**MS (ES)** *m*/*z* 273 (M + H⁺), 221, 91.

### Préparation du 7-bromo-5-phenyl-2,3,4,5-tetrahydro-1H-1,4-benzodiazepin-2-one (compose 164)

Le composé **163** (1 éq, 0,25 mmol, 80 mg) est dissout dans 2 mL de méthanol. Le réducteur sur billes de polymères (3 éq, 0,75 mmol, loading : 4,4 mmol/g, 170 mg) est ajouté, ainsi que quelques gouttes d'acide acétique (50 µL). La réaction se déroule à température ambiante pendant 3 jours. Après filtration puis évaporation, le produit obtenu est purifié par chromatographie sur gel de silice (cyclohexane / acétate d'éthyle dans un gradient 80 : 20 à 1 : 2), et le produit désiré **164** est obtenu avec un rendement de 52 %.
**¹H NMR** (400MHz, CDCl₃)
*δ :* 8.48 (s, 1 H, N**H**), 7.52-6.86 (m, 8H, H_{Ar}), 5.18 (s, 1H, C**H**), 3.45-3.35 (q, *J*=*10 - 14.8Hz,* 2H, C**H₂**), 2.86 (s, 1 H, N**H**).
**MS (ES)** *m*/*z* 317 (M + H⁺), 91.

### Préparation du 4-phenyl-2,3-dihydro-1H-1,5-benzodiazepin-2-one (composé 165)

Dans un pilulier, le 1,2-diamino-benzène (1 éq, 2 mmol, 216 mg) et le 3-oxo-3-phenylpropanoate d'éthyle (1 éq, 2 mmol, 384 mg) sont mélangés. Le mélange réactionnel est alors chauffé à 150° C pendant deux heures. Le résidu est ensuite dilué dans de l'acétate d'éthyle et de l'acide chlorhydrique (pH∼5), puis la phase organique est lavée à l'eau, puis avec une solution saturée de chlorure de sodium. Enfin, la solution est séchée avec Na₂SO₄ puis évaporée. Une purification sur colonne de gel de silice (cyclohexane / acétate d'éthyle 70 : 30) permet d'obtenir 325 mg de **165**, soit un rendement de 69 %.
**¹H NMR** (400MHz, CDCl₃)
*δ :* 12.88 (s, 1 H, NH), 8.17-6.64 (m, 9H, H_{Ar}), 5.50 (s, 2H, C**H₂**).
**MS (ES)** *m*/*z* 237 (M + H⁺), 195.

### Préparation du 4-phenyl-2,3,4,5-tetrahydro-1H-1,5-benzodiazepin-2-one (composé 166)

Le composé **165** (1 éq, 0,42 mmol, 100 mg) est dissout dans 2 mL de méthanol. Le réducteur sur billes de polymères (3 éq, 1,27 mmol, loading : 4,4 mmol/g, 290 mg) est ajouté, ainsi que quelques gouttes d'acide acétique (50 µL). La réaction se déroule à température ambiante pendant 3 jours. Après filtration puis évaporation, le produit obtenu est purifié par chromatographie sur gel de silice (cyclohexane / acétate d'éthyle dans un gradient 80 : 20 à 1 : 2), et le produit désiré **166** est obtenu avec un rendement de **77 %**.

Le second procédé suivant permet également la synthèse du composé 166 : dans un pilulier, on mélange le 1,2-diamino-benzène (1 éq, 10 mmol, 1,08 g) avec l'acide cinnamique (1 éq, 10 mmol, 1,48 g). Le mélange réactionnel est alors chauffé à 150° C sans solvant pendant deux heures. Le résidu est dilué dans du dichlorométhane et une solution de Na₂CO₃ à 5 %. La phase organique est extraite plusieurs fois avec la solution de Na₂CO₃, puis avec une solution saturée en chlorure de sodium. Enfin, la solution est séchée avec Na₂SO₄ puis évaporée. Une purification sur colonne de gel de silice (cyclohexane / acétate d'éthyle dans un gradient 80 : 20 à 1 : 1) permet d'isoler **166** avec un rendement de 12 % (286 mg).
**¹H NMR** (400MHz, CDCl₃)
*δ :* 9.15 (s, 1 H, NH), 7.40-6.72 (m, 9H, H_{Ar}), 5.76 (s, 1 H, N**H**), 4.91-4.89 (m, 1 H, CH), 2.54-2.52 (d, *J*=*6Hz,* 2H, C**H₂**).
**MS (ES)** *m*/*z* 239 (M + H⁺), 197, 131.

### Préparation de l'Ethyl 4-oxo-2-phenyl-2,3,4,5-tetrahydro-1H-1,5-benzodiazepine-1-carboxylate (composé 168)

Le composé **166** (1 éq, 0,218 mmol, 52 mg) est dissout avec du chloroformate d'éthyle (1,2 éq, 0,262 mmol, 25 µL) dans une solution composée de dichlorométhane et de triéthylamine dans des proportions 10 : 1. Après deux jours à température ambiante, le contenu du pilulier est évaporé. Une purification sur colonne de gel de silice (cyclohexane / acétate d'éthyle dans un gradient 90 : 10 à 1 : 1) permet d'isoler 15 mg de **168** soit un rendement de 21 %.
**¹H NMR** (400MHz, CDCl₃)
*δ :* 9.10 (s, 1H, N**H**), 7.62-6.72 (m, 9H, H_{Ar}), 5.10 (s, 1H, C**H**), 4.24 (m, 2H, C**H₂**) 2.62-2.61 (d, *J*=*6Hz,* 2H, C**H₂**), 1.46 (t, *J*=*8Hz,* 3H, C**H₃**).
**MS (ES)** *m*/*z* 311 (M + H⁺), 269, 207, 135.

### Synthèse des benzo[b][1,4]diazepines

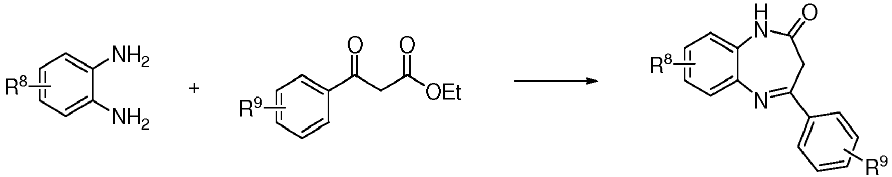

à une suspension agitée de diamine (0.5 mmol; 54 mg; 1 équiv.) dans le toluene (2 mL) est ajouté un β-ketoester (0.5 mmol; 1 équiv.). Le mélange est agité à reflux (120°C) pendant 3 h. Le mélange est dilué dans le EtOAc, acidifié (pH 5), extrait avec EtOAc, filtré, évaporé et lavé avec Et₂O pour donner le composé souhaité.

### À partir de benzene-1,2-diamine :

### Préparation du (E)-4-m-tolyl-1H-benzo[b][1,4]diazepin-2(3H)-one (composé 176)

À partir d'éthyl 3-oxo-3-*m*-tolylpropanoate, on obtient un solide brun (31%)
¹H NMR (CDCl₃, 400MHz): δ 7.94 (s, 1 H), 7.91 (d, 1 H, *J*= 8 Hz), 7.69 (bs, 1 H), 7.54 (d, 1 H, *J* = 8.8 Hz), 7.38 (t, 1 H, *J =* 7.6 Hz), 7.31 (m, 2H), 7.05 (dd, 1 H, *J =* 1.6 and 7.6 Hz), 3.59 (s, 2H), 2.45 (s, 3H).
ESI+MS: calcd for C₁₆H₁₄N₂O: 250.11; found: 250.1 (MH⁺)

### Préparation de (E)-4-(3-methoxyphenyl)-1H-benzo[b][1,4]diazepin-2(3H)-one (composé 177)

À partir d'éthyl 3-(3-methoxyphenyl)-3-oxopropanoate, on obtient un solide brun (20%)
¹H NMR (CDCl₃, 400MHz): δ 7.68 (m, 3H), 7.55 (dd, 1 H, *J* = 2 and 8 Hz), 7.40 (t, 1 H, *J=* 8.4 Hz), 7.28 (m, 1 H), 7.06 (dt, 2H, *J* = 2.8 and 8 Hz), 3.91 (s, 3H), 3.58 (s, 2H).
¹³C NMR (CDCl₃, 100MHz): δ 167.3, 159.9, 158.8, 139.8, 138.9, 129.7, 128.9, 128.3, 126.5, 125.2, 121.6, 120.4, 117.7, 112.2, 55.5, 39.9.
ESI+MS: calcd for C₁₆H₁₄N₂O_{2:} 266.11; found: 267.0 (MH⁺)

### Préparation de (E)-4-(3-nitrophenyl)-1H-benzo[b][1,4]diazepin-2(3H)-one (composé 178)

À partir d'ethyl 3-(3-nitrophenyl)-3-oxopropanoate, on obtient un solide brun (23%)
¹H NMR (CDCl₃, 400MHz): δ 9.00 (t, 1 H, J = 2 Hz), 8.40 (d, 1 H, *J* = 8 Hz), 8.35 (dd, 1 H, *J* = 1.6 and 8 Hz), 7.73 (bs, 1 H), 7.68 (t, 1 H, *J =* 8.4 Hz), 7.55 (m, 1 H), 7.32 (m, 2H), 7.09 (m, 1H), 3.62 (s, 2H).
ESI+MS: calcd for C₁₅H₁₁N₃O₃: 281.08; found: 282.0 (MH⁺)

### Préparation de (E)-4-(3-chlorophenyl)-1H-benzo[b][1,4]diazepin-2(3H)-one (composé 179)

À partir d'éthyl 3-(3-chlorophenyl)-3-oxopropanoate, on obtient un solide brun (15%).
¹H NMR (CDCl₃, 400MHz): δ 8.14 (t, 1H, *J* = 1.6 Hz), 7.96 (d, 1H, *J=* 7.6 Hz), 7.76 (bs, 1H), 7.53-7.41 (m, 3H), 7.29 (m, 2H), 7.07 (dd, 1 H, *J* = 1.2 and 6.8 Hz), 3.56 (s, 2H).
¹³C NMR (CDCl₃, 100MHz): δ 167.1, 157.3, 139.7, 139.3, 135.0, 131.0, 129.9, 128.9, 128.4, 127.8, 126.9, 125.8, 125.3, 121.7, 39.7.

### Préparation de (E)-4-(3-bromophenyl)-1H-benzo[b][1,4]diazepin-2(3H)-one (composé 180)

À partir d'éthyl 3-(3-bromophenyl)-3-oxopropanoate, on obtient un solide brun (7%).
¹H NMR (CDCl₃, 400MHz): δ 8.30 (t, 1 H, *J =* 1.6 Hz), 8.00 (d, 1 H, *J* = 8 Hz), 7.77 (bs, 1 H), 7.63 (dd, 1 H, *J =* 0.8 and 8 Hz), 7.52 (dd, 1 H, J = 2 and 7.6 Hz), 7.36 (t, 1 H, *J* = 8 Hz), 7.27 (m, 1 H), 7.07 (dd, 1 H, *J* = 1.6 and 7.2 Hz), 3.55 (s, 2H).
¹³C NMR (CDCl₃, 100MHz): δ 166.9, 157.3, 139.7, 139.3, 133.9, 130.7, 130.2, 128.8, 128.4, 126.9, 126.3, 125.3, 123.1, 121.7, 39.7.

### Préparation de (E)-4-(3-(trifluoromethyl)phenyl)-1H-benzo[b][1,4]diazepin-2(3H)-one (composé 181)

À partir d'éthyl 3-(3-(trifluoromethyl)phényl)-3-oxopropanoate, on obtient un solide brun (34%).
¹H NMR (CDCl₃, 400MHz): δ 8.42 (s, 1 H), 8.26 (d, 1 H, *J=* 7.6 Hz), 7.78 (bs, 1 H), 7.76 (d, 1 H, *J* = 8.4 Hz), 7.63 (t, 1 H, *J* = 7.6 Hz), 7.54 (dd, 1 H, *J* = 2.4 and 8 Hz), 7.33-7.29 (m, 2H), 7.08 (dd, 1 H, *J* = 2.4 and 7.2 Hz), 3.60 (s, 2H).

### à partir de 4-bromobenzene-1,2-diamine

à une suspension agitée de diamine (0.5 mmol; 94 mg; 1 équiv.) dans le toluène (2 mL) est ajouté un β-ketoester (0.5 mmol; 1 équiv.).

Le mélange est agité à reflux (120°C) pendant 3 h. Le mélange est dilué avec EtOAc, acidifié (pH 5), extrait avec EtOAc, filtré, évaporé et lavé avec Et₂O.

### Préparation du (E)-7-bromo-4-m-tolyl-1H-benzo[b][1,4]diazepin-2(3H)-one (composé 182)

À partir d'éthyl 3-oxo-3-*m*-tolylpropanoate, on obtient un solide brun (28%)
¹H NMR (CDCl₃, 400MHz): δ 7.89 (m, 3H), 7.40-7.32 (m, 4H), 3.58 (s, 2H), 2.44 (s, 3H).
ESI+MS: calcd for C₁₆H₁₃BrN₂O: 328.02; found: 328.9 (MH⁺)

### Préparation de (E)-7-bromo-4-(3-methoxyphenyl)-1H-benzo[b][1,4]diazepin-2(3H)-one (composé 183)

À partir d'éthyl 3-(3-méthoxyphényl)-3-oxopropanoate, on obtient un solide brun (10%) ESI+MS: calcd for C₁₆H₁₃BrN₂O₂: 344.02; found: 344.9 (MH⁺)

### Préparation de (E)-7-bromo-4-(3-nitrophenyl)-1H-benzo[b][1,4]diazepin-2(3H)-one (composé 184)

À partir de 3-(3-nitrophényl)-3-oxopropanoate, on obtient un solide brun (6.5%)
ESI+MS: calcd for C₁₅H₁₀BrN₃O₃: 358.99; found: 359.8 (MH⁺)

### Préparation de (E)-7-bromo-4-(3-chlorophenyl)-1H-benzo[b][1,4]diazepin-2(3H)-one (composé 185)

À partir d'éthyl 3-(3-chlorophényl)-3-oxopropanoate, on obtient un solide brun (23%) ESI+MS: calcd for C₁₅H₁₀BrClN₂O: 347.97; found: 348.8 (MH⁺)

### Préparation de (E)-7-bromo-4-(3-bromophenyl)-1H-benzo[b][1,4]diazepin-2(3H)-one (composé 186)

À partir d'éthyl 3-(3-bromophényl)-3-oxopropanoate, on obtient un solide brun (10%) ESI+MS: calcd for C₁₅H₁₀Br₂N₂O: 391.92; found: 392.8 (MH⁺)

### Préparation de (E)-7-bromo-4-(3-(trifluoromethyl)phenyl)-1H-benzo[b][1,4]diazepin-2(3H)-one (composé 186)

À partir d'éthyl 3-(3-(trifluoromethyl)phenyl)-3-oxopropanoate, on obtient un solide brun (29%)
ESI+MS: calcd for C₁₆H₁₀BrF₃N₂O: 381.99; found: 382.8 (MH⁺)

### Exemple 2 - mise en évidence de l'activité des composés selon l'invention

### 1) PRINCIPE DU CRIBLAGE A HAUT DEBIT :

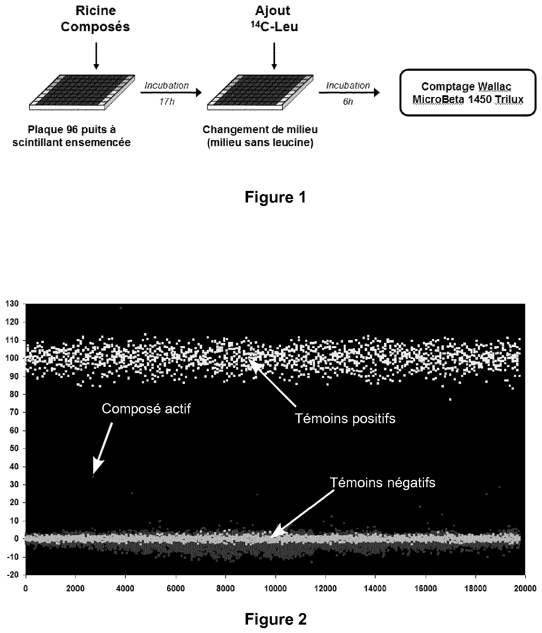

Un test cellulaire a été développé pour mettre en évidence l'activité cytotoxique de la ricine tout en étant adapté aux contraintes du criblage à haut débit (voir à la **Figure 1** la représentation schématique de ce test). L'utilisation d'un tel test présente plusieurs avantages :
(i) sélection de molécules qui peuvent agir sur les différentes étapes de l'intoxication cellulaire (liaison aux récepteurs, internalisation, routage intracellulaire, activité enzymatique...) ;
(ii) sélection de molécules qui pénètrent la cellule ;
(iii) élimination des composés cytotoxiques.

En outre, le test utilisé mesure directement la capacité des cellules à synthétiser des protéines, ce qui en fait un excellent test de criblage car cette voie de biosynthèse est stoppée par la ricine.

Le principe du test cellulaire est le suivant, les composés des chimiothèques sont préincubés avec la ricine et l'ensemble ajouté au milieu de culture des cellules épithéliales humaines de poumon (cellules A549, 60000 cellules/puits, [ricine] = 10⁻¹⁰M) cultivées sur des plaques présentant un fond en scintillant solide (*Scintiplates,* GE). Après incubation, le milieu est éliminé et remplacé par un milieu de culture dépourvu de leucine contenant le traceur radioactif, qui est la [¹⁴C]-leucine (0,05 µCi/puits). La mesure de l'incorporation de la [¹⁴C]-leucine est ensuite réalisée après une seconde incubation. La présence du traceur dans les cellules traduit la présence d'un inhibiteur (C=50 µM) dans le puits (**Figure 1**).

Les puits colorés en jaunes sont des contrôles positifs (A549 traitées avec la ricine (10⁻¹⁰M) en présence de 20 mM de lactose qui est un inhibiteur de liaison de la ricine aux cellules), tandis que les puits verts : contrôles négatifs (cellules traitées avec la ricine seule). Les puits rouges : cellules en contact avec les composés des banques en présence de ricine (80 composés différents par plaque, 50µM final).

Le bilan du criblage réalisé est représenté dans le graphe de la figure 2.

### 2) MISE EN OEUVRE DU CRIBLAGE A HAUT DEBIT :

### 2.1. Activité protectrice contre la toxine de la ricine des composés selon l'invention

Les composés ont été testés sur cellules A549 à la concentration de 30 µM par incubation avec différentes concentrations de ricine (10⁻⁹ à 10⁻¹² M) avec le protocole décrit précédemment pour le criblage à haut débit. La radioactivité mesurée est alors proportionnelle au taux de survie des cellules. L'analyse des données par régression non linéaire permet d'estimer l'EC₅₀, soit la concentration efficace pour laquelle on observe 50 % d'assimilation de leucine radioactive ce qui correspond à 50% de cellules viables. Plus la valeur de l'EC₅₀ est élevée, plus la protection cellulaire est importante car il faut alors une concentration plus élevée de ricine pour générer la même cytotoxicité.

Les résultats, présentés dans le tableau ci-dessous sont indiqués sous la forme d'un indice de protection (IP = ratio EC₅₀ composé / EC₅₀ ricine) : plus il est élevé, plus les cellules sont protégées contre l'action de la ricine (l'effet est protecteur si le ratio est supérieur à 1).

| **N°** | **Structures** | **Bio** | **N°** | **Structures** | **Bio** |
|---|---|---|---|---|---|
| **19** | 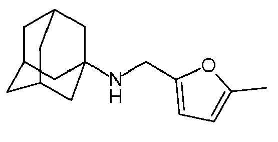 | **1,31** | **122** | 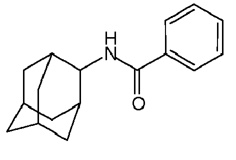 | **1,49** |
| **3** | 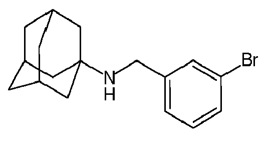 | **2,65** | **128** | 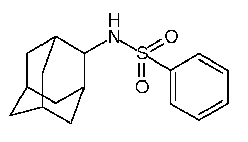 | **1,30** |
| **7** | 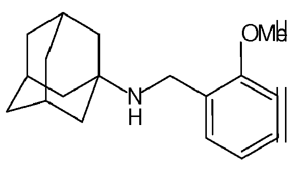 | **1,07** | **64** | 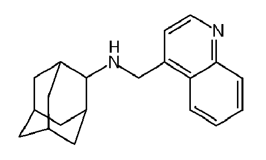 | **1,16** |
| **9** | 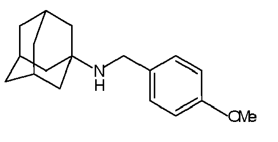 | **1,88** | **65** | 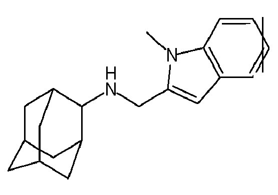 | **1,61** |
| **148** | 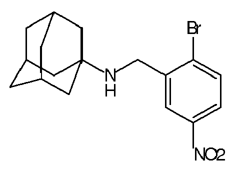 | **2,82** | **94** | 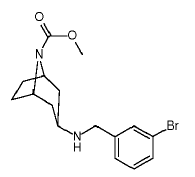 | **1,18** |
| **21** | 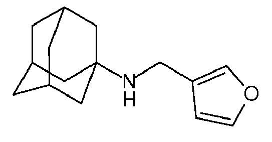 | **1,01** | **89** | 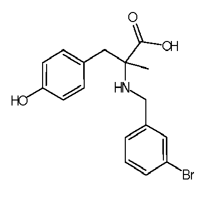 | **1,41** |
| **1** | 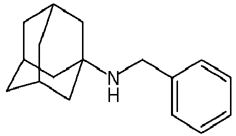 | **1,58** | **109** | 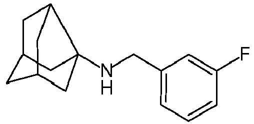 | **1,32** |
| **123** | 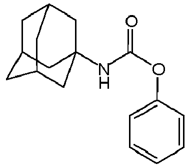 | **1,02** | **78** | 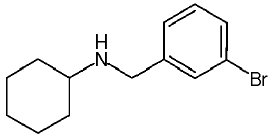 | **1,09** |
| **113** | 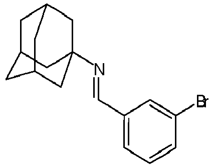 | **1,09** | **79** | 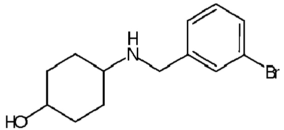 | **1,02** |
| **121** | 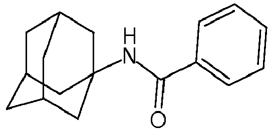 | **1,225** | **80** | 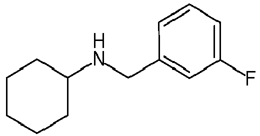 | **1,05** |
| **110** | 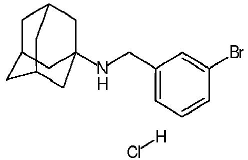 | **3,13** | **81** | 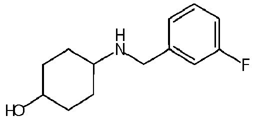 | **1,03** |
| **32** | 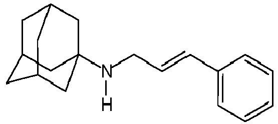 | **1,16** | **75** | 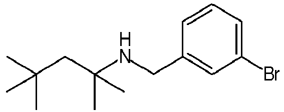 | **1,62** |
| **15** | 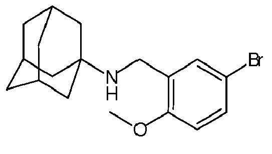 | **4,64** | **86** | 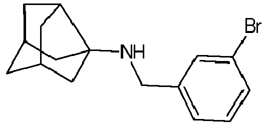 | **2,24** |
| **111** | 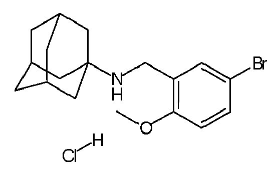 | **3,07** | **77** | 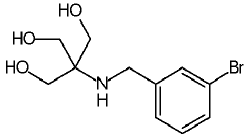 | **1,15** |
| **5** | 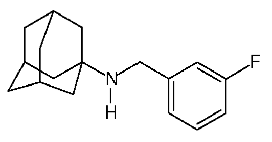 | **2,13** | **84** | 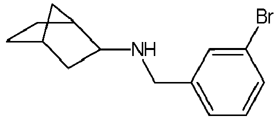 | **1,25** |
| **124** | 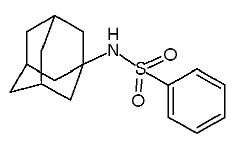 | **1,08** | **83** | 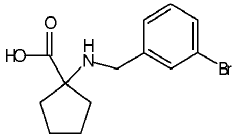 | **1,05** |
| **26** | 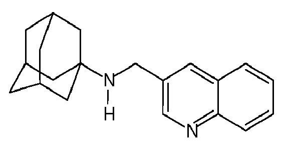 | **1,06** | | 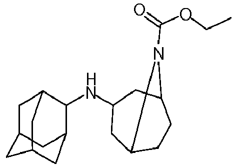 | **1,07** |
| **27** | 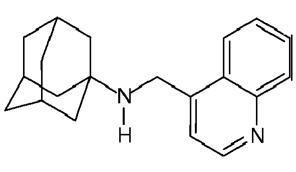 | **1,23** | **120** | 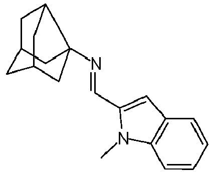 | **1,09** |
| **28** | 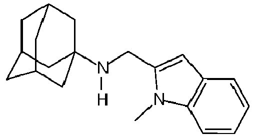 | **2,52** | **102** | 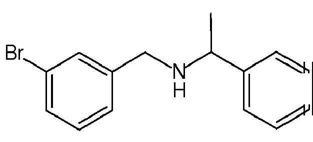 | **1,34** |
| **24** | 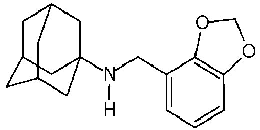 | **1,49** | **99** | 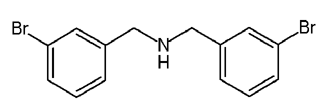 | **1,09** |
| **56** | 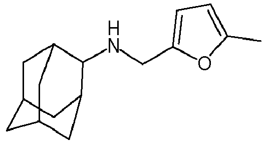 | **1,09** | **105** | 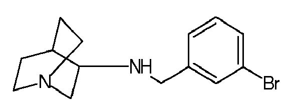 | **1,38** |
| **36** | 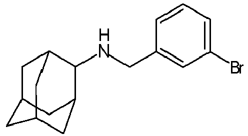 | **2,28/3,57** | **106** | 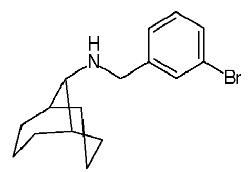 | **3,64** |
| **50** | 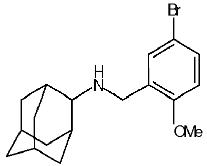 | **1,22** | **108** | 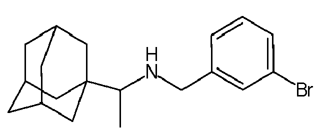 | **1,20** |
| **53** | 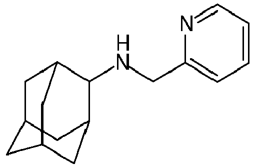 | **1,17** | **107** | 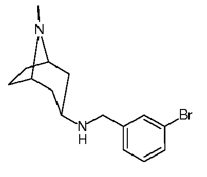 | **2,33** |
| **54** | 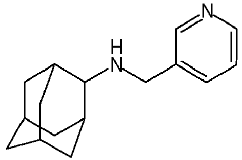 | **1,17** | **165** | 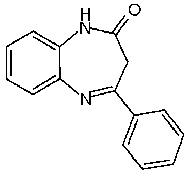 | **1(a)** |
| **55** | 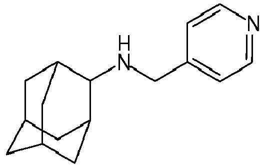 | **1,21** | **166** | 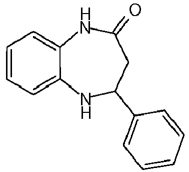 | **1,14 (a)** |
| **59** | 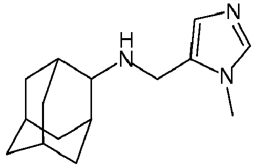 | **1,30** | **166** | 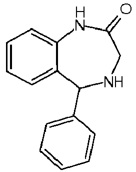 | **1,11 (a)** |
| **132** | 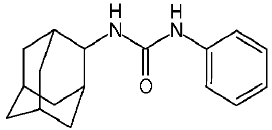 | **1,03** | **179** | 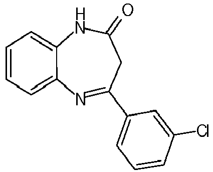 | **1,04** |
| **133** | 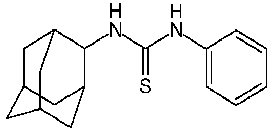 | **1,10** | **149** | 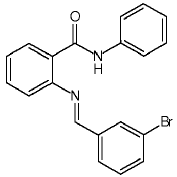 | **1,02** |
| **51** | 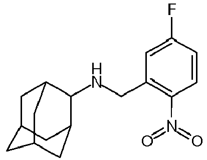 | **1,063** | **151** | 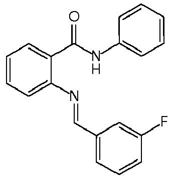 | **1,04** |
| **117** | 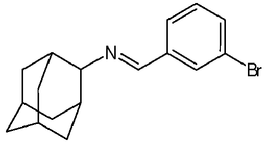 | **2,199** | **154** | 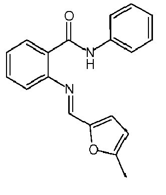 | **1,37** |
| **118** | 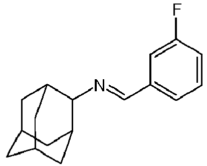 | **1,48** | **157** | 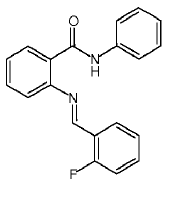 | **1,27** |
| **112** | 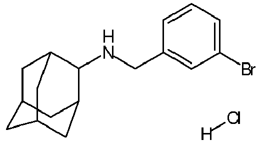 | **3,54** | **138** | 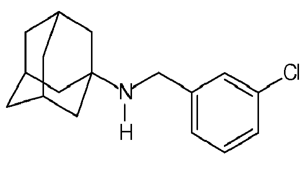 | **1,55** |
| **192** | 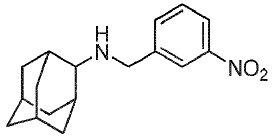 | **1,2** | **131** | 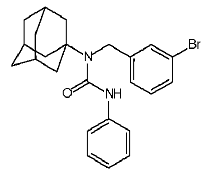 | **1,43** |
| **69** | 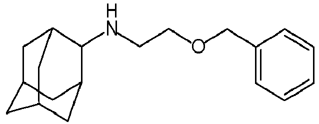 | **1,84** | **139** | 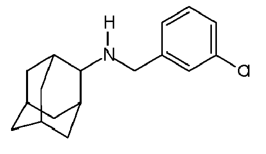 | **2,97** |
| **69** | 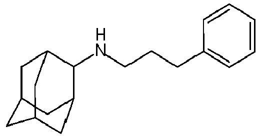 | **1,52** | **140** | 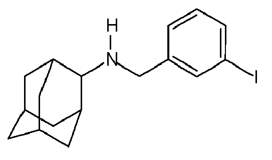 | **2,62** |
| **34** | 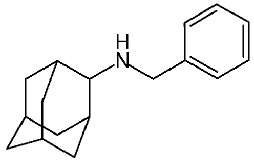 | **1,93** | **141** | 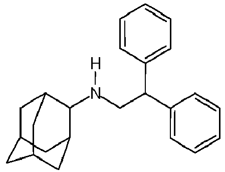 | **1,10** |
| **39** | 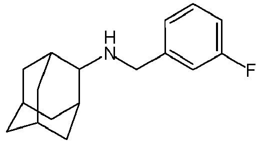 | **3,77** | **142** | 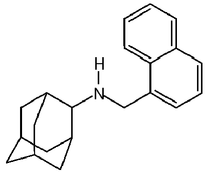 | **1,41** |
| **72** | 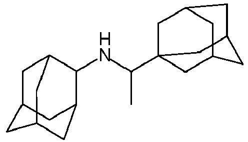 | **1,27** | **143** | 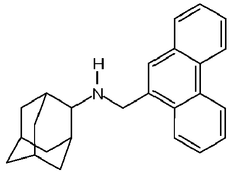 | **1,64** |
| **40** | 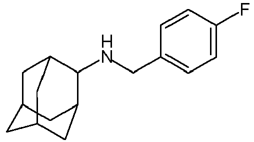 | **1,16** | **161** | 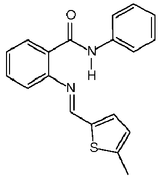 | **2,98** |
| **193** | 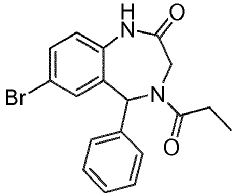 | **4,1** | | | |

On observe bien que ces composés inhibent l'action cytotoxique de la ricine. De plus, ce sont les seuls inhibiteurs connus à ce jour pour protéger les cellules A549 (épithélium pulmonaire humain) contre la ricine.

Ces composés sont les premiers inhibiteurs actifs sur cellules épithéliales humaines pulmonaires et digestives vis-à-vis de l'activité toxique de la ricine.

### 2.2. Activité protectrice contre la toxine diphtérique des composés selon l'invention

Les composés ont également été testés sur cellules Vero (ATCC n°CCL-81) à la concentration de 30 µM par incubation avec différentes concentrations de toxine diphtérique (10⁻⁹ à 10⁻¹²M) (Sigma) avec le protocole décrit au point 2.1.

Les résultats, présentés dans le tableau ci-dessous représentent également un indice de protection (IP = ratio EC₅₀ composé / EC₅₀ ricine) : plus il est élevé, plus les cellules sont protégées contre l'action de la toxine diphtérique.

| Composé | Structure | activité |
|---|---|---|
| 5 | 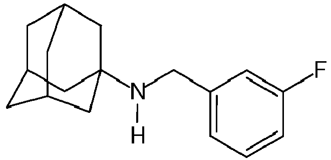 | 2,2 |
| 9 | 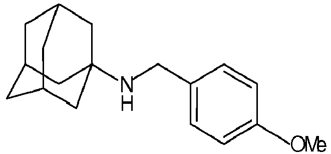 | 1,7 |
| 39 | 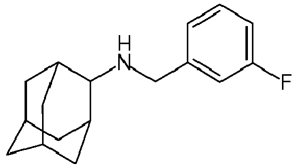 | 1,5 |
| 110 | 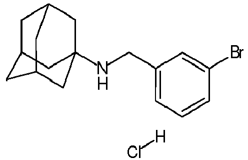 | 2,6 |
| 111 | 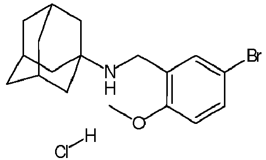 | 1,8 |
| 112 | 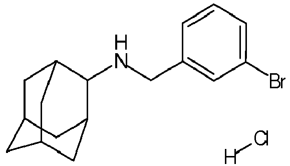 | 3,6 |
| 139 | 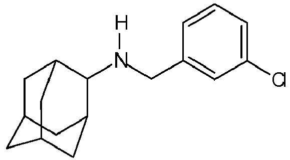 | 1,7 |
| 140 | 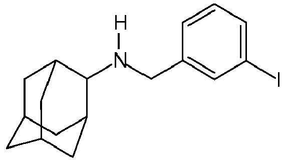 | 3,0 |

## Revendications

1. Utilisation d'un composé de formule générale (I) : 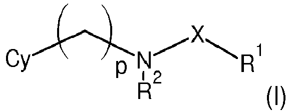 dans laquelle :
**Cy** représente un groupement 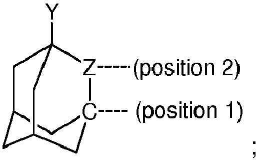 **Y** est choisi parmi un atome d'hydrogène ou une fonction hydroxyle et **Z** est un atome de carbone ou une liaison (Cy est alors un noyau noradamantyle),
étant entendu que quand **Cy** est un noyau adamantyle, la chaîne 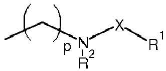 lui est attachée en position 1 ou 2,
**p** représente 0 ou 1 ;
**X** représente soit :
- une liaison ;
- une chaîne alkyle en C₁-C₆, éventuellement insaturée, éventuellement ramifiée, éventuellement substituée par un radical phényle, une fonction acide et/ou un radical alkylester en C₁-C₃; ladite chaîne étant éventuellement interrompue par un atome d'oxygène,
- -CO-, -O-CO-, -CO-NH- ou 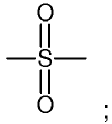
**R¹** représente un radical de 1 à 21 atomes de carbone éventuellement ramifié et/ou cyclique, saturé ou non, dont un ou plusieurs des atomes de carbone peut être remplacé par un atome d'azote, d'oxygène et/ou de soufre ; ledit radical étant éventuellement mono ou bi-substitué par un atome d'halogène, une fonction -COOH, -OH, - NO₂ un radical alkyl en C₁-C₃, un radical alcoxy en C₁-C₃ ou un radical acyloxy en C₁-C₃ ;
**R²** représente soit une liaison, soit est choisi parmi un atome d'hydrogène, un radical alkyl en C₁-C₃, éventuellement insaturé, éventuellement ramifié, un radical acyl en C₂-C₄ ou le radicale 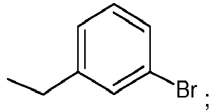 étant entendu que lorsque **R²** est une liaison alors l'atome d'azote portant R² et **X** ou l'atome de carbone adjacent (lorsque p = 1) sont liés par une double liaison,
étant entendu que **X**, **R¹** et **R²** peuvent former avec l'atome d'azote adjacent un cycle imidazole, oxazole, triazole, benzimidazol, éventuellement partiellement saturé tel notamment, que le dihydroimidazole, éventuellement substitué par un radical phényle ou pyridine ;
et leurs sels pharmaceutiquement acceptables
pour la préparation d'une composition pharmaceutique destinée à la prévention et/ou au traitement des intoxications à au moins une toxine à mode d'action intracellulaire ou à au moins un virus utilisant la voie d'internalisation pour infecter les cellules eucaryotes de mammifères.

2. Utilisation selon la revendication 1, **caractérisée en ce que** ledit composé de formule générale (I) est tel que **R¹** est un radical phényl éventuellement substitué et/ou **X** est -CH₂-et/ou **R²** est un atome d'hydrogène.

3. Utilisation selon la revendication 1, **caractérisée en ce que** ledit composé de formule générale (I) est tel que **R¹** est le radical : 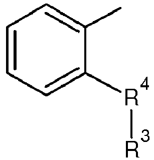 dans lequel :
- **R³** est choisi parmi un radical cyclique à 5 ou 6 atomes de carbone, saturé ou non ; un radical bicyclique à 9 ou 10 atomes de carbone, saturé ou non ; un radical tricyclique à 14 atomes de carbone, saturé ou non ; un radical hétérocyclique à 5 atomes, saturé ou non ; un radical hétérocycle à 6 atomes, saturé ou non ; un radical bihétérocyclique à 9 ou 10 atomes, saturé ou non ; lesdits radicaux étant optionnellement substitués par au moins un atome d'halogène, -NO₂, -OH ou un radical alkyl en C₁-C₃ ; et
- **R⁴** est choisi parmi -CO-O-, -N=CH- ou -NH-CH₂-.

4. Utilisation selon la revendication 1, **caractérisée en ce que** ledit composé de formule générale (I) est choisi parmi :
Composé 1 : *N*-benzyladamantylamine
Composé 2 : *N*-(2-bromobenzyl)adamantylamine
Composé 3 : *N*-(3-bromobenzyl)adamantylamine
Composé 4 : *N*-(4-bromobenzyl)adamantylamine
Composé 5 : *N*-(3-fluorobenzyl)adamantylamine
Composé 6 : *N*-(3-hydroxybenzyl)adamantylamine
Composé 7 : *N*-(2-methoxybenzyl)adamantylamine
Composé 8 : *N*-(3-methoxybenzyl)adamantylamine
Composé 9 : *N-*(4-methoxybenzyl)adamantylamine
Composé 10 : *N*-(2-nitrobenzyl)adamantylamine
Composé 11 : *N*-(4-nitrobenzyl)adamantylamine
Composé 12 : N-(4-carbethoxybenzyl)adamantylamine
Composé 13 : 4-bromo-2-((1-adamantylino)methyl)phenol
Composé 14 : *N*-(2-bromo-5-nitrobenzyl)adamantylamine
Composé 15 : *N*-[(2-methoxy-5-bromo)benzyl]adamantylamine
Composé 16 : *N*-((pyridin-2-yl)methyl)adamantylamine
Composé 17 : *N*-((pyridin-3-yl)methyl)adamantylamine
Composé 18 : *N*-((pyridin-4-yl)methyl)adamantylamine
Composé 19 : *N*-((5-methylfuran-2-yl)methyl)adamantylamine
Composé 20 : *N*-((5-methylthiophen-2-yl)methyl)cyclohexanamine
Composé 21 : *N*-[(3-furyl)methyl]adamantylamine
Composé 22 : *N*-((1-methyl-1*H* imidazol-5-yl)methyl)adamantylamine
Composé 23 : *N*-[(5-N-methylimidazolyl)methyl]adamantylamine
Composé 24 : Benzo[*d*][1,3]dioxol-4-yl)methyl)adamantylamine
Composé 25 : *N*-((5-nitrobenzo[*d*][1,3]dioxol-6-yl)methyl)adamantylamine
Composé 26 : *N*-((quinolin-3-yl)methyl)adamantylamine
Composé 27 : *N*-((quinolin-4-yl)methyl)adamantylamine
Composé 28 : *N*-((1-methyl-1*H* indol-2-yl)methyl)adamantylamine
Composé 29 : *N*-phenethyladamantylamine
Composé 30 : *N*-(3-phenylpropyl)adamantylamine
Composé 31 : *N*-(2-(benzyloxy)ethyl)adamantylamine
Composé 32 : *N*-cinnamyladamantylamine
Composé 33 : *N-methyl(3-bromobenzyl)adamantylamine*
Composé 34 : *N*-benzyl-2-adamantylamine
Composé 35 : *N*-(2-bromobenzyl)-2-adamantylamine
Composé 36 : *N*-(3-bromobenzyl)-2-adamantylamine
Composé 37 : *N*-(4-bromobenzyl)adamantylamine
Composé 38 : *N*-(2-fluorobenzyl) -2-adamantylamine
Composé 39 : *N-*(3-fluorobenzyl)-2-adamantylamine
Composé 40 : *N*-(4-fluorobenzyl)-2-adamantylamine
Composé 41 : *N*-(3-hydroxybenzyl)-2-adamantylamine
Composé 42 : *N*-(2-methoxybenzyl)-2-adamantylamine
Composé 43 : *N*-(3-methoxybenzyl)-2-adamantylamine
Composé 44 : *N*-(4-methoxybenzyl)-2-adamantylamine
Composé 45 : *N*-(2-nitrobenzyl)-2-adamantylamine
Composé 46 : *N*-(4-nitrobenzyl)-2-adamantylamine
Composé 47 : *N*-(4-carbethoxybenzyl)-2-adamantylamine
Composé 48 : 4-bromo-2-((2-adamantylamino)methyl)phenol
Composé 49 : *N*-(2-bromo-5-nitrobenzyl)-2-adamantylamine
Composé 50 : *N*-(5-bromo-2-methoxybenzyl)-2-adamantylamine
Composé 51 : *N*-(5-fluoro-2-nitrobenzyl)-2-adamantylamine
Composé 52 : N-(2,5-difluorobenzyl)-2-adamantylamine
Composé 53 : *N*-((pyridin-2-yl)methyl)-2-adamantylamine
Composé 54 : *N*-*((*pyridin-3-yl)methyl)-2-adamantylamine
Composé 55 : *N*-((pyridin-4-yl)methyl)-2-adamantylamine
Composé 56 : *N*-((5-methylfuran-2-yl)methyl)-2-adamantylamine
Composé 57 : *N*-((5-methylthiophen-2-yl)methyl)-2-adamantylamine
Composé 58 : *N*-((furan-3-yl)methyl)-2-adamantylamine
Composé 59 : N-((1-methyl-1H-imidazol-5-yl)methyl)-2-adamantylamine
Composé 60 : N-[(5-N-methylimidazolyl)methyl]-2-adamantylamine
Composé 61 : Benzo[d][1,3]dioxol-4-yl)methyl)-2-adamantylamine
Composé 62 : *N*-((5-nitrobenzo[d][1,3]dioxol-6-yl)methyl)-2-adamantylamine
Composé 63 : *N*-((quinolin-3-yl)methyl)-2-adamantylamine
Composé 64 : *N*-((quinolin-4-yl)methyl)-2-adamantylamine
Composé 65 : *N*-((1-methyl-1*H-*indol-2-yl)methyl)-2-adamantylamine
Composé 66 : *N*-(1-(3-bromophenyl)ethyl)-2-adamantylamine
Composé 67 : *N*-benzhydryl-2-adamantylamine
Composé 68 : *N*-(2-(benzyloxy)ethyl)-2-adamantylamine
Composé 69 : *N*-(phenylpropyl)-2-adamantylamine
Composé 70 : *N*-(1-phenylethyl)-2-adamantylamine
Composé 71 : *N*-(1-(pyridin-2-yl)ethyl)-2-adamantylamine
Composé 72 : *N-(-2-adamantylmethyl)-1-(adamantyl)ethanamine*
Composé 73 : *N-methyl(-3bromobenzyl)-2-adamantylamine*
Composé 86 : *N*-(3-bromobenzyl)-noradamantylamine
Composé 87 : N-(3-bromobenzyl)-1-hydroxy-2-adamantylamine
Composé 109 : *N*-(3-fluorobenzyl)noradamantylamine
Composé 110 : *N*-(3-bromobenzyl)adamantylamine hydrochloryde salt
Composé 111 : *N*-(5-bromo-2-methoxybenzyl)adamantylamine hydrochloryde salt
Composé 112 : *N*-(2-bromobenzyl)-2-adamantylamine hydrochloryde salt
Composé 113 : (*E*)-*N*-(3-bromobenzylidene)adamantylamine
Composé 114 : (*E*)-*N-*(3-fluorobenzylidene)adamantylamine
Composé 115 : (*E*)-*N*-((1-methyl-1*H*-indol-2-yl)methylene)adamantylamine
Composé 116 : (*E*)-*N*-benzylideneadamantylamine
Composé 117 : (*E*)-*N*-(3-bromobenzylidene)adamantylamine
Composé 118 : (*E*)-*N*-(3-fluorobenzylidene)adamantylamine
Composé 119 : (*E*)-*N*-(3-bromobenzylidene)noradamantylamine
Composé 120 : (*E*)-*N*-((1-methyl-1*H*-indol-2-yl)methylene)noradamantylamine
Composé 121 : *N*-1-adamantylbenzamide
Composé 122 : *N*-2-adamantylbenzamide
Composé 123 : phenyl N-adamantylcarbamate
Composé 124 : N-adamantyl benzenesulfonamide
Composé 125 : N-adamantyl-N-(3-bromobenzyl)acetamide
Composé 126 : phenyl N-2-adamantylcarbamate
Composé 127 : N-adamantyl-N-(3-bromobenzyl)benzamide
Composé 128 : N-2-adamantyl benzenesulfonamide
Composé 129 : 1-(adamantyl)-3-phenylurea
Composé 131 : 1-(adamantyl)-1-(3-bromobenzyl)-3-phenylurea
Composé 132 : 1-(2-adamantyl)-3-phenylurea
Composé 134 : 1-(adamantyl)-2,5-dihydro-1H-imidazole
Composé 135 : 1-(adamantyl)-2,5-dihydrooxazole
Composé 136 : 1-(adamantyl)-1-phenyl-4,5-dihydro-1H-imidazole
Composé 137 : 1-(adamantyl)-3a,4,5,6,7,7a-hexahydro-1H-benzo[d]imidazole
Composé 138 : *N*-(3-chlorobenzyl)adamantylamine
Composé 139 : *N*-(3-chlorobenzyl)-2-adamantylamine
Composé 140 : *N*-(3-iodobenzyl)-2-adamantylamine
Composé 141 : N-(2,2-diphenylethyl)-2-adamantylamine
Composé 142 : N-(naphthalen-1-ylmethyl)-2-adamantylamine
Composé 143 : N-(phenanthren-9-ylmethyl)-2-adamantylamine
Composé 144 : 4-((adamantylamino)methyl)benzoic acid
Composé 145 : adamantylamino-4-phenyl-1H-1,2,3-triazole
Composé 146 : adamantylamino-4-phenyl-1H-1,2,3-triazole
Composé 147 : 2-(adamantylamino-1H-1,2,3-triazol-4-yl)pyridine
Composé 148 : *N*-(2-bromo-5-nitrobenzyl)adamantylamine
Composé 191 : *N*-cinnamyl-2-adamantylamine
Composé 192 : N-(3-nitrobenzyl)-2-adamantylamine

5. Composés de formule générale (I) : 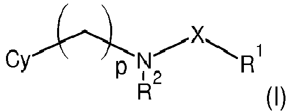 dans laquelle :
**Cy** représente un groupement 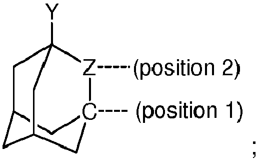 **Y** est choisi parmi un atome d'hydrogène ou une fonction hydroxyle et **Z** est un atome de carbone ou une liaison (Cy est alors un noyau noradamantyle),
la chaîne 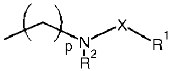 étant attachée au noyau adamantyle **Cy** en position 1 ou 2,
**p** représente 0 ou 1 ;
**X** représente soit :
- une liaison ;
- une chaîne alkyle en C₁-C₆, éventuellement insaturée, éventuellement ramifiée, éventuellement substituée par un radical phényl, une fonction acide et/ou un radical alkylester en C₁-C₃; ladite chaîne étant éventuellement interrompue par un atome d'oxygène,
- -CO-, -O-CO-, -CO-NH- ou 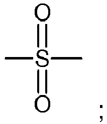
**R¹** représente un radical de 1 à 21 atomes de carbone éventuellement ramifié et/ou cyclique, saturé ou non, dont un ou plusieurs des atomes de carbone peut être remplacé par un atome d'azote, d'oxygène et/ou de soufre ; ledit radical étant éventuellement mono ou bi-substitué par un atome d'halogène, une fonction -COOH, -OH, - NO₂ un radical alkyl en C₁-C₃, un radical alcoxy en C₁-C₃ ou un radical acyloxy en C₁-C₃ ;
**R²** représente soit une liaison, soit est choisi parmi un atome d'hydrogène, un radical alkyl en C₁-C₃, éventuellement insaturé, éventuellement ramifié, un radical acyl en C₂-C₄ ou le radical 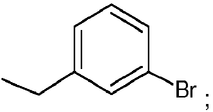 étant entendu que lorsque **R²** est une liaison alors l'atome d'azote portant R² et **X** ou l'atome de carbone adjacent (lorsque p = 1) sont liés par une double liaison,
étant entendu que **X**, **R¹** et **R²** peuvent former avec l'atome d'azote adjacent un cycle imidazole, oxazole, triazole, benzimidazol, éventuellement partiellement saturé tel notamment, que le dihydroimidazole, éventuellement substitué par un radical phényl ou pyridine ;
et leurs sels pharmaceutiquement acceptables
à l'exclusion des composés 1, 6, 8, 11, 18, 29, 30, 34, 113, 114, 116, 121, 122, 124, 128, 135 et 145.

6. Composés de formule générale (I) selon la revendication 5, **caractérisés en ce que R¹** est un radical phényl éventuellement substitué et/ou **X** est -CH₂- et/ou **R²** est un atome d'hydrogène.

7. Composé selon la revendication 5 ou 6 pour une utilisation comme médicament.

8. Médicament comprenant au moins un composé selon la revendication 5 ou 6, dans au moins un véhicule pharmaceutiquement acceptable.

9. Utilisation selon l'une quelconque des revendications 1 à 4, **caractérisée en ce que** ladite composition est destinée à être administrée par voie orale, aérienne, parentérale ou locale.

10. Utilisation selon l'une quelconque des revendications 1 à 4, pour la préparation d'une composition pharmaceutique pour prévenir et/ou traiter une intoxication à la ricine.
ledit composé étant en particulier choisi parmi les composés 1, 3, 5, 9, 15, 19, 24, 28, 34, 36, 39, 59, 65, 67, 68, 69, 86, 109, 110, 111, 112, 117, 118, 122, 128, 131, 138, 139, 140, 142, 143 et 148.

11. Utilisation selon la revendication 10, **caractérisée en ce que** le composé de formule générale (I) est défini par la formule générale (I.1)
dans laquelle : 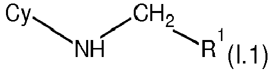 **Cy** représente un groupement 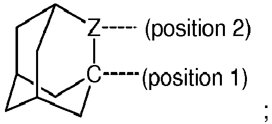 Z est un atome de carbone ou une liaison (Cy est alors un noyau noradamantyle) étant entendu que quand **Cy** est un noyau adamantyle, l'atome d'azote lui est attaché en position 1 ou 2,
**R¹** représente :
- un cycle phényl, éventuellement substitué par un radical -OCH₃ en para de l'atome de carbone lié à la chaine -CH₂-NH-Cy ; ledit cycle étant alternativement éventuellement substitué par un atome d'halogène en position méta de l'atome de carbone lié à la chaine - CH₂-NH-Cy et dans ce cas, ledit cycle porte éventuellement une seconde substitution en position para dudit atome d'halogène, ladite seconde substitution étant choisie parmi -NO₂ et -OCH₃ ;
- un cycle indole, imidazole ou furane substitué par un radical méthyle,
- un cycle benzo-(1,3)-dioxolo, un cycle naphthalènyle ou un cycle phénanthrényle ;
et leur sels pharmaceutiquement acceptables,
ledit composé de formule générale (I.1) étant en particulier choisi parmi les composés 1, 3, 5, 9, 15, 19, 24, 28, 34, 36, 39, 59, 65, 86, 109, 110, 111, 112, 138, 139, 140, 142, 143 et 148.

12. Utilisation selon la revendication 10, **caractérisée en ce que** le composé de formule générale (I) est défini par la formule générale (I.2) : 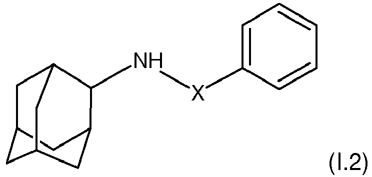 dans laquelle **X** représente -(CH₂)₂-O-CH₂-, -(CH₂)₃-, -CO-, -SO₂-
et leur sels pharmaceutiquement acceptables.
ledit composé de formule générale (I.2) étant en particulier choisi parmi les composés 68, 69, 122 et 128.

13. Utilisation selon la revendication 10, **caractérisée en ce que** le composé de formule générale (I) est défini par la formule générale (I.3) 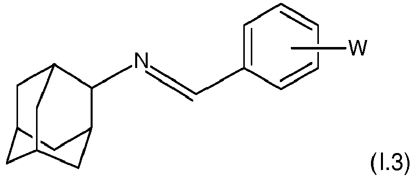 dans laquelle **W** représente un atome d'halogène
et leur sels pharmaceutiquement acceptables,
ledit composé de formule générale (I.3) étant en particulier choisi parmi les composés 117 et 118.

14. Utilisation selon l'une quelconque des revendications 1 à 4, pour la préparation d'une composition pharmaceutique pour prévenir et/ou traiter une intoxication à la toxine diphtérique.

15. Utilisation selon la revendication 14, **caractérisée en ce que** le composé de formule générale (I) est défini par la formule générale (I.5) : 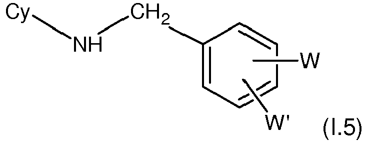 dans laquelle :
**Cy** représente un groupement: 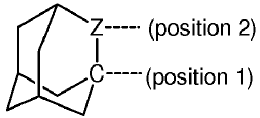 auquel l'atome d'azote est attaché en position 1 ou 2,
**W et W'** sont, indépendamment l'un de l'autre, choisis parmi un atome d'hydrogène, un atome d'halogène et un radical alcoxy en C₁-C₃
et leur sels pharmaceutiquement acceptables,
ledit composé de formule générale (I) étant en particulier choisi parmi les composés 5, 9, 39, 110, 111, 112, 139 et 140.
